(19) Europäisches Patentamt
European Patent Office
Office européen des brevets

(11) Veröffentlichungsnummer: **0 006 217**
**B1**

(12) # EUROPÄISCHE PATENTSCHRIFT

(45) Veröffentlichungstag der Patentschrift: **31.03.82**

(51) Int. Cl.³: **C 07 C 103/46**, C 07 C 103/84, A 61 K 31/195

(21) Anmeldenummer: **79101911.0**

(22) Anmeldetag: **12.06.79**

(54) **Substituierte Aminoalkansäuren, ihre Verwendung und Herstellung und sie enthaltende Arzneimittel.**

(30) Priorität: **14.06.78 CH 6505/78**

(43) Veröffentlichungstag der Anmeldung:
**09.01.80 Patentblatt 80/1**

(45) Bekanntmachung des Hinweises auf die Patenterteilung:
**31.03.82 Patentblatt 82/13**

(84) Benannte Vertragsstaaten:
**AT BE CH DE FR GB IT LU NL SE**

(56) Entgegenhaltungen:
**DE-A-1 917 036**
**FR-A-2 100 836**
**DE-A-2 450 680**

(73) Patentinhaber: **Byk Gulden Lomberg Chemische Fabrik GmbH, Byk-Gulden-Strasse 2, D-7750 Konstanz (DE)**

(72) Erfinder: **Krastinat, Walter, Dr. Dipl.-Ing., Erfurter Strasse 9, D-7750 Konstanz (DE)**
Erfinder: **Riedel, Richard, Dr., Bruelstrasse 22, D-7750 Konstanz (DE)**
Erfinder: **Wolf, Horst, Dr. Dipl.-Chem., Brandesstrasse 29, D-7750 Konstanz (DE)**

**EP 0 006 217 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Substituierte Aminoalkansäuren, ihre Verwendung und Herstellung und sie enthaltende Arzneimittel

Die Erfindung betrifft substituierte Aminoalkansäuren, ihre Verwendung und Herstellung und sie enthaltende Arzneimittel.

Die erfindungsgemäßen Verbindungen werden in der pharmazeutischen Industrie zur Herstellung von Medikamenten verwendet.

Naphthylaminoalkansäuren und -carbonsäureestern wird eine zentral depressive und stark sedative Wirkung zugeschrieben (DE-OS 1 543 802). Bei der Untersuchung von N-Benzoyl-anilinoalkansäuren (D. Evans et al., J. Med. Chem. 12 [1969] 1006 – 10) wiesen die entsprechenden Buttersäuren keine entzündungshemmende Wirkung auf, während in der deutschen Offenlegungsschrift DE-OS 1 917 036 choleretisch wirkende N-Acyl-anilinoalkansäuren beschrieben werden, denen auch noch weitere Wirkungen zugesprochen werden (DE-OS 2 450 680). $\alpha$-Phenyl-benzyliden-$\omega$-aminoalkansäuren und ihre Derivate sollen in antiepileptisch wirkenden Mitteln eingesetzt werden (DE-OS 2 634 288). In einer Reihe von deutschen Offenlegungsschriften (DE-OS 2 131 626, 2 131 674, 2 131 675, 2 131 679 und 2 131 680) werden Trialkoxybenzoylaminoalkancarbonsäuren beschrieben, die zur Prophylaxe und Behandlung des Herzinfarktes eingesetzt werden sollen. Es wurde nun eine neue Klasse von Acyl(bicyclisches aryl)aminoalkansäuren synthetisiert, die weder in den erwähnten Publikationen genannt, noch durch sie nahegelegt wird. Ferner wurde gefunden, daß diese Acyl(bicyclisches aryl)-aminoalkansäuren interessante, besonders vorteilhafte pharmakologische Eigenschaften aufweisen.

Gegenstand der Erfindung sind Acyl(bicyclisches aryl)-aminoalkansäuren der allgemeinen Formel I

$$\begin{array}{l} R^1-CO-N-C_nH_{2n}-COOH \\ \qquad\qquad\quad | \\ \qquad\qquad\quad A \end{array} \qquad (I)$$

worin

R¹ einen aliphatischen oder alicyclischen Kohlenwasserstoffrest oder eine Phenylgruppe

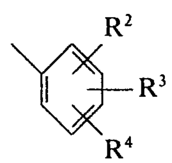

A einen gegebenenfalls teilweise hydrierten bicyclischen Arylrest mit 8 bis 12 Kohlenstoffatomen, der gegebenenfalls ein- oder mehrfach durch Halogenatome, Alkyl- oder Alkoxygruppen mit 1 bis 4 Kohlenstoffatomen, gegebenenfalls substituierte Aminogruppen, Hydroxy-, Nitro- oder Trifluormethylgruppen substituiert ist,

$R^2$, $R^3$ und $R^4$ gleich oder verschieden sind und ein Wasserstoffatom, ein Halogenatom, eine Alkylgruppe, eine Hydroxygruppe, eine Alkoxygruppe, eine Acyloxygruppe, eine gegebenenfalls substituierte Aminogruppe, eine Nitrogruppe oder eine Trifluormethylgruppe und

n 3, 4 oder 5 bedeuten,

und ihre Salze mit anorganischen oder organischen Basen.

Als aliphatische Kohlenwasserstoffreste, die gesättigt oder ungesättigt sein können, kommen geradkettige oder verzweigte Alkylreste mit 1 bis 7 Kohlenstoffatomen in Frage. Geradkettige Alkylreste sind der Methyl-, Ethyl-, Propyl-, Butyl-, Pentyl-, Hexyl- oder Heptylrest, von denen die mit 1 bis 5, vor allem mit 1 bis 3, Kohlenstoffatomen bevorzugt sind. Verzweigte Alkylreste mit 3 bis 7 Kohlenstoffatomen sind z. B. der Isopropyl-, Isobutyl-, sek.-Butyl- oder tert.-Butylrest, von denen die mit 3 bis 5, vor allem mit 3 Kohlenstoffatomen bevorzugt sind. Ungesättigte Kohlenstoffreste sind Alkenyl- und Alkinylreste mit 2 bis 7 Kohlenstoffatomen, beispielsweise der Ethenyl-, der Ethinyl-, der 1-Propenyl-, 1,3-Butadienyl-, 2-Butinylrest, von denen der 1-Propenylrest bevorzugt ist. Als alicyclische Kohlenwasserstoffreste kommen Cycloalkylreste mit 3 bis 10 Kohlenstoffatomen, beispielsweise der Cyclopropyl-, Cyclobutyl-, Cyclopentyl-, Cyclohexyl-, Cycloheptyl- oder Cyclooctylrest, von denen die mit 5 bis 7 Kohlenstoffatomen bevorzugt sind, in Frage. Als Halogenatome $R^2$, $R^3$ und $R^4$ kommen Fluor, Chlor oder Brom, bevorzugt Fluor und Chlor, insbesondere Chlor, in Betracht. Als Alkylgruppen bzw. Alkoxygruppen $R^2$, $R^3$ und $R^4$ seien unter anderem die mit 1 bis 4 Kohlenstoffatomen genannt, von denen die mit 1 bis 3, vor allem die mit 1 Kohlenstoffatom(en) bevorzugt sind. Als Acyloxygruppen kommen unter anderem $-O-CO-R^1$-Gruppen, in denen R¹ die vorstehend angegebene Bedeutung hat, in Betracht, von denen die Alkanoyloxygruppen mit 1 bis 7, insbesondere mit 2 bis 5, Kohlenstoffatomen, vor allem die Acetoxygruppe, bevorzugt sind. Neben der unsubstituierten Aminogruppe kommen als Substituenten $R^2$, $R^3$ und $R^4$ auch substituierte Aminogruppen in Frage, von

denen beispielsweise genannt seien Alkylamino- und Dialkylaminogruppen mit 1 bis 4, vorzugsweise mit 1 oder 2 Kohlenstoffatomen im Alkylrest sowie Acylaminogruppen mit den üblichen zum Schutz von Aminogruppen verwendeten Acylgruppen, wie Alkanoylgruppen mit 2 bis 5 Kohlenstoffatomen.

Als bicyclische Arylreste mit 8 bis 12 Kohlenstoffatomen kommen beispielsweise solche in Betracht, die sich von Pentalen, Inden, Naphthalin, Azulen, Heptalen, Biphenylen ableiten. Hydrierte bicyclische Arylreste leiten sich von Di-, Tetra- oder Hexahydroderivaten ab, als deren Vertreter genannt seien 1,4-Dihydronaphthalin, 1,2,3,4-Tetrahydronaphthalin, Indan, 6,7,8,9-Tetrahydro-5H-benzocyclohepten, 4,5,6,7,8,9-Hexahydro-1H-cyclopentacycloocten. Kohlenstoffatome der Substituenten bleiben bei der Berechnung der Zahl der Kohlenstoffatome der Arylsubstituenten außer Betracht.

Als Salze kommen Salze mit anorganischen und organischen Basen in Betracht. Pharmakologisch nicht verträgliche Salze werden nach an sich bekannten Methoden in pharmakologisch, d. h. biologisch, verträgliche Salze übergeführt, die unter den erfindungsgemäßen Salzen bevorzugt sind. Als Kationen für die Salzbildung werden vor allem die Kationen der Alkalimetalle, Erdalkalimetalle oder Erdmetalle verwendet, es kommen jedoch auch die entsprechenden Kationen organischer Stickstoffbasen, wie Amine, Aminoalkanole, Aminozucker, basische Aminosäuren etc. zur Anwendung.

Beispielsweise seien die Salze von Lithium, Natrium, Kalium, Magnesium, Calcium, Aluminium, Ethylendiamin, Dimethylamin, Diethylamin, Morpholin, Piperidin, Piperazin, Methylcyclohexylamin, Benzylamin, Ethanolamin, Diethanolamin, Triethanolamin, Tris-(hydroxymethyl)-aminomethan, 2-Amino-2-methylpropanol, 2-Amino-2-methyl-1,3-propandiol, Glucamin, N-Methylglucamin, Glucosamin, N-Methylglucosamin, Lysin, Ornithin, Arginin, Chinolin genannt.

Eine Ausgestaltung der Erfindung sind Acyl(bicyclisches aryl)aminoalkansäuren der allgemeinen Formel I*

$$\begin{array}{l} R^{1*}-CO-N-C_{n^*}H_{2n^*}-COOH \\ \qquad\qquad\;\; | \\ \qquad\qquad\; A^* \end{array} \qquad (I^*)$$

worin

$R^{1*}$ einen aliphatischen Kohlenwasserstoffrest mit 1 bis 5 Kohlenstoffatomen, einen alicyclischen Kohlenwasserstoffrest mit 5 bis 7 Kohlenstoffatomen oder einen Phenylrest

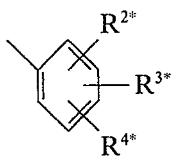

A* einen durch $R^{5*}$, $R^{6*}$ substituierten Indenyl-, Indanyl-, Naphthyl-, Dihydronaphthyl- oder Tetrahydronaphthylrest,

n* 3, 4 oder 5 bedeuten,

$R^{2*}$, $R^{3*}$ und $R^{4*}$ gleich oder verschieden sind und ein Wasserstoffatom, ein Halogenatom, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Nitrogruppe, eine Hydroxygruppe oder eine Trifluormethylgruppe bedeuten,

$R^{5*}$ und $R^{6*}$ gleich oder verschieden sind und ein Wasserstoffatom, ein Halogenatom, eine Methylgruppe, eine Alkoxygruppe mit 1 bis 4 Kohlenstoffatomen, eine Nitrogruppe oder eine Trifluormethylgruppe darstellen,

und ihre Salze mit anorganischen und organischen Basen.

Bevorzugte durch $R^{5*}$ und $R^{6*}$ substituierte A*-Reste sind der 5-Indanyl-, 1-Naphthyl- oder 1,2,3,4-Tetrahydronaphthyl-(1)-rest.

Eine weitere Ausgestaltung der Erfindung sind Acyl(bicyclisches aryl)aminoalkansäuren der allgemeinen Formel I**

$$\begin{array}{l} R^{1**}-CO-N-C_{n^{**}}H_{2n^{**}}-COOH \\ \qquad\qquad\;\;\; | \\ \qquad\qquad\;\; A^{**} \end{array} \qquad (I^{**})$$

worin

$R^{1**}$ einen aliphatischen Kohlenwasserstoffrest mit 1 bis 5 Kohlenstoffatomen oder einen durch $R^{2**}$, $R^{3**}$ und $R^{4**}$ substituierten Phenylrest,

$A^{**}$ eine Gruppe der Formel

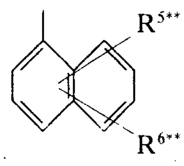

$n^{**}$ 3, 4 oder 5 bedeuten,

$R^{2**}$, $R^{3**}$ und $R^{4**}$ gleich oder verschieden sind und ein Wasserstoffatom, ein Halogenatom, eine Methylgruppe, eine Methoxygruppe, eine Nitrogruppe, eine Hydroxygruppe oder eine Trifluormethylgruppe darstellen,

$R^{5**}$ und $R^{6**}$ gleich oder verschieden sind und ein Wasserstoffatom, ein Chloratom, eine Ethoxygruppe, eine Methoxygruppe oder eine Nitrogruppe darstellen,

und ihre Salze mit anorganischen und organischen Basen.

Eine weitere Ausgestaltung der Erfindung sind Acyl(bicyclisches aryl)aminoalkansäuren der allgemeinen Formel I***

$$R^{1***}-CO-\underset{\displaystyle A^{***}}{\underset{|}{N}}-C_{n^{***}}H_{2n^{***}}-COOH \qquad (I^{***})$$

worin

$R^{1***}$ einen aliphatischen Kohlenwasserstoffrest mit 1 bis 5 Kohlenstoffatomen oder einen durch $R^{2***}$, $R^{3***}$, $R^{4***}$ substituierten Phenylrest,

$A^{***}$ eine Gruppe der Formel

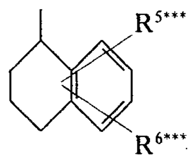

$n^{***}$ 3, 4 oder 5 bedeuten,

$R^{2***}$, $R^{3***}$ und $R^{4***}$ gleich oder verschieden sind und ein Wasserstoffatom, ein Halogenatom, eine Methylgruppe, eine Methoxygruppe, eine Nitrogruppe, eine Hydroxygruppe oder eine Trifluormethylgruppe darstellen,

$R^{5***}$ und $R^{6***}$ gleich oder verschieden sind und ein Wasserstoffatom, ein Chloratom, eine Methoxygruppe oder eine Nitrogruppe darstellen,

und ihre Salze mit anorganischen und organischen Basen.

Eine weitere Ausgestaltung der Erfindung sind Acyl(bicyclisches aryl)aminoalkansäuren der allgemeinen Formel I****

$$R^{1****}-CO-\underset{\displaystyle A^{****}}{\underset{|}{N}}-C_{n^{****}}H_{2n^{****}}-COOH \qquad (I^{****})$$

worin

$R^{1****}$ einen aliphatischen Kohlenwasserstoffrest mit 1 bis 5 Kohlenstoffatomen oder einen durch $R^{2****}$, $R^{3****}$, $R^{4****}$ substituierten Phenylrest,

$A^{****}$ eine Gruppe der Formel

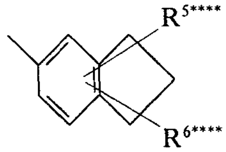

$n^{****}$ 3, 4 oder 5 bedeuten,

$R^{2****}$, $R^{3****}$ und $R^{4****}$ gleich oder verschieden sind und ein Wasserstoffatom, ein Halogenatom, eine Methylgruppe, eine Methoxygruppe, eine Nitrogruppe, eine Hydroxygruppe oder eine

Trifluormethylgruppe darstellen,

$R^{5****}$ und $R^{6****}$ gleich oder verschieden sind und ein Wasserstoffatom, ein Chloratom, eine Methoxygruppe oder eine Nitrogruppe darstellen,

und ihre Salze mit anorganischen und organischen Basen.

Bevorzugte Vertreter der Ausgestaltungen I**, I*** und I**** sind solche, in denen $R^{1**}$, $R^{1***}$ bzw. $R^{1****}$ eine Alkylgruppe mit 1 bis 3 Kohlenstoffatomen oder einen substituierten Phenylrest; $R^{2**}$, $R^{2***}$ bzw. $R^{2****}$ ein Wasserstoffatom; $R^{3**}$, $R^{3***}$ bzw. $R^{3****}$ ein Wasserstoffatom, ein Chloratom, eine Methoxygruppe, eine Hydroxygruppe oder eine Trifluormethylgruppe; $R^{4**}$, $R^{4***}$ bzw. $R^{4****}$ ein Wasserstoffatom oder ein Chloratom; $R^{5**}$, $R^{5***}$ bzw. $R^{5****}$ ein Wasserstoffatom oder eine Methoxygruppe und $R^{6**}$, $R^{6***}$ bzw. $R^{6****}$ ein Wasserstoffatom darstellen und n**, n*** bzw. n**** die oben angegebene Bedeutung haben und ihre pharmakologisch verträglichen Salze mit anorganischen oder organischen Basen.

Besonders bevorzugte Vertreter der Ausgestaltung I**, I*** bzw. I**** sind solche, in denen $R^{1**}$, $R^{1***}$ bzw. $R^{1****}$ eine Alkylgruppe mit 1 bis 3 Kohlenstoffatomen oder einen substituierten Phenylrest; $R^{2**}$, $R^{2***}$ bzw. $R^{2****}$ ein Wasserstoffatom; $R^{3**}$, $R^{3***}$ bzw. $R^{3****}$ ein Chloratom oder eine Hydroxygruppe; $R^{4**}$, $R^{4***}$ bzw. $R^{4****}$ ein Wasserstoffatom oder ein Chloratom; $R^{5**}$, $R^{5***}$ bzw. $R^{5****}$ ein Wasserstoffatom oder eine Methoxygruppe und $R^{6**}$, $R^{6***}$ bzw. $R^{6****}$ ein Wasserstoffatom darstellen und n**, n*** bzw. n**** die oben angegebene Bedeutung haben und ihre pharmakologisch verträglichen Salze mit anorganischen oder organischen Basen.

Weitere besonders bevorzugte Vertreter sind solche der Ausgestaltungen I** und I***, in denen $R^{1**}$ bzw. $R^{1***}$ eine Alkylgruppe mit 1 bis 3 Kohlenstoffatomen oder einen substituierten Phenylrest; $R^{2**}$ bzw. $R^{2***}$ ein Wasserstoffatom; $R^{3**}$ bzw. $R^{3***}$ ein Chloratom oder eine Hydroxygruppe; $R^{4**}$ bzw. $R^{4***}$ ein Wasserstoffatom; $R^{5**}$ bzw. $R^{5***}$ ein Wasserstoffatom oder eine Methoxygruppe und $R^{6**}$ bzw. $R^{6***}$ ein Wasserstoffatom darstellen und n** bzw. n*** 3 oder 4 bedeuten.

Erfindungsgemäße Verbindungen sind beispielsweise:

4-[4-Methoxy-N-(4-chlor-1-naphthyl)-benzymido]-buttersäure;
4-[3-Trifluormethyl-N-(2-methyl-1-naphthyl)-benzamido]-buttersäure;
4-[3,5-Dichlor-N-(2-methoxy-1-naphthyl)-benzamido]-buttersäure;
4-[2-Fluor-N-(4-methyl-1-naphthyl)-benzamido]-buttersäure;
4-[N-(4-Methoxy-1-naphthyl)-n-butyramid]-buttersäure;
4-[3-Methoxy-4-methyl-N-(1-naphthyl)-benzamido]-buttersäure;
4-[N-(4-Hydroxy-1-naphthyl)-4-methylbenzamido]-buttersäure;
4-[3-Fluor-4-methyl-N-(4-nitro-1-naphthyl)-benzamido]-buttersäure;
4-[3,4-Diethoxy-N-(1-naphthyl)-benzamido]-buttersäure;
4-[4-Methyl-3-nitro-N-(7-methoxy-1-naphthyl)-benzamido]-buttersäure;
4-[N-(4-Brom-1-naphthyl)-hexanamido]-buttersäure;
4-[N-(3,4-Dinitro-1-naphthyl)-acetamido]-buttersäure;
4-[N-(3-Brom-4-methyl-1-naphthyl)-isovalerylamido]-buttersäure;
4-[4-Chlor-3-nitro-N-(4-ethoxy-1-naphthyl)-benzamido]-valeriansäure;
4-[2,4-Dichlor-N-(4-chlor-1-naphthyl)-benzamido]-valeriansäure;
5-[N-(4-Methoxy-1-naphthyl)-3-methylbenzamido]-valeriansäure;
5-[N-(5-Nitro-1-naphthyl)-propionamido]-valeriansäure;
6-[3,4-Dichlor-N-(1-naphthyl)-benzamido]-capronsäure;
6-[4-Methyl-3-nitro-N-(4-ethoxy-1-naphthyl)-benzamido]-capronsäure;
6-[N-(1-Naphthyl)-methacryloylamido]-capronsäure;
6-[N-(5-Methoxy-2-naphthyl)-crotonoylamido]-capronsäure;
4-[N-(5-Methoxy-1,2,3,4-tetrahydro-1-naphthyl)-hexanamido]-buttersäure;
4-[N-(7-Methoxy-1,2,3,4-tetrahydro-2-naphthyl)-propionamido]-buttersäure;
6-[N-(6,7-Dimethoxy-1,2,3,4-tetrahydro-2-naphthyl)-acetamido]-capronsäure;
4-[3,4-Dichlor-N-(5,6,7,8-tetrahydro-1-naphthyl)-benzamido]-buttersäure;
4-[N-(1-Indanyl)-acetamido]-buttersäure;
4-[4-Chlor-N-(2-indanyl)-benzamido]-buttersäure;
6-[3,4-Dichlor-N-(2-indanyl)-benzamido]-capronsäure;
4-[3,4-Dimethoxy-N-(4-indanyl)-benzamido]-buttersäure;
6-[3-Fluor-4-methyl-N-(4-indanyl)-benzamido]-capronsäure;
6-[N-(5-Indanyl)-isovalerylamido]-capronsäure.

Besonders interessante Vertreter der erfindungsgemäßen Verbindungen sind

4-[N-(1-Naphthyl)-acetamido]-buttersäure;
4-[3-Trifluormethyl-N-(1-naphthyl)-benzamido]-buttersäure;
4-[5-Chlor-2-methoxy-N-(1-naphthyl)-benzamido]-buttersäure;
5-[4-Chlor-N-(1-naphthyl)-benzamido]-valeriansäure;

6-[4-Chlor-N-(1-naphthyl)-benzamido]-capronsäure;
5-[N-(1-Naphthyl)-isobutyramido]-valeriansäure;
Natrium-4-[N-(6-methoxy-1,2,3,4-tetrahydro-1-naphthyl)-propionamido]-butyrat;
4-[3,4-Dichlor-N-(indan-5-yl)-benzamido]-buttersäure;
5-[4-Chlor-N-(indan-5-yl)-benzamido]-valeriansäure;
4-[2-Hydroxy-N-(1-naphthyl)-benzamido]-buttersäure
und ihre pharmakologisch verträglichen Salze.

Die erfindungsgemäßen Verbindungen weisen wertvolle pharmakologische Eigenschaften auf, die sie gewerblich verwertbar machen. Sie entfalten an Warmblütlern in erster Linie eine Magenschutzwirkung und eine Steigerung der Pankreassekretion, daneben üben sie eine Wirkung auf Leber und Galle (antihepatotoxischer Effekt und Cholerese) aus. Außerdem bewirken sie eine Hemmung der Glukosebildung aus Lactat und Pyruvat in der Leber.

Aufgrund ihrer vorteilhaften Wirksamkeit sind die Acyl(bicyclisches aryl)aminoalkansäuren zur Behandlung und Prophylaxe von Krankheiten, die auf Erkrankungen des Magens oder Darms oder auf Minderleistungen von Pankreas, Galle und/oder Leber beruhen, geeignet. Beispielsweise werden behandelt Magen- oder Darmulcera, Billroth II, Pankreas-Insuffizienz, Sprue, Maldigestionen und Malabsorptionen verschiedener Genese, akute und chronische Pankreatitis, indirekte Störungen der Pankreas-Funktion (Unterstützung der Sekretin- und Pankreozymin-Produktion), daneben Gallenblasen- und Gallenwegentzündungen, Gallenflußstörungen, Motilitätsstörungen der Gallenwege, Völlegefühl, Blähungen, Obstipationen, Oberbauchbeschwerden, hepatobiläre Funktionsstörungen, akute und chronische Hepatitis, Leberintoxikationen, Fettleber, Diabetes (maturity onset diabetes), Insulinmangeldiabetes in der Form des »brittle diabetes«, diabetische Spätschäden.

Gegenstand der Erfindung ist außerdem die Verwendung der erfindungsgemäßen Verbindungen bei der Bekämpfung der oben angegebenen Krankheiten. Ebenso umfaßt die Erfindung die Verwendung der erfindungsgemäßen Verbindungen zur Herstellung von Arzneimitteln, die zur Bekämpfung der angeführten Krankheiten eingesetzt werden.

Ein weiterer Gegenstand der Erfindung sind Arzneimittel, die eine oder mehrere der Acyl(bicyclisches aryl)aminoalkansäuren der allgemeinen Formel I

$$R^1-CO-\underset{\displaystyle A}{\underset{|}{N}}-C_nH_{2n}-COOH \qquad (I)$$

worin

R¹ einen aliphatischen oder alicyclischen Kohlenwasserstoffrest oder eine Phenylgruppe

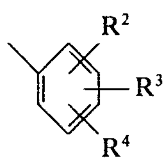

A einen gegebenenfalls teilweise hydrierten bicyclischen Arylrest mit 8 bis 12 Kohlenstoffatomen, der gegebenenfalls ein- oder mehrfach durch Halogenatome, Alkyl- oder Alkoxygruppen mit 1 bis 4 Kohlenstoffatomen, gegebenenfalls substituierte Aminogruppen, Hydroxy-, Nitro- oder Trifluormethylgruppen substituiert ist,

$R^2$, $R^3$ und $R^4$ gleich oder verschieden sind und ein Wasserstoffatom, ein Halogenatom, eine Alkylgruppe, eine Hydroxygruppe, eine Alkoxygruppe, eine Acyloxygruppe, eine gegebenenfalls substituierte Aminogruppe, eine Nitrogruppe oder eine Trifluormethylgruppe und

n 3, 4 oder 5 bedeuten,

und/oder ihre pharmakologisch verträglichen Salze mit anorganischen oder organischen Basen enthalten.

Ausgestaltungen der Arzneimittel sind solche, die Acyl(bicyclisches aryl)aminoalkansäuren der Formel I*, I**, I***, I**** oder ihre bevorzugten Vertreter und/oder ihre pharmakologisch verträglichen Salze mit anorganischen oder organischen Basen enthalten.

Die Arzneimittel werden nach an sich bekannten Verfahren hergestellt. Als Arzneimittel können die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazutische Trägerstoffe, beträgt der Wirkstoffgehalt dieser Mischungen 1 bis 95, vorzugsweise 15 bis 85 Gewichtsprozent der Gesamtmischung.

In Übereinstimmung mit der Erfindung können im human- und veterinärmedizinischen Bereich die

Wirkstoffe in jeder beliebigen Form, z. B. systemisch, angewandt werden unter der Voraussetzung, daß die Ausbildung bzw. Aufrechterhaltung von ausreichenden Blut- oder Gewebsspiegeln oder Lokalkonzentrationen an Wirkstoffen gewährleistet ist. Das kann entweder durch orale, rektale oder parenterale Gabe in geeigneten Dosen erreicht werden. Vorteilhafterweise liegt die pharmazeutische Zubereitung des Wirkstoffes in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulats, einer Lösung, einer Emulsion, einer Suspension, eines Sols oder eines Gels sein.

Unter »Einheitsdosis« im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z. B. in Form einer Tablette mit Bruchkerbe.

Die pharmazeutischen Zubereitungen gemäß der Erfindung enthalten, wenn sie in Einheitsdosen vorliegen und für die Applikation, z. B. am Menschen, bestimmt sind, etwa 0,5 bis 1000 mg, vorteilhafterweise 1 bis 750 mg und insbesondere 5 bis 500 mg Wirkstoff.

Im allgemeinen hat es sich sowohl in der Human- als auch in der Veterinärmedizin als vorteilhaft erwiesen, den oder die Wirkstoffe bei oraler Gabe in einer Tagesdosis von etwa 0,01 bis etwa 40, vorzugsweise 0,1 bis 30, insbesondere 0,2 bis 20 mg/kg Körpergewicht, gegebenenfalls in Form mehrerer, vorzugsweise 2 bis 3 Einzelgaben zur Erzielung der gewünschten Ergebnisse zu verabreichen. Eine Einzelgabe enthält den oder die Wirkstoffe in Mengen von etwa 0,01 bis etwa 20, vorzugsweise 0,1 bis 15, insbesondere 0,2 bis 10 mg/kg Körpergewicht.

Bei einer parenteralen Behandlung, z. B. einer intramuskulären oder intravenösen Applikation, können ähnliche Dosierungen zur Anwendung kommen. Bei dieser Therapie werden etwa 50 bis 1000 mg Wirkstoff appliziert.

Die therapeutische Verabreichung der pharmazeutischen Zubereitung erfolgt bei Dauermedikation im allgemeinen zu festgelegten Zeitpunkten, wie 1- bis 4mal am Tage, z. B. jeweils vor oder nach den Mahlzeiten und/oder am Abend. Bei akuten Anlässen erfolgt die Medikation zu variierendem Zeitpunkt. Unter bestimmten Gegebenheiten kann es erforderlich sein, von den genannten Dosierungen abzuweichen, und zwar in Abhängigkeit von der Art, dem Körpergewicht und dem Alter des zu behandelnden Individuums, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann der Fachmann aufgrund seines Fachwissens jederzeit vornehmen.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzuges, eines Beutels oder eines anderen Behältnisses, für den therapeutisch aktiven Bestandteil in Anwendung kommen. Ein Trägerstoff kann z. B. als Vermittler für die Arzneimittelaufnahme durch den Körper, als Formulierungshilfsmittel, als Süßungsmittel, als Geschmackskorrigens, als Farbstoff oder als Konservierungsmittel dienen. Die Trägerstoffe werden jeweils vom Fachmann auf die mit den pharmazeutischen Zubereitungen zu behandelnden Krankheiten abgestimmt.

Zur oralen Anwendung können z. B. Tabletten, Dragées, harte und weiche Kapseln, z. B. aus Gelatine, dispergierbare Pulver, Granulate, wäßrige und ölige Suspensionen, Emulsionen, Lösungen oder Sirupe kommen.

Tabletten können inerte Verdünnungsmittel, z. B. Calciumcarbonat, Calciumphosphat, Natriumphosphat oder Lactose; Granulierungs- und Verteilungsmittel, z. B. Maisstärke oder Alginate; Bindemittel, z. B. Stärke, Gelatine oder Akaziengummi; und Gleitmittel, z. B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption des Arzneimittels im Gastrointestinaltrakt und damit z. B. eine bessere Verträglichkeit, Protrahierung oder eine Retardierung erreicht wird. Gelatinekapseln können den Arzneistoff vermischt mit einem festen, z. B. Calciumcarbonat oder Kaolin, oder einem öligen, z. B. Oliven-, Erdnuß- oder Paraffinöl, Verdünnungsmittel enthalten.

Wäßrige Suspensionen können Suspendiermittel, z. B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z. B. Polyoxyethylenstearat, Heptadecaethylenoxycetanol, Polyoxyethylensorbitolmonooleat, Polyoxyethylensorbitanmonooleat oder Lecithin; Konservierungsmittel, z. B. Methyl- oder Propylhydroxybenzoate; Geschmacksmittel; Süßungsmittel, z. B. Natriumcyclamat, Saccharin, enthalten.

Ölige Suspensionen können z. B. Erdnuß-, Oliven-, Sesam-, Kokos- oder Paraffinöl und Verdickungsmittel, wie z. B. Bienenwachs, Hartparaffin oder Cetylalkohol, enthalten; ferner Süßungsmittel, Geschmacksmittel und Antioxidantien.

In Wasser dispergierbare Pulver und Granulate können die Arzneistoffe in Mischung mit Dispergier-, Benetzungs- und Suspendiermitteln, z. B. den obengenannten, sowie mit Süßungsmitteln, Geschmacksmitteln und Farbstoffen enthalten.

Emulsionen können z. B. Oliven-, Erdnuß oder Paraffinöl neben Emulgiermitteln, wie z. B. Akaziengummi, Traganthgummi, Phosphatiden, Sorbitanmonooleat, Polyoxyethylensorbitanmonooleat, und Süßungs- und Geschmacksmitteln enthalten.

Zur rektalen Anwendung der Arzneistoffe gelangen Suppositorien, die mit Hilfe von bei Rektaltemperatur schmelzenden Bindemitteln, beispielsweise Kakaobutter oder Polyethylenglycolen, hergestellt werden.

Zur parenteralen Anwendung der Arzneistoffe dienen steril injizierbare wäßrige Suspensionen, isotonische Salzlösungen oder sonstige Lösungen, die Dispergier- oder Benetzungsmittel und/oder pharmakologisch verträgliche Verdünnungsmittel, z. B. Propylen- oder Butylenglycil, enthalten können.

Der oder die Wirkstoffe können gegebenenfalls mit einem oder mehreren der angegebenen Träger- oder Zusatzstoffe auch in mikroverkapselter Form formuliert werden.

Sollen die erfindungsgemäßen Acyl(bicyclisches aryl)aminoalkansäuren und/oder ihre Salze zur Behandlung von Krankheiten, die auf Erkrankungen des Magens oder Darms oder auf Minderleistungen von Pankreas, Galle und/oder Leber beruhen, eingesetzt werden, so können die pharmazeutischen Zubereitungen auch einen oder mehrere andere pharmakologisch aktive Bestandteile anderer Arzneimittelgruppen, wie Antacida, beispielsweise Aluminiumhydroxid, Magnesiumaluminat; Tranquilizer, wie Benzodiazepine, beispielsweise Diazepam; Spasmolytica, wie z. B. Bietamiverin, Camylofin; Anticholinergica, wie z. B. Oxyphencyclimin, Phencarbamid; Entschäumungsmittel, beispielsweise Dimethylpolysiloxan; Laxantien, beispielsweise Bisacodyl; Quellmittel; gegebenenfalls auch Fermente, Gallensäuren, Antibiotika, Vitamine, Aminosäuren, Fettsäuregemische etc. enthalten.

Sollen die erfindungsgemäßen Acyl(bicyclisches aryl)aminoalkansäuren und/oder ihre Salze als Antidiabetika formuliert werden, so können die pharmazeutischen Zubereitungen weiterhin einen oder mehrere pharmakologisch aktive Bestandteile anderer Arzneimittelgruppen, wie zusätzliche Antidiabetica (Sulfonamide, Sulfonylharnstoffe u. a.), z. B. Carbutamid, Tolbutamid, Chlorpropamid, Glibenclamid, Glibornurid, Glisoxepid, Gliquidon, Glymidin, oder Hypolipidämika, wie Benzafibrat oder Nikotinsäure sowie deren Derivate und Salze enthalten.

Ein weiterer Gegenstand der Erfindung ist ein Verfahren zur Herstellung der Acyl(bicyclisches aryl)aminoalkansäuren der allgemeinen Formel I

$$R^1-CO-\underset{\displaystyle A}{\underset{|}{N}}-C_nH_{2n}-COOH \qquad (I)$$

worin

$R^1$ einen aliphatischen oder alicyclischen Kohlenwasserstoffrest oder eine Phenylgruppe

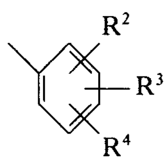

A einen gegebenenfalls teilweise hydrierten bicyclischen Arylrest mit 8 bis 12 Kohlenstoffatomen, der gegebenenfalls ein- oder mehrfach durch Halogenatome, Alkyl- oder Alkoxygruppen mit 1 bis 4 Kohlenstoffatomen, gegebenenfalls substituierte Aminogruppen, Hydroxy-, Nitro- oder Trifluormethylgruppen substituiert ist,

$R^2$, $R^3$ und $R^4$ gleich oder verschieden sind und ein Wasserstoffatom, ein Halogenatom, eine Alkylgruppe, eine Hydroxygruppe, eine Alkoxygruppe, eine Acyloxygruppe, eine gegebenenfalls substituierte Aminogruppe, eine Nitrogruppe oder eine Trifluormethylgruppe und

n 3, 4 oder 5 bedeuten,

sowie ihrer Salze mit anorganischen und organischen Basen, das dadurch gekennzeichnet ist, daß man

a) eine (bicyclisches Aryl)aminoalkansäure der allgemeinen Formel II

$$A-NH-C_nH_{2n}-COOH \quad (II)$$

worin A und n die vorstehend angegebene Bedeutung haben, gegebenenfalls unter Schutz der Carboxylgruppe, mit einem Acylderivat der allgemeinen Formel III

$$R^1-CO-R^7 \quad (III)$$

worin $R^7$ eine Fluchtgruppe oder eine $R^1-CO-O$-Gruppe darstellt und $R^1$ die oben angegebene Bedeutung hat, acyliert und gewünschtenfalls anschließend in die Salze überführt oder

b) eine (bicyclisches Aryl)aminoalkensäure der allgemeinen Formel IV

$$R^1-CO-\underset{\displaystyle A}{\underset{|}{N}}-C_nH_{2n-2}-COOH \quad (IV)$$

worin $R^1$, A und n die vorstehend angegebene Bedeutung haben, gegebenenfalls unter Schutz der Carboxylgruppe, hydriert und gewünschtenfalls anschließend in die Salze überführt oder

c) ein funktionelles Acyl(bicyclisches Aryl)aminoalkansäurederivat der allgemeinen Formel V

$$R^1-CO-\underset{\displaystyle A}{\underset{|}{N}}-C_nH_{2n}-G \quad (V)$$

worin $R^1$, A und n die oben angegebene Bedeutung haben und G ein funktionelles Derivat einer Carboxylgruppe bedeutet, solvolysiert und gewünschtenfalls anschließend in die Salze überführt.

Zur Herstellung der Verbindungen der Ausgestaltungen I*, I**, I*** bzw. I**** werden entsprechende Ausgangsmaterialien II*, II**, II*** bzw. II****; III*, III**; III*** bzw. III****; IV*, IV**, IV*** bzw. IV**** oder V*, V**, V*** bzw. V****, worin die Substituenten die oben angegebene Bedeutung haben, umgesetzt.

Werden die (bicyclisches Aryl)aminoalkansäuren der Formel II unter Schutz der Carboxylgruppe zur Reaktion gebracht, so gelangen solche Vertreter zum Einsatz, deren Schutzgruppen nicht mit den Acylderivaten III reagieren. Geeignete Vertreter sind beispielsweise Ester von Alkanolen, u. a. mit 1 bis 5 Kohlenstoffatomen, oder Phenalkanolen, wie Methyl-, Propyl-, Butyl-, Benzyl- oder Phenethylester, gegebenenfalls auch Lösungen mit anorganischen oder organischen Basen, wie Alkali- oder Erdalkalimetallhydroxiden, Ammoniak, tertiäre Stickstoffbasen (z. B. Triethylamin, Pyridin).

In den Acylderivaten III ist eine Fluchtgruppe $R^7$ beispielsweise eine Hydroxygruppe, ein Halogenatom, vorzugsweise ein Chlor- oder Bromatom, eine Alkylsulfonyloxy- oder Benzolsulfonyloxygruppe, wie eine Mesyloxy- oder p-Tolylsulfonyloxygruppe, eine Alkoxygruppe, vorzugsweise eine Methoxy- oder Ethoxygruppe, eine Alkylmercaptogruppe, wie eine Methylmercapto- oder Ethylmercaptogruppe.

Die Reaktion der (bicyclisches Aryl)aminoalkansäuren II mit den Acylderivaten III wird nach an sich bekannten Verfahren durchgeführt. Die Umsetzung wird in geeigneten Lösungsmitteln, wie Wasser oder Kohlenwasserstoffen, z. B. Benzol, Toluol, Xylol oder Ethern, z. B. Tetrahydrofuran, Dioxan, 1,2-Dimethoxyethan, oder Ketonen, z. B. Ethylmethylketon, oder Amiden, z. B. Dimethylformamid, oder Sulfoxiden, z. B. Dimethylsulfoxid, durchgeführt. Zweckmäßigerweise wird die Acylierung, wenn $R^7$ eine Fluchtgruppe bedeutet, in Gegenwart eines säurebindenden Mittels (Protonenacceptors) vorgenommen. Als solche sind beispielsweise geeignet Alkalimetallhydroxide, wie Natriumhydroxid, Kaliumhydroxid, oder Alkalimetallcarbonate, wie Natriumcarbonat, Kaliumcarbonat, oder tertiäre Amine, wie Pyridin, Triethylamin, Ethyldiisopropylamin. Stellen die Acylderivate III Säureanhydride dar, d. h. bedeutet $R^7$ eine $R^1-CO-O$-Gruppe, so genügt auch das Erhitzen der Verbindung II und III in einem inerten Lösungsmittel.

Die Reaktion kann in weiten Grenzen variiert werden, z. B. $-20$ bis $+100°C$, wobei Temperaturen um Raumtemperatur (10 bis 30°C) bevorzugt sind. Werden die Verbindungen II unter Schutz der Carboxylgruppe acyliert, so wird nach der Acylierung die Schutzgruppe in üblicher Weise wieder abgespalten. Werden Salze als Schutzgruppe eingesetzt, so erfolgt die Freisetzung der erhaltenen Säuren I durch Umsetzung mit einer geeigneten Mineralsäure, wie Salzsäure, Schwefelsäure etc. Werden Ester als Schutzgruppen eingesetzt, so folgt auf die Acylierung die Verseifung des Reaktionsproduktes zu Verbindungen der Formel I. Die Verseifung wird vorzugsweise mit einer alkoholischen (z. B. ethanolischen) Alkalimetallhydroxid- (z. B. Kaliumhydroxid-) Lösung bei Raumtemperatur vorgenommen, gegebenenfalls unter Zusatz eines inerten Verdünnungsmittels, wie Dioxan oder Benzol.

Die Ausgangsverbindungen der Formel II werden nach verschiedenen an sich bekannten Verfahren

hergestellt. So werden sie durch Umsetzung von Halogenalkansäuren der Formel VI, vorzugsweise unter Schutz der Carboxylgruppe als Estergruppe, mit einem (bicyclisches Aryl)amin der Formel VII

$$R^8—C_nH_{2n}—COOH \qquad (VI) \qquad\qquad A—NH_2 \qquad (VII)$$

worin n und A die oben angegebene Bedeutung haben und R⁸ ein Halogenatom, vorzugsweise ein Chlor- oder Bromatom, darstellt, erhalten. Die Reaktion wird zweckmäßig in Gegenwart eines inerten Lösungsmittels, z. B. Benzol, Cyclohexan, Diethylether, unter Zusatz eines Protonenacceptors durchgeführt. Als solcher dient beispielsweise ein Überschuß des zur Reaktion eingesetzten Amins VII. Gewünschtenfalls kann jedoch auch ein anderer Protonenacceptor hinzugefügt werden.

Die Vorprodukte II werden auch durch Umsetzung eines Alkensäureesters VIII

$$CH_2{=}CH—C_{n-2}H_{2n-4}—CO—O—R^9 \qquad (VIII)$$

worin n die oben angegebene Bedeutung hat und $R^9$ eine Alkylgruppe mit 1 bis 5 Kohlenstoffatomen oder eine Benzylgruppe darstellt, mit einem (bicyclisches Aryl)amin VII erhalten. Die Reaktion wird zweckmäßig in Gegenwart von inerten Lösungsmitteln, wie Kohlenwasserstoffen, z. B. Benzol, Toluol, Xylol, oder Ethern, z. B. Tetrahydrofuran, Dioxan, 1,2-Dimethoxyethan, oder Ketonen, z. B. Ethylmethylketon, oder Amiden, z. B. Dimethylformamid, oder Sulfoxiden, z. B. Dimethylsulfoxid, oder Nitrilen, z. B. Acetonitril, durchgeführt.

Zweckmäßigerweise werden die Reaktionsteilnehmer in dem Lösungsmittel, z. B. durch Kochen unter Rückfluß, erwärmt. Die Schutzgruppe $R^9$ wird zweckmäßigerweise erst nach Umsetzung der gewonnenen Zwischenprodukte II mit den Acylderivaten III unter Erhalt der Endprodukte I abgespalten.

Die Ausgangsverbindungen II werden ferner durch Solvolyse von funktionellen (bicyclisches Aryl)-aminoalkansäurederivaten der allgemeinen Formel IX

$$A—NH—C_nH_{2n}—G \qquad (IX)$$

worin A, n und G die oben angegebene Bedeutung haben, nach dem Fachmann bekannten Verfahren erhalten. Zweckmäßige Ausführungsformen werden unter der Verfahrensmaßnahme c) beschrieben.

Die Vorprodukte II werden weiterhin durch Umsetzung von (bicyclisces Aryl)aminen VII mit Lactonen der allgemeinen Formel X

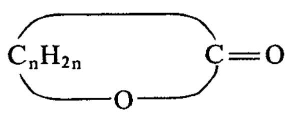

(X)

worin n die oben angegebene Bedeutung hat, in an sich bekannter Weise, erhalten. Die Umsetzung erfolgt beispielsweise durch Umsetzung der Reaktionspartner in siedenden inerten Lösungsmitteln, wie Ethern, z. B. Diethylether, Tetrahydrofuran oder Nitrilen, z. B. Acetonitril. Die erhaltene Säure kann im Anschluß daran in die entsprechenden Ester, z. B. durch Erhitzen in den entsprechenden Alkanolen in Gegenwart einer Mineralsäure, wie Salz- oder Schwefelsäure, übergeführt werden.

Die Zwischenprodukte II werden alternativ durch Hydrierung von (bicyclisches Aryl)iminoalkansäuren XI

$$A—N{=}CH—(CH_2)_{n-1}—COOH \qquad (XI)$$

worin A und n die oben angegebene Bedeutung haben, gegebenenfalls unter Schutz der Carboxylgruppe, in an sich bekannter Weise erhalten. Die Hydrierung erfolgt beispielsweise mit Raney-Nickel unter Wasserstoffdrücken von 1 bis 250 atm bei Raumtemperatur in absolutem Ethanol.

Die Säuren XI sind durch Umsetzung der (bicyclisches Aryl)amine VII mit Oxosäureestern XII

$$O{=}CH—(CH_2)_{n-1}—CO—O—R^9 \qquad (XII)$$

worin n und $R^9$ die oben angegebene Bedeutung haben, zugänglich.

Die Hydrierung gemäß Verfahrensvariante b) erfolgt nach Methoden, wie sie dem Fachmann bekannt sind. So werden die (bicyclisces Aryl)aminoalkensäuren IV mit Wasserstoff in Gegenwart eines Übergangsmetall- oder Edelmetall-Katalysators oder der entsprechenden Oxide oder Komplexe in inerten Lösungsmitteln hydriert. Geeignete Metalle sind z. B. Platin, Palladium, Iridium, Rhodium. Eine Übersicht über die Hydrierungsverfahren findet sich u. a. in Kirk-Othmer 11, 418–462; Ullmann 10, 109–114, 541–555; 14, 630–649. Die Abspaltung einer gegebenenfalls vorhandenen Schutzgruppe

erfolgt in üblicher Weise.

Die Alkensäuren IV werden beispielsweise aus den Halogenalkensäureestern XIII

$$R^{10}—C_nH_{2n-2}—CO—O—R^9 \qquad (XIII)$$

worin $R^9$ und n die oben angegebene Bedeutung haben und $R^{10}$ ein Halogenatom, vorzugsweise ein Bromatom, bedeutet, durch Aminierung mit einem (bicyclisces Aryl)amin VII, Acylierung mit einem Acylderivat III und gegebenenfalls anschließende Verseifung gewonnen. Die Herstellung erfolgt nach an sich bekannten Verfahren, Halogenierung (Herstellung von XIII) und Aminierung z. B. analog J. Heterocycl. Chem. 8 (1971) 21; Acylierung und Verseifung werden analog der Beschreibung der vorliegenden Anmeldung durchgeführt.

Die Halogenalkansäuren VI und (bicyclisches Aryl)amine VII sind bekannte Verbindungen oder werden nach Analogieverfahren hergestellt; beispielsweise sind die Halogenalkansäuren VI durch Solvolyse, wie Hydrolyse oder Alkoholyse, der entsprechenden Lactone und anschließende Halogenierung zugänglich. (Bicyclisches Aryl)amine VII werden durch Reduktion entsprechender Nitroverbindungen, die durch Nitrierung entsprechender bicyclischer Aryle zugänglich sind, gewonnen.

Die Solvolyse gemäß Verfahrensvariante c) erfolgt nach dem Fachmann bekannten Verfahren. Unter einem funktionellen Säurederivat wird hierbei ein Abkömmling verstanden, dessen funktionelle Gruppe G durch Solvolyse in die freie Carboxylgruppe überführbar ist. Typische Vertreter sind beispielsweise solche, worin G eine –CN-Gruppe oder eine

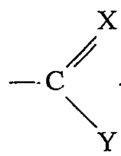 -Gruppe

bedeutet, worin

X ein Sauerstoff- oder ein Schwefelatom oder ein substituiertes Stickstoffatom, insbesondere eine Imino-, Alkylimino- oder Hydroxyiminogruppe darstellt und

Y eine Hydroxygruppe oder einen monovalenten eliminierbaren elektrophilen Rest, insbesondere eine freie oder substituierte Aminogruppe, vorzugsweise eine Monoalkyl- oder Dialkyl- oder Arylaminogruppe, eine Hydroxyamino- oder Hydrazinogruppe, eine Hydrazobenzolgruppe, eine 2-Hydroxyethylaminogruppe, eine freie oder substituierte Mercaptogruppe, vorzugsweise eine Alkylthiogruppe, eine substituierte Hydroxygruppe, vorzugsweise eine Alkoxygruppe, einen Azido-, einen Chlor- oder Bromrest, eine Morpholinogruppe oder eine Piperidinogruppe, bedeutet, wobei Y nicht eine Hydroxygruppe ist, wenn X ein Sauerstoffatom darstellt.

Unter einem Alkylrest einer Alkylimino-, einer Monoalkylamino-, einer Dialkylamino-, einer Alkylthio- und einer Alkoxygruppe wird ein Alkylrest mit bis zu 5 Kohlenstoffatomen, unter einem Arylrest einer Arylaminogruppe wird ein Arylrest bis zu 10 Kohlenstoffatomen verstanden.

Bevorzugte Vertreter der Säurederivate V sind solche, in denen G eine –CN-Gruppe oder eine

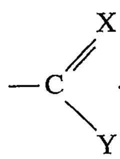 -Gruppe

darstellt, worin

X ein Sauerstoffatom, ein Schwefelatom oder eine Iminogruppe und

Y einen Amino-, Monoalkylamino-, Dialkylamino-, Phenylamino-, Alkoxy-, Alkylthio-, Chlor- oder Bromrest

bedeutet.

Besonders bevorzugte Vertreter der Säurederivate V sind die entsprechenden Säureamide, Säurealkylester und Nitrile, d. h. solche Verbindungen der Formel V, in denen G eine $CO-NH_2$-, $-CO-NH-R^9$-, $-CO-NR^9_2$-, $-CO-O-R^9$- oder –CN-Gruppe darstellt und $R^9$ die oben angegebene Bedeutung hat. Sie stellen wertvolle Zwischenprodukte für die Herstellung der Verbindungen I und ihrer Salze dar.

Zur Solvolyse der funktionellen Carbonsäurederivate V verwendet man ein wasserabgebendes Medium, das ganz oder teilweise aus Wasser bzw. aus bei den Hydrolysebedingungen

wasserabspaltenden Mitteln besteht. Die Reaktion kann als Homogenreaktion geführt werden, in welchem Fall man meist in Gegenwart eines polaren organischen Lösungsmittels oder eines Lösungsvermittlers arbeitet. Vorteilhafterweise werden als Lösungsmittel beispielsweise niedermolekulare Alkohole, Dioxan, Aceton, niedermolekulare Carbonsäuren, N-Methylpyrrolidon, Sulfolan oder Dimethylsulfoxid verwendet. Man kann aber die Hydrolyse auch als Heterogenreaktion führen. Der pH-Wert des wasserabgebenden Mediums richtet sich nach der chemischen Natur des eingesetzten Säurederivates, aber auch nach der Natur der gewünschten Verbindung der allgemeinen Formel I, er kann daher neutral, sauer oder basisch sein. Er wird mit Säuren, Basen oder Puffern auf den gewünschten Wert eingestellt.

Die Hydrolysetemperaturen liegen zwischen 0°C und dem Siedepunkt des wasserabgebenden Mediums, im allgemeinen zwischen 0° und 150°C, insbesondere zwischen 20° und 120°C. Die Hydrolysetemperaturen hängen im einzelnen auch davon ab, ob unter Druck oder ohne Druck gearbeitet wird. Die Reaktionszeiten liegen je nach Ansatz, Reaktionstemperaturen und übrigen Reaktionsparametern zwischen 10 Minuten und 20 Stunden. Nach beendeter Hydrolyse werden die Säuren I nach üblichen Methoden, z. B. durch Umkristallisation oder durch Ansäuern ihrer Lösungen, gegebenenfalls unter Einengung ihrer Lösungen, isoliert. Zu ihrer Reinigung kann deren alkalische Lösung mit einem organischen Lösungsmittel, das mit der alkalischen Lösung nicht mischbar ist, beispielsweise Diethylether, Benzol, Chlorbenzol, Chloroform oder Methylenchlorid, extrahiert werden.

Die Carbonsäurederivate V werden nach dem Fachmann geläufigen Methoden erhalten. Beispielsweise werden sie durch Reaktion von funktionellen Halogenalkansäurederivaten XIV

$$R^8—(CH_2)_n—G \qquad (XIV)$$

worin $R^8$, n und G die oben angegebene Bedeutung haben, mit (bicyclisces Aryl)aminen VII und anschließende Acylierung mit Acylderivaten III erhalten.

Die Überführung der Säuren der allgemeinen Formel I oder der Ausgestaltungen I*, I**, I***, I**** in ihre Salze kann durch direkte alkalische Hydrolyse der Säurederivate der allgemeinen Formel V erfolgen. Als alkalische Reaktionspartner wird diejenige anorganische oder organische Base verwendet, deren Salz gewünscht wird. Man erhält die Salze aber auch, indem man die Säuren der allgemeinen Formel I mit dem stöchiometrischen Äquivalent an entsprechender Base, z. B. Natriumhydroxid oder Natriumalkoholat, umsetzt, oder leicht lösliche Salze durch doppelte Umsetzung in schwer lösliche Salze überführt, oder beliebige Salze in parmakologisch verträgliche Salze überführt.

Die folgenden Beispiele erläutern die Erfindung näher, ohne sie einzuschränken. Die Abkürzung F. bzw. Sdp. bedeutet Schmelzpunkt bzw. Siedepunkt. Die Temperaturangaben erfolgen in Grad Celsius (°C).

## Beispiel 1

### 4-[p-Chlor-N-(α-naphthyl)-benzamido]-buttersäure
$R^1$ = p-Chlorphenyl, A = α-Naphthyl, n = 3

4,0 g α-Naphthylamin, 3,6 g Ethyldiisopropylamin, 5,5 g 4-Brombuttersäure-ethylester und 20 ml Cyclohexan werden zusammen 3 Stunden unter Rückfluß zum Sieden erhitzt. Nach dem Erkalten wird der Niederschlag abfiltriert und das Filtrat im Vakuum eingedampft. Der Eindampfrückstand wird in 30 ml Benzol gelöst. Nach Zugabe von 3,5 g Ethyldiisopropylamin wird eine Mischung von 4,7 g p-Chlorbenzoylchlorid und 10 ml Benzol bei Raumtemperatur innerhalb 30 Minuten unter Rühren zugetropft. Anschließend wird noch 1 Stunde gerührt, der Niederschlag abfiltriert und das Filtrat eingedampft. Der Eindampfrückstand wird in 50 ml Benzol gelöst und mit einer Lösung von 2,0 g Kaliumhydroxid in 20 ml Ethanol vermischt. Nach 12stündigem Stehen bei Raumtemperatur wird die Lösung zweimal mit je 50 ml Wasser extrahiert. Die vereinigten wäßrigen Phasen werden einmal mit Diethylether gewaschen und dann mit verdünnter Salzsäure angesäuert. Die Fällung wird abfiltriert und aus einem Gemisch Isopropylalkohol—Wasser, 1 : 1, umkristallisiert. Es werden 6,3 g (61,3% d. Th.) 4-[p-Chlor-N-(α-naphthyl)-benzamido]-buttersäure, F. 173—174°, erhalten.

## Beispiel 2

### 4-[2,4-Dichlor-N-(α-naphthyl)-benzamido]-buttersäure
$R^1$ = 2,4-Dichlorphenyl, A = α-Naphthyl, n = 3

Diese Verbindung wird gemäß dem in Beispiel 1 beschriebenen Verfahren erhalten, jedoch unter Verwendung von 2,4-Dichlorbenzoylchlorid. F. 134—136° (aus Isopropylalkohol—Wasser 1 : 1).

Beispiel 3

4-[m-Trifluormethyl-N-(α-naphthyl)-benzamido]-buttersäure
$R^1$ = m-Trifluormethylphenyl, A = α-Naphthyl, n = 3

Diese Verbindung wird gemäß dem in Beispiel 1 beschriebenen Verfahren erhalten, jedoch unter Verwendung von m-Trifluormethyl-benzoylchlorid. F. 128—129° (aus Isopropylalkohol—Wasser 1 : 1).

Beispiel 4

4-[o-Hydroxy-N-(α-naphthyl)-benzamido]-buttersäure
$R^1$ = o-Hydroxyphenyl, A = α-Naphthyl, n = 3

Diese Verbindung wird gemäß dem in Beispiel 1 beschriebenen Verfahren erhalten, jedoch unter Verwendung von o-Acetoxybenzoylchlorid. F. 126—128° (aus Ethanol—Wasser 1 : 1).

Beispiel 5

4-[5-Chlor-2-methoxy-N-(α-naphthyl)-benzamido]-buttersäure
$R^1$ = 5-Chlor-2-methoxyphenyl, A = α-Naphthyl, n = 3

Diese Verbindung wird gemäß dem in Beispiel 1 beschriebenen Verfahren erhalten, jedoch unter Verwendung von 5-Chlor-2-methoxy-benzoylchlorid. F. 170—171° (aus Isopropylalkohol).

Beispiel 6

4-[3,4,5-Trimethoxy-N-(α-naphthyl)-benzamido]-buttersäure
$R^1$ = 3,4,5-Trimethoxyphenyl, A = α-Naphthyl, n = 3

Diese Verbindung wird gemäß dem in Beispiel 1 beschriebenen Verfahren erhalten, jedoch unter Verwendung von 3,4,5-Trimethoxybenzoylchlorid. F. 143—145° (aus Isopropylalkohol—Diethylether 2 : 1).

Beispiel 7

4-[N-(α-Naphthyl)-acetamido]-buttersäure
$R^1 = CH_3$, A = α-Napthyl, n = 3

Diese Verbindung wird gemäß dem in Beispiel 1 beschriebenen Verfahren hergestellt, jedoch unter Verwendung von Acetylchlorid. F. 117—119° (aus Cyclohexan).

Beispiel 8

5-[p-Chlor-N-(α-naphthyl)-benzamido]-valeriansäure
$R^1$ = p-Chlorphenyl, A = α-Naphthyl, n = 4

a) 5-[p-Chlor-N-(α-naphthyl)-benzamido]-valeriansäure-ethylester

7,2 g α-Naphthylamin, 6,5 g Ethyldiisopropylamin, 10,5 g 5-Bromvaleriansäure-ethylester und 40 ml Cyclohexan werden 20 Stunden unter Rückfluß zum Sieden erhitzt. Nach dem Erkalten wird der Niederschlag abfiltriert und das Filtrat im Vakuum eingedampft. Der Eindampfrückstand wird in 80 ml Benzol gelöst. Nach Zugabe von 6,8 g Ethyldiisopropylamin wird eine Mischung von 9,2 g p-Chlorbenzoylchorid und 20 ml Benzol innerhalb 1 Stunde unter Rühren bei Raumtemperatur zugetropft. Nach 24 Stunden wird das Reaktionsgemisch mit Diethylether verdünnt und der Niederschlag abfiltriert. Das Filtrat wird nacheinander mit Wasser, verdünnter Salzsäure und Natriumhydrogencarbonatlösung gewaschen, dann getrocknet und eingedampft. Der Eindampfrückstand wird durch Chromatographie über eine Kieselgelsäule, Laufmittel Dichlormethan, gereinigt und dann aus einem 1 : 1-Gemisch von Cyclohexan—Petrolether (Sdp. 50—70°) umkristallisiert. Es werden 13,2 g (64% d. Th.) 5-[p-Chlor-N-(α-naphthyl)-benzamido]-valeriansäure-ethylester, F. 74—75°, erhalten.

b) 5-[p-Chlor-N-(α-naphthyl-benzamido]-valeriansäure

12,8 g 5-[p-Chlor-N-(α-naphthyl)-benzamido]-valeriansäure-ethylester in 100 ml Benzol werden mit einer Lösung von 2,6 g Kaliumhydroxid in 20 ml Ethanol vermischt. Nach 2 Tagen wird das Lösungsmittel abdestilliert, der Rückstand in Wasser gelöst, die wässerige Lösung mit Diethylether gewaschen und dann mit verdünnter Salzsäure angesäuert. Das Reaktionsprodukt wird mit Diethylether extrahiert und der nach dem Trocknen und Abdestillieren des Lösungsmittels verbleibende Rückstand aus Essigsäureethylester – Petrolether (1 : 1) umkristallisiert. Es werden 10,6 g (88,9% d. Th.) 5-[p-Chlor-N-(α-naphthyl)-benzamido]-valeriansäure, F. 169 – 170°, erhalten.

Beispiel 9

6-[p-Chlor-N-(α-naphthyl)-benzamido]-capronsäure
$R^1$ = p-Chlorphenyl, A = α-Naphthyl, n = 5

Nach dem in Beispiel 8a angegebenen Verfahren, wobei jedoch anstelle von 5-Bromvaleriansäure-ethylester 6-Bromcapronsäure-ethylester verwendet wird, erhält man 6-[p-Chlor-N-(α-naphthyl)-benzamido]-capronsäure-ethylester, F. 76,5 – 77,5° (aus Cyclohexan – Petrolether 1 : 1). Durch Verseifung dieses Esters gemäß Beispiel 8b wird 6-[p-Chlor-N-(α-naphthyl)-benzamido]-capronsäure, F. 131 – 132° (aus Essigsäureethylester – Petrolether 1 : 1) erhalten.

Beispiel 10

4-[p-Chlor-N-(α-naphthyl)-benzamido]-valeriansäure
$R^1$ = p-Chlorphenyl, A = α-Naphthyl, n = 4

Nach dem in Beispiel 1 angegebenen Verfahren, wobei anstelle von 4-Brombuttersäure-ethylester 4-Bromvaleriansäure-ethylester verwendet wird, erhält man 4-[p-Chlor-N-(α-naphthyl)-benzamido]-valeriansäure, F. 119 – 120° (aus Essigsäureethylester – Petrolether 1 : 1).

Beispiel 11

4-[p-Chlor-N-(7-methoxy-1-naphthyl)-benzamido]-buttersäure
$R^1$ = p-Chlorphenyl, A = 7-Methoxy-1-naphthyl, n = 3

10,4 g 1-Amino-7-methoxy-naphthalin, 7,8 g Ethyldiisopropylamin, 11,8 g 4-Brombuttersäure-ethylester und 50 ml Cyclohexan werden 5 Stunden unter Rückfluß zum Sieden erhitzt. Nach dem Erkalten werden der Niederschlag abfiltriert, das Filtrat eingedampft und der Eindampfrückstand wird in 70 ml Benzol gelöst; nach Zugabe von 7,8 g Ethyldiisopropylamin und 10,5 g p-Chlorbenzoylchlorid wird das Gemisch 2 Stunden bei Raumtemperatur gerührt. Der nach dem Abfiltrieren des Niederschlags und Eindampfen des Filtrats hinterbleibende Rückstand wird chromatographisch gereinigt (Aluminiumoxid-Säule, Laufmittel: Dichlormethan). Es werden 12,3 g (48,1% d. Th.) 4-[p-Chlor-N-(7-methoxy-1-naphthyl)-benzamido]-buttersäure-ethylester, F. 92 – 94° (aus Isopropylalkohol) erhalten.

Durch Verseifung von 9,0 g dieses Esters, gemäß Beispiel 8b, werden 7,4 g (88% d. Th.) 4-[p-Chlor-N-(7-methoxy-1-naphthyl)-benzamido]-buttersäure, F. 148 – 150° (aus Isopropylalkohol) erhalten.

Beispiel 12

4-[p-Chlor-N-(4-ethoxy-1-naphthyl)-benzamido]-buttersäure
$R^1$ = p-Chlorphenyl, A = 4-Ethoxy-1-naphthyl, n = 3

Nach dem in Beispiel 1 angegebenen Verfahren, wobei anstelle von α-Naphthylamin 1-Amino-4-naphthol-ethylether eingesetzt wird, erhält man 4-[p-Chlor-N-(4-ethoxy-1-naphthyl)-benzamido]-buttersäure, F. 111 – 113° (aus Essigsäureethylester – Petrolether 1 : 1).

Beispiel 13

4-[N-(1,2,3,4-Tetrahydro-1-naphthyl)-acetamido]-buttersäure
$R^1 = -CH_3$, A = 1,2,3,4-Tetrahydro-1-naphthyl, n = 3

14,7 g 1-Amino-1,2,3,4-tetrahydronaphthalin und 9,7 g 4-Brombuttersäure-ethylester werden zusammen 48 Stunden bei Raumtemperatur gerührt. Nach Zugabe von 200 ml Diethylether wird der Niederschlag abfiltriert und das Filtrat mit verdünnter Salzsäure ausgeschüttelt. Der Salzsäureextrakt wird mit verdünnter Ammoniaklösung neutralisiert (pH ~7). Das abgeschiedene Öl wird in Diethylether aufgenommen und der nach dem Trocknen und Abdestillieren des Lösungsmittels verbleibende Rückstand (10,9 g) in 100 ml Benzl gelöst. Nach Zugabe von 5,9 g Ethyldiisopropylamin werden 3,6 g Acetylchlorid langsam unter Rühren eingetropft. Nach 3 Stunden wird das Reaktionsgemisch mit 200 ml Diethylether versetzt und nacheinander mit Wasser, verdünnter Salzsäure und mit Natriumhydrogencarbonat-Lösung ausgeschüttelt. Der nach dem Trocknen und Abdestillieren des Lösungsmittels verbleibende Rückstand (12,4 g) wird in 100 ml Benzol gelöst und mit einer Lösung von 3,0 g Kaliumhydroxid in 20 ml Ethanol versetzt. Nach 24 Stunden wird das Gemisch mit Wasser ausgeschüttelt, die wässerige Phase wird angesäuert. Der zunächst ölige Niederschlag wird in Dichlormethan aufgenommen. Nach dem Abdestillieren des Lösungsmittels kristallisiert das Produkt nach mehreren Tagen. Es wird aus einer Mischung Isopropylalkohol—Petrolether (2 : 1) umkristallisiert. Es werden so 7,9 g (57,7% d. Th.) 4-[N-(1,2,3,4-Tetrahydro-1-naphthyl)-acetamido-buttersäure, F. 141—142°, erhalten.

Beispiel 14

4-[N-(1,2,3,4-Tetrahydro-1-naphthyl)-isobutyramido]-buttersäure
$R^1 = -CH(CH_3)_2$, A = 1,2,3,4-Tetrahydro-1-naphthyl, n = 3

Nach dem in Beispiel 13 angegebenen Verfahren, wobei anstelle von Acetylchlorid Isobutyrylchlorid verwendet wird, erhält man 4-[N-(1,2,3,4-Tetrahydro-1-naphthyl)-isobutyramido]-buttersäure, F. 92,5—93,5°.

Beispiel 15

Natrium-4-[N-(6-methoxy-1,2,3,4-tetrahydro-1-naphthyl)-propionamido]-butyrat
$R_1 = -CH_2-CH_3$, A = 6-Methoxy-1,2,3,4-tetrahydro-1-naphthyl, n = 3

Nach dem in Beispiel 13 angegebenen Verfahren, wobei 6-Methoxy-1-amino-1,2,3,4-tetrahydronaphthalin anstelle von 1-Amino-1,2,3,4-tetrahydronaphthalin und Propionylchlorid anstelle von Acetylchlorid verwendet werden, erhält man 4-[N-(6-Methoxy-1,2,3,4-tetrahydro-1-naphthyl)-propionamido]-buttersäure als zähes Öl, das auch nach mehreren Wochen nicht kristallisiert. Eine Lösung von 8,9 g dieser Säure in 90 ml Isopropanol wird mit einer Natriumisopropylatlösung vermischt, die durch Auflösen von 0,60 g Natrium in 50 ml Isopropanol hergestellt wird. Das Lösungsmittel wird abdestilliert und der Rückstand mit wasserfreiem Diethylether versetzt. Nach einiger Zeit erstarrt er zu einer festen Kristallmasse. Diese wird zerrieben und mehrmals mit wasserfreiem Diethylether gewaschen. Es werden so 8,3 g (87,3% d. Th.) des Natriumsalzes der 4-[N-(6-Methoxy-1,2,3,4-tetrahydro-1-naphthyl)-propionamido]-buttersäure als farblose hygroskopische Kristalle, die unscharf bei 75° bis 85° schmelzen, erhalten.

Beispiel 16

Natrium-4-[N-(6-Methoxy-1,2,3,4-tetrahydro-1-naphthyl)-isobutyramido]-butyrat
$R_1 = -CH(CH_3)_2$, A = 6-Methoxy-1,2,3,4-tetrahydro-1-naphthyl, n = 3

Die Titelverbindung wird gemäß Beispiel 15 hergestellt, jedoch unter Verwendung von Isobutyrylchlorid anstelle von Propionylchlorid. Sie bildet stark hygroskopische Kristalle, die unscharf bei 90° bis 95° schmelzen.

Beispiel 17

5-[N-($\alpha$-Naphthyl)-isobutyramido]-valeriansäure
$R_1 = -CH(CH_3)_2$, A = $\alpha$-Naphthyl, n = 4

Diese Verbindung wird gemäß dem in Beispiel 1 beschriebenen Verfahren hergestellt, jedoch unter Verwendung von 5-Bromvaleriansäureethylester anstelle von 4-Brombuttersäureethylester bzw. von Isobutyrylchlorid anstelle von p-Chlorbenzoylchlorid. F. = 93–94° (aus Diethylether–Petrolether 1 : 1).

Beispiel 18

5-[p-Chlor-N-(indan-5-yl)-benzamido]-valeriansäure
$R^1$ = p-Chlorphenyl, A = Indan-5-yl, n = 4

a) 5-(Indan-5-yl-amino)-valeriansäure-ethylester

13,3 g 5-Aminoindan, 23,0 g 5-Bromvaleriansäureethylester und 14,2 g Ethyldiisopropylamin werden zusammen 12 Stunden bei 50° gerührt. Nach Zusatz von Diethylether werden der Niederschlag abfiltriert, das Lösungsmittel abgezogen und aus dem Rückstand bei 50° und einem Druck von 1,33 Pa alle flüchtigen Bestandteile abdestilliert. Der verbleibende Rückstand wird aus Petrolether umkristallisiert. Es werden so 8,3 g (55,7% d. Th.) 5-(Indan-5-yl-amino)-valeriansäure-ethylester, F. 61–63°, erhalten.

b) 5-[p-Chlor-N-(indan-5-yl)-benzamido]-valeriansäure

6,3 g 5-(Indan-5-yl-amino)-valeriansäure-ethylester und 3,1 g Ethyldiisopropylamin in 50 ml Benzol werden mit 4,2 g p-Chlorbenzoylchlorid umgesetzt. Der nach dem Abfiltrieren des Niederschlags und Eindampfen des Filtrats hinterbliebende Rückstand wird, wie in Beispiel 8b beschrieben, verseift. Nach dem Umkristallisieren des Rohprodukts aus Essigsäureethylester–Petrolether (1 : 2) werden 7,9 g (88,1% d. Th.) 5-[p-Chlor-N-(indan-5-yl)-benzamido]-valeriansäure, F. 132–134°, erhalten.

Beispiel 19

4-[3,4-Dichlor-N-(indan-5-yl)-benzamido]-buttersäure
$R^1$ = 3,4-Dichlorphenyl, A = Indan-5-yl, n = 3

Nach dem in Beispiel 18 angegebenen Verfahren, wobei 4-Brombuttersäureethylester anstelle von 5-Bromvaleriansäureethylester und 3,4-Dichlorbenzoylchlorid anstelle von p-Chlorbenzoylchlorid verwendet werden, erhält man 4-[3,4-Dichlor-N-(indan-5-yl)-benzamido]-buttersäure, F. 58–59° (aus Essigsäureethylester–Petrolether [1 : 2]).

Beispiel 20

Ampullen mit 600 mg 4-[N-($\alpha$-Naphthyl)-acetamido]-buttersäure, Chargengröße 250 kg

25,0 kg 1,2-Propylenglykol und 150,0 kg aqua bidestillata werden vorgelegt, 15,000 kg 4-[N-($\alpha$-Naphthyl)-acetamido]-buttersäure zugegeben und anschließend wird langsam, unter Rühren, Natronlauge (10 Gew.-% NaOH) zugegeben. Wenn sich alles gelöst hat, wird der pH-Wert auf 7,5–8,0 eingestellt. 0,0625 kg Natriumpyrosulfit werden zugegeben und die Mischung wird gerührt, bis sich alles gelöst hat. Mit aqua bidestillata wird auf 250 kg aufgefüllt. Die Lösung wird in 10-ml-Ampullen abgefüllt und bei 120° 30 Minuten im Autoklaven sterilisiert.

Beispiel 21

Ampullen mit 600 mg 4-[5-Chlor-2-methoxy-N-($\alpha$-naphthyl)-benzamido]-buttersäure, Chargengröße 250 kg

50,000 kg 1,2-Propylenglykol und 150,0 kg aqua bidestillata werden vorgelegt. Dann werden unter Rühren 15 kg 4-[5-Chlor-2-methoxy-N-($\alpha$-naphthyl)-benzamido]-buttersäure zugegeben. Anschließend wird Natronlauge (10 Gew.-% NaOH) hinzugefügt und die Mischung dabei auf einen pH-Wert von 8,0 eingestellt. Mit aqua bidestillata wird auf 250 kg ergänzt. Die Lösung wird in 10-ml-Ampullen abgefüllt und 30 Minuten bei 120° im Autoklaven sterilisiert.

### Beispiel 22

Tabletten mit 50 mg 6-[p-Chlor-N-($\alpha$-naphthyl)-benzamido]-capronsäure

25,000 kg 6-[p-Chlor-N-($\alpha$-naphthyl)-benzamido]-capronsäure, 35,000 kg Milchzucker und 26,000 kg Maisstärke werden mit 21,500 kg Polyvinylpyrrolidon (MG ~25.000) in ungefähr 6 Liter Wasser granuliert. Das Granulat wird durch ein Sieb von 1,25 mm Maschenweite gesiebt und nach dem Trocknen werden 8,000 kg Carboxymethylcellulose, 2,500 kg Talkum und 1,000 kg Magnesiumstearat zugegeben. Man verpreßt das trockene Granulat zu Tabletten von 8 mm Durchmesser, 250 mg Gewicht und einer Härte von 5—6 kg.

In ähnlicher Weise werden Tabletten mit Natrium-4-[N-(6-methoxy-1,2,3,4-tetrahydro-1-naphthyl)-propionamido]-butyrat bzw. 4-[3,4-Dichlor-N-(indan-5-yl)-benzamido]-buttersäure hergestellt.

### Beispiel 23

Tabletten mit 100 mg 4-[p-Chlor-N-($\alpha$-naphthyl)-benzamido]-valeriansäure

40,000 kg 4-[p-Chlor-N-($\alpha$-naphthyl)-benzamido]-valeriansäure, 24,000 kg Milchzucker und 16,000 kg Maisstärke werden mit 4,000 kg Polyvinylpyrrolidon (MG ~25.000) in ungefähr 5,5 Liter Wasser granuliert und durch ein Sieb von 1,25 mm Maschenweite gepreßt. Nach dem Trocknen werden 10,000 kg Carboxymethylcellulose, 4,000 kg Talkum und 2,000 kg Magnesiumstearat zugegeben. Auf einer Exzentermaschine wird das Granulat zu Tabletten von 9 mm Durchmesser, 250 mg Gewicht und einer Härte von 4—5 verpreßt.

MG = Molekulargewicht.

### Beispiel 24

Tabletten mit 300 mg 5-[N-($\alpha$-Naphthyl)-isobutyramido]-valeriansäure

60,000 kg 5-[N-($\alpha$-Naphthyl)-isobutyramido]-valeriansäure, 12,000 kg Milchzucker und 8,000 kg Maisstärke werden mit 4,000 kg Polyvinylpyrrolidon (MG ~25.000) in ungefähr 6 Liter Wasser granuliert und durch ein Sieb von 1,25 mm Maschenweite gepreßt. Nach dem Trocknen werden 10,000 kg Carboxymethylcellulose, 4,000 kg Talkum und 2,000 kg Magnesiumstearat zugegeben. Auf einem Rundläufer wird das Granulat zu Tabletten von 11 mm Durchmesser, 500 mg Gewicht und einer Härte von 6—7 kg verpreßt.

### Beispiel 25

10 000 Kapseln mit einem Wirkstoffgehalt von 50 mg werden aus folgenden Bestandteilen hergestellt: 500 g 6-[p-Chlor-N-($\alpha$-naphthyl)-benzamido]-capronsäure, 495 g mikrokristalline Cellulose und 5 g amorphe Kieselsäure. Der Wirkstoff in feingepulverter Form, die Cellulose und die Kieselsäure werden gut vermischt und in Hartgelatinekapseln Größe 4 abgefüllt.

## Pharmakologie

Die Acyl-(bicyclisches aryl)-aminoalkansäuren entfalten eine starke Magenschutzwirkung und eine Steigerung der Pankreassekretion bei Ratten, daneben üben sie an Ratten eine Wirkung auf Leber und Galle (Leberschutz und Cholerese) aus, wobei sie sich bekannten Handelspräparaten, z. B. Piprozolin, Carbenoxolon, überlegen erwiesen. Außerdem bewirken sie eine Hemmung der Glukosebildung aus Lactat und Pyruvat in der Leber von Ratten, wobei sie sich bekannten Handelspräparaten, z. B. Buformin, Phenformin, überlegen erweisen.

In den anschließenden Tabellen werden die untersuchten Verbindungen durch eine laufende Nummer gekennzeichnet, die wie folgt zugeordnet ist:

| Lfd. Nr. | Name der Verbindung |
|---|---|
| 1 | Piprozolin |
| 2 | Carbenoxolon |
| 3 | Buformin |
| 4 | Phenformin |
| 5 | 4-[p-Chlor-N-(α-naphthyl)-benzamido]-buttersäure |
| 6 | 4-[N-(α-Naphthyl)-acetamido]-buttersäure |
| 7 | 4-[3,4,5-Trimethoxy-N-(α-naphthyl)-benzamido-buttersäure |
| 8 | 4-[2,4-Dichlor-N-(α-naphthyl)-benzamido]-buttersäure |
| 9 | 4-[m-Trifluormethyl-N-(α-naphthyl)-benzamido]-buttersäure |
| 10 | 4-[p-Chlor-N-(4-ethoxy-1-naphthyl)-benzamido]-buttersäure |
| 11 | 4-[5-Chlor-2-methoxy-N-(α-naphthyl)-benzamido]-buttersäure |
| 12 | 4-[p-Chlor-N-(α-naphthyl)-benzamido]-valeriansäure |
| 13 | 4-[p-Chlor-N-(7-methoxy-1-naphthyl)-benzamido]-buttersäure |
| 14 | 5-[p-Chlor-N-(α-naphthyl)-benzamido]-valeriansäure |
| 15 | 6-[p-Chlor-N-(α-naphthyl)-benzamido]-capronsäure |
| 16 | 4-[N-(1,2,3,4-Tetrahydro-1-naphthyl)-acetamido]-buttersäure |
| 17 | 4-[N-(1,2,3,4-Tetrahydro-1-naphthyl)-isobutyramido]-buttersäure |
| 18 | 5-[N-(α-Naphthyl)-isobutyramido]-valeriansäure |
| 19 | Natrium-4-[N-(6-methoxy-1,2,3,4-tetrahydro-1-naphthyl)-propionamido]-butyrat |
| 20 | 4-[3,4-Dichlor-N-(indan-5-yl)-benzamido]-buttersäure |
| 21 | 5-[p-Chlor-N-(indan-5-yl)-benzamido]-valeriansäure |
| 22 | 4-[2-Hydroxy-N-(1-naphthyl)-benzamido]-buttersäure |

**Tabelle I gibt die Magenschutzwirkung (Rückbildung der durch Pylorusligatur und Verabreichung von 100 mg/kg p. o. Acetylsalicylsäure provozierten Magenulcera) nach intraduodenaler Applikation bei der Ratte, die letale Wirkung nach intraperitonealer Gabe bei der Maus sowie den therapeutischen Quotienten (TQ = $LD_{50}/ED_{50}$) von Vertretern der erfindungsgemäßen Verbindungen wieder.**

Tabelle I

Magenschutzwirkung

| Verbindung | Toxizität $LD_{50}$ [mg/kg] (Maus i.p.) | Antiulcerogene Wirkung $ED_{50}$*) mg/kg intraduodenal | TQ $LD_{50}/ED_{50}$ |
|---|---|---|---|
| 2 | 120 | 50 | 2.4 |
| 9 | 160 | 13 | 12.3 |
| 10 | 70 | 10 | 7.0 |
| 11 | 270 | 50 | 5.4 |
| 12 | 180 | 5.5 | 32.7 |
| 13 | 120 | 26 | 4.6 |
| 14 | 170 | 20 | 8.5 |
| 15 | 160 | 30 | 5.3 |
| 18 | >400 | 14 | 28.6 |
| 19 | >400 | <10 | >40 |
| 20 | 180 | <10 | >18 |
| 21 | 150 | 20 | 7.5 |
| 22 | >400 | 10 | >40 |

*) $ED_{50}$ = Dosis, die den mittleren Ulcusindex um 50% mindert.

In Tabelle II ist der Einfluß auf die Pankreassekretion von narkotisierten Ratten nach intraduodenaler Applikation ($ED_{50}$) und die letale Wirkung an der Maus ($LD_{50}$) nach intraperitonealer Applikation von Vertretern der erfindungsgemäßen Verbindungen sowie der therapeutische Quotient (TQ = $LD_{50}/ED_{50}$) wiedergegeben.

Tabelle II

Pankreassekretion, Toxizität und therapeutischer Quotient

| Verbindung | Toxizität $LD_{50}$ [mg/kg] (Maus, i.p.) | Pankreassekretion $ED_{50}$*) [mg/kg] (Ratte, i.d.) | TQ $LD_{50}/ED_{50}$ |
|---|---|---|---|
| 1 | 1070**) | 35 | 31 |
| 5 | 140 | 2.5 | 56 |
| 6 | 750 | ~1.5 | ~500 |
| 7 | 360 | ~7 | ~ 51 |
| 8 | 240 | 7.5 | 32 |
| 9 | 160 | <1 | >160 |
| 10 | 70 | 0.7 | 100 |
| 11 | 270 | ~1 | ~270 |
| 12 | 180 | ~1 | ~180 |
| 13 | 120 | 0.3 | 400 |
| 14 | 170 | 1.2 | 142 |
| 15 | 160 | ~1 | ~160 |
| 17 | >400 | ~3 | ≳133 |
| 19 | >400 | <1 | >400 |
| 20 | 180 | <1 | >180 |
| 22 | >400 | 10 | > 40 |

*) $ED_{50}$ = Dosis, die eine Steigerung der Pankreassekretion (Flüssigkeitsvolumen; 30-min-Fraktion) um maximal 50% bewirkt.
**) $LD_{50}$ [p.o.], zit. aus Herrmann et al. Arzneim. Forsch. 27 (1977) 467.

In Tabelle III werden Untersuchungen zur antihepatotoxischen Wirkung ($ED_{25;50}$) der erfindungsgemäßen Verbindungen nach oraler Applikation an wachen Ratten und die letale Wirkung nach intraperitonealer Applikation an der Maus ($LD_{50}$) sowie der therapeutische Quotient (TQ = $LD_{50}/ED_{25}$ bzw. $LD_{50}/ED_{50}$) wiedergegeben.

Tabelle III

Antihepatotoxizität, Toxizität und therapeutischer Quotient

| Verbindung | Toxizität $LD_{50}$ [mg/kg] Maus i.p. | Antihepatotoxischer Effekt (Ratte p.o.) $ED_{25}$*) | $ED_{50}$*) | TQ $\frac{LD_{50}}{ED_{25}}$ | $\frac{LD_{50}}{ED_{50}}$ |
|---|---|---|---|---|---|
| 1 | 1070**) | 200 | >300 | 5.4 | < 3.6 |
| 5 | 140 | ~ 5 | 40 | ~28 | 3.5 |
| 6 | 750 | <10 | 50 | >75 | 15 |
| 7 | 360 | 17 | $\gtrsim$ 30 | 21.2 | $\lesssim$12 |
| 8 | 240 | 10 | 26 | 24 | 9.2 |
| 11 | 270 | ~30 | | ~ 9 | |
| 12 | 180 | ~15 | | ~12 | |
| 13 | 120 | 7 | 16 | 17.1 | 7.5 |
| 15 | 160 | ~ 7 | | ~22.9 | |
| 16 | >400 | <10 | | >40 | |
| 20 | 180 | 10 | | 18 | |

*) $ED_{25}$ bzw. $ED_{50}$ = Dosis, die für $CCl_4$-lebergeschädigte Ratten die Hexobarbital-Narkose um 25 bzw. 50% verkürzt.
**) $LD_{50}$ [p.o.], zit. aus Herrmann et al. Arzneim. Forsch. 27 (1977) 467.

**In Tabelle IV ist der Einfluß auf die Gallesekretion (Cholerese) von narkotisierten Ratten nach intraduodenaler Applikation ($ED_{50}$) und die letale Wirkung an der Maus ($LD_{50}$) nach intraperitonealer Applikation von Vertretern der erfindungsgemäßen Verbindungen sowie der therapeutische Quotient (TQ = $LD_{50}/ED_{50}$) wiedergegeben.**

Tabelle IV

Gallesekretion, Toxizität und therapeutischer Quotient

| Verbindung | Toxizität $LD_{50}$ [mg/kg] (Maus i.p.) | Gallesekretion $ED_{50}$***) [mg/kg] (Ratte i.d.) | TQ $LD_{50}/ED_{50}$ |
|---|---|---|---|
| 1 | 1070**) | 40 | 26.8 |
| 7 | 360 | 10 | 36 |

***) $ED_{50}$ = Dosis, die eine Steigerung der Gallesekretion (Flüssigkeitsvolumen; 30-min-Fraktion) um maximal 50% bewirkt.
**) $LD_{50}$ [p.o.], zit. aus Herrmann et al. Arzneim. Forsch. 27 (1977) 467.

**In Tabelle V werden die Ergebnisse der Untersuchung des Einflusses von Vertretern der erfindungsgemäßen Verbindungen auf die Glucosebildung aus Lactat und Pyruvat an der isoliert perfundierten Leber nüchterner Ratten, wobei die Hemmung der Glucosebildung bei einer**

Substanzkonzentration von 0,2 mmol/l im Perfusat und die $ED_{50}$ — ermittelt aus 4 Konzentrationen im Bereich von 0.02—1.00 mmol/l — dargestellt sind, und die letale Wirkung an der Maus ($LD_{50}$) nach intraperitonealer Applikation wiedergegeben.

Tabelle V

Hemmung der Glucosebildung aus Lactat und Pyruvat in der isoliert perfundierten Rattenleber und Toxizität an der Maus

| Lfd. Nr. | Glucosebildung %-Veränderung*) | $ED_{50}$**) [mg/l] | $LD_{50}$ (i.p.) [mg/kg] |
|---|---|---|---|
| 3 | − 1 | >1000 | 213***) |
| 4 | − 3 | >1000 | 150****) |
| 5 | −48 | 81 | 140 |
| 14 | −86 | 31 | 170 |
| 15 | −82 | 24 | 160 |
| 17 | −33 | 97 | >400 |

*) Substanzkonzentration 0.2 mmol/l im Perfusat.
**) Dosis, die eine Hemmung der Glucosebildung um 50% bewirkt.
***) Zitiert aus Söling, H. D., Creutzfeldt, W. Int. Biguanid Symp., Aachen 1960, Stuttgart, Thieme Verlag.
****) Zitiert nach Bertarelli, P., Boll. chim. farm. 97 (1958) 396.

Die erfindungsgemäßen Verbindungen zeichnen sich gegenüber den Vergleichsverbindungen 3 und 4 durch eine wesentlich stärkere Hemmung der Glucosebildung aus Lactat und Pyruvat aus. Während 3 und 4 bei den verwendeten Konzentrationen nahezu keine Hemmung bewirken, lassen sich mit den erfindungsgemäßen Verbindungen Hemmeffekte bis zu 86% erzielen.

Die Ermittlung der pharmakologischen Eigenschaften erfolgte nach folgenden Methoden:

### Einfluß auf die Pankreas- und Gallesekretion der narkotisierten Ratte

#### Versuchsdurchführung

Männliche Sprague-Dawley-Ratten (250—300 g Körpergewicht) werden mit 1,2 g/kg Urethan i. m. narkotisiert. Dann wird die Bauchhöhle medial geöffnet, der Ductus choledochus kurz oberhalb seiner Mündung in das Duodenum sowie nahe der Leberpforte ligiert, beide Gangabschnitte werden leberwärts katheterisiert.

Da bei der Ratte alle Pankreaskanäle in den mittleren Abschnitt des Ductus choledochus münden, lassen sich auf diese Weise aus dem distalen (ligierten) Gangabschnitt das Pankreassekret und aus dem proximalen Teil des Ductus choledochus die Galle getrennt ableiten.

Die sezernierten Mengen an Pankreas- und Gallensaft werden in 30-Min.-Abständen während 2 Stunden vor bis 3 Stunden nach der intraduodenalen Verabreichung der zu prüfenden Verbindungen (verabreichtes Flüssigkeitsvolumen 5 ml/kg) gemessen.

Die Körpertemperatur der Tiere wird mittels Heizkissen und Bestrahlung auf 36—38° C gehalten; die Temperaturkontrolle erfolgt rectal.

#### Auswertung

Die Flüssigkeitsvolumina der 30-Min.-Fraktionen nach der Substanzgabe werden jeweils auf die vor der Substanzapplikation ausgeschiedene Galle- bzw. Pankreassaftmenge (= 100%, Mittelwert aus den beiden letzten Messungen) bezogen. Die maximale prozentuale Steigerung der Pankreas- bzw. Gallesekretion wird in Abhängigkeit von der Dosis dargestellt und daraus die $ED_{50}$ durch Interpolation ermittelt.

Prüfung auf antihepatotoxische Wirkung

Einfluß auf die Hexobarbitalschlafdauer der Ratte nach $CCl_4$-Leberschädigung

Versuchsdurchführung

In Anlehnung an Vogel et al. (Arzneim.-Forsch. 25 [1975] 82) wird bei nüchternen weiblichen Sprague-Dawley-Ratten (190 ± 10 g Körpergewicht, 10 Tiere/Dosis pro Versuchsansatz) durch orale Verabreichung von Tetrachlorkohlenstoff (0,15 ml/kg $CCl_4$ in 2,5 ml/kg Olivenöl) ein Leberzellenschaden erzeugt, dessen Ausmaß an der Veränderung der Hexobarbitalnatrium-Schlafdauer (50 mg/ml/kg i. v.; Schwanzvene, Injektionsdauer 45–60 sec) 47 Stunden nach der $CCl_4$-Gabe bestimmt wird. Die zu prüfenden Verbindungen werden eine Stunde vor der $CCl_4$-Gabe oral in einem Flüssigkeitsvolumen von 10 ml/kg verabreicht.

Auswertung

Der antihepatotoxische Effekt der zu prüfenden Verbindungen (Natrium-Salze in wäßriger Lösung) wird an der prozentualen Verkürzung der durch die $CCl_4$-Leberzellschädigung bedingten Schlafdauerverlängerung bei den behandelten Gruppen gegenüber der Schlafdauerverlängerung der $CCl_4$-Kontrollgruppe (= 100%) gemessen. Die $ED_{25}$ bzw $_{50}$ wurde durch Interpolation aus der Dosis-Wirkungskurve ermittelt.

Prüfung der antiulcerogenen Wirkung

Die Ulcusprovokation erfolgt bei 24 h nüchtern gehaltenen Ratten (weiblich, 180–200 g) durch Pylorusligatur (unter Ethernarkose) und orale Applikation von 100 mg/10 ml/kg Acetylsalicylsäure. Die Substanzgabe erfolgt intraduodenal (2,5 ml/kg) unmittelbar nach der Pylorusligatur. Der Wundverschluß wurde mittels Michelklammern vorgenommen. 4 h danach erfolgt die Tötung der Tiere im Etherrausch durch Atlas-Dislokation und die Resektion des Magens. Der längs eröffnete Magen wird auf einer Korkplatte fixiert, mit einem Stereomikroskop werden bei 10facher Vergrößerung Anzahl und Größe (= Durchmesser) vorhandener Ulcera ermittelt. Das Produkt aus Schweregrad (gemäß nachfolgender Punkteskala) und Anzahl der Ulcera dient als individueller Ulcusindex.

Punkteskala:

| | |
|---|---|
| keine Ulcera | 0 |
| Ø 0,1–1,4 mm | 1 |
| 1,5–2,4 mm | 2 |
| 2,5–3,4 mm | 3 |
| 3,5–4,4 mm | 4 |
| 4,5–5,4 mm | 5 |
| >5,5 mm | 6 |

Als Maß für den antiulcerogenen Effekt dient die Minderung des mittleren Ulcus-Index jeder behandelten Gruppe gegenüber dem der Kontrollgruppe (= 100%).

Bestimmung der Hemmung der Glucosebildung an der isoliert perfundierten Rattenleber

Zur Verwendung kommen junge männliche Sprague-Dawley-Ratten (160–200 g). Die Haltung der Tiere erfolgt in Käfigen bis zu 5 Tieren in einem temperierten Raum (23°C) mit festem Tag/Nacht-Rhythmus (12/12 h).

Den Tieren wird 20–22 Stunden vor der Operation das Futter entzogen. Wasseraufnahme erfolgt ad libitum. Operation und Perfusion der Leber erfolgen nach der Technik von R. Scholz et al. (Eur. J. Biochem. 38 [1973] 64–72). Als Perfusionsflüssigkeit wird Krebs-Henseleit-Bicarbonat-Puffer (pH 7,4), der mit einer Sauerstoff/Kohlendioxid-Mischung (95/5) gesättigt ist und 1,6 mmol/l L-Lactate und 0,2 mmol/l Pyruvat enthält, verwandt. Die Perfusionsflüssigkeit wird in die Leber über eine in die Pfortader eingeführte Kanüle gepumpt. Die austretende Perfusionsflüssigkeit wird über eine in die Vena cava eingeführte Kanüle gesammelt. Die Leber wird ca. 2 Stunden perfundiert. Die Testverbindungen werden von der 32. bis 80. Minute der Perfusion in ansteigenden Konzentrationen (0.02–1.00 mmol/l) je 16 Min. infundiert.

Proben der Austrittsperfusionsflüssigkeit werden in Einminutenintervallen gesammelt und auf Glucose, Lactat und Pyruvat nach enzymatischen Standardmethoden analysiert. Die in Tabelle V

angegebenen Prozentwerte beziehen sich auf den vor und nach Zugabe der Verbindungen vorliegenden Zustand, wobei die allein durch Lactat und Pyruvat bedingten Veränderungen gleich 100% gesetzt wurden.

Bestimmung der Toxizität

Die Toxizitätsuntersuchungen werden an weiblichen NMRI-Mäusen (Körpergewicht 22–26 g) durchgeführt. Die Tiere (5 Tiere pro Dosis) erhalten Futter und Wasser ad libitum. Verschiedene Dosierungen der Substanzen werden intraperitoneal verabreicht. Die Beobachtungsdauer beträgt 14 Tage. Die $DL_{50}$, d. h. die Dosis, bei der 50% der Tiere sterben, wird aus der Dosiswirkungskurve graphisch ermittelt.

**Patentansprüche für die Vertragstaaten: BE, CH, DE, FR, GB, IT, LU, NL, SE**

1. Acyl(bicyclisches aryl)aminoalkansäuren der allgemeinen Formel I

$$R^1-CO-\underset{\displaystyle A}{\underset{|}{N}}-C_nH_{2n}-COOH \qquad (I)$$

worin

$R^1$ einen aliphatischen oder alicyclischen Kohlenwasserstoffrest oder eine Phenylgruppe

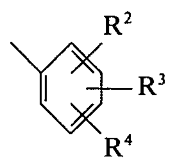

A einen gegebenenfalls teilweise hydrierten bicyclischen Arylrest mit 8 bis 12 Kohlenstoffatomen, der gegebenenfalls ein- oder mehrfach durch Halogenatome, Alkyl- oder Alkoxygruppen mit 1 bis 4 Kohlenstoffatomen, gegebenenfalls substituierte Aminogruppen, Hydroxy-, Nitro- oder Trifluormethylgruppen substituiert ist,

$R^2$, $R^3$ und $R^4$ gleich oder verschieden sind und ein Wasserstoffatom, ein Halogenatom, eine Alkylgruppe, eine Hydroxygruppe, eine Alkoxygruppe, eine Acyloxygruppe, eine gegebenenfalls substituierte Aminogruppe, eine Nitrogruppe oder eine Trifluormethylgruppe und

n 3, 4 oder 5 bedeuten,

und ihre Salze mit anorganischen oder organischen Basen.

2. Acyl(bicyclisches aryl)aminoalkansäuren nach Anspruch 1, gekennzeichnet durch die allgemeine Formel I*

$$R^{1*}-CO-\underset{\displaystyle A^*}{\underset{|}{N}}-C_{n*}H_{2n*}-COOH \qquad (I^*)$$

worin

$R^{1*}$ einen aliphatischen Kohlenwasserstoffrest mit 1 bis 5 Kohlenstoffatomen, einen alicyclischen Kohlenwasserstoffrest mit 5 bis 7 Kohlenstoffatomen oder einen Phenylrest

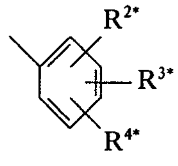

A* einen durch $R^{5*}$, $R^{6*}$ substituierten Indenyl-, Indanyl-, Naphthyl-, Dihydronaphthyl- oder Tetrahydronaphthylrest,

n* 3, 4 oder 5 bedeuten,

$R^{2*}$, $R^{3*}$ und $R^{4*}$ gleich oder verschieden sind und ein Wasserstoffatom, ein Halogenatom, eine

Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Nitrogruppe, eine Hydroxygruppe oder eine Trifluormethylgruppe bedeuten,

$R^{5*}$ und $R^{6*}$ gleich oder verschieden sind und ein Wasserstoffatom, ein Halogenatom, eine Methylgruppe, eine Alkoxygruppe mit 1 bis 4 Kohlenstoffatomen, eine Nitrogruppe oder eine Trifluormethylgruppe darstellen,

und ihre Salze mit anorganischen oder organischen Basen.

3. Verbindungen nach Anspruch 2, in denen $A^*$ einen durch $R^{5*}$ und $R^{6*}$ substituierten 5-Indanyl-, 1-Naphthyl- oder 1,2,3,4-Tetrahydronaphthyl-(1)-rest darstellt.

4. Acyl(bicyclisches aryl)aminoalkansäuren nach Anspruch 1, gekennzeichnet durch die allgemeine Formel I**

$$R^{1**}-CO-\underset{\displaystyle A^{**}}{\underset{|}{N}}-C_{n^{**}}H_{2n^{**}}-COOH \qquad (I^{**})$$

worin

$R^{1**}$ einen aliphatischen Kohlenwasserstoffrest mit 1 bis 5 Kohlenstoffatomen oder einen durch $R^{2**}$, $R^{3**}$ und $R^{4**}$ substituierten Phenylrest,

$A^{**}$ eine Gruppe der Formel

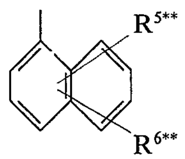

$n^{**}$ 3, 4 oder 5 bedeuten,

$R^{2**}$, $R^{3**}$ und $R^{4**}$ gleich oder verschieden sind und ein Wasserstoffatom, ein Halogenatom, eine Methylgruppe, eine Methoxygruppe, eine Nitrogruppe, eine Hydroxygruppe oder eine Trifluormethylgruppe darstellen,

$R^{5**}$ und $R^{6**}$ gleich oder verschieden sind und ein Wasserstoffatom, ein Chloratom, eine Ethoxygruppe, eine Methoxygruppe oder eine Nitrogruppe darstellen,

und ihre Salze mit anorganischen oder organischen Basen.

5. Acyl(bicyclisches aryl)aminoalkansäuren nach Anspruch 1, gekennzeichnet durch die allgemeine Formel I***

$$R^{1***}-CO-\underset{\displaystyle A^{***}}{\underset{|}{N}}-C_{n^{***}}H_{2n^{***}}-COOH \qquad (I^{***})$$

worin

$R^{1***}$ einen aliphatischen Kohlenwasserstoffrest mit 1 bis 5 Kohlenstoffatomen oder einen durch $R^{2***}$, $R^{3***}$, $R^{4***}$ substituierten Phenylrest,

$A^{***}$ eine Gruppe der Formel

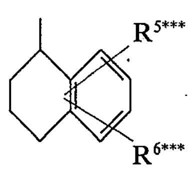

$n^{***}$ 3, 4 oder 5 bedeuten,

$R^{2***}$, $R^{3***}$ und $R^{4***}$ gleich oder verschieden sind und ein Wasserstoffatom, ein Halogenatom, eine Methylgruppe, eine Methoxygruppe, eine Nitrogruppe, eine Hydroxygruppe oder eine Trifluormethylgruppe darstellen,

$R^{5***}$ und $R^{6***}$ gleich oder verschieden sind und ein Wasserstoffatom, ein Chloratom, eine Methoxygruppe oder eine Nitrogruppe darstellen,

und ihre Salze mit anorganischen oder organischen Basen.

6. Acyl(bicyclisches aryl)aminoalkansäuren nach Anspruch 1, gekennzeichnet durch die allgemeine Formel I****

$$R^{1****}—CO—\underset{\underset{\displaystyle A^{****}}{|}}{N}—C_{n^{****}}H_{2n^{****}}—COOH \qquad (I^{****})$$

worin

$R^{1****}$ einen aliphatischen Kohlenwasserstoffrest mit 1 bis 5 Kohlenstoffatomen oder einen durch $R^{2****}$, $R^{3****}$, $R^{4****}$ substituierten Phenylrest,
$A^{****}$ eine Gruppe der Formel

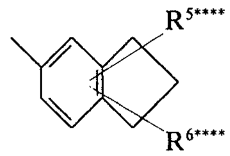

$n^{****}$ 3, 4 oder 5 bedeuten,
$R^{2****}$, $R^{3****}$ und $R^{4****}$ gleich oder verschieden sind und ein Wasserstoffatom, ein Halogenatom, eine Methylgruppe, eine Methoxygruppe, eine Nitrogruppe, eine Hydroxygruppe oder eine Trifluormethylgruppe darstellen,
$R^{5****}$ und $R^{6****}$ gleich oder verschieden sind und ein Wasserstoffatom, ein Chloratom, eine Methoxygruppe oder eine Nitrogruppe darstellen,

und ihre Salze mit anorganischen oder organischen Basen.

7. Verbindungen nach Anspruch 4 oder 5 oder 6, in denen $R^{1**}$, $R^{1***}$ bzw. $R^{1****}$ eine Alkylgruppe mit 1 bis 3 Kohlenstoffatomen oder einen substituierten Phenylrest; $R^{2**}$, $R^{2***}$ bzw. $R^{2****}$ ein Wasserstoffatom; $R^{3**}$, $R^{3***}$ bzw. $R^{3****}$ ein Wasserstoffatom, ein Chloratom, eine Methoxygruppe, eine Hydroxygruppe oder eine Trifluormethylgruppe; $R^{4**}$, $R^{4***}$ bzw. $R^{4****}$ ein Wasserstoffatom oder ein Chloratom; $R^{5**}$, $R^{5***}$ bzw. $R^{5****}$ ein Wasserstoffatom oder eine Methoxygruppe und $R^{6**}$, $R^{6***}$ bzw. $R^{6****}$ ein Wasserstoffatom darstellen und $n^{**}$, $n^{***}$ bzw. $n^{****}$ die oben angegebene Bedeutung haben, und ihre pharmakologisch verträglichen Salze mit anorganischen oder organischen Basen.

8. Verbindungen nach Anspruch 4 oder 5 oder 6, in denen $R^{1**}$, $R^{1***}$ bzw. $R^{1****}$ eine Alkylgruppe mit 1 bis 3 Kohlenstoffatomen oder einen substituierten Phenylrest; $R^{2**}$, $R^{2***}$ bzw. $R^{2****}$ ein Wasserstoffatom; $R^{3**}$, $R^{3***}$ bzw. $R^{3****}$ ein Chloratom oder eine Hydroxygruppe; $R^{4**}$, $R^{4***}$ bzw. $R^{4****}$ ein Wasserstoffatom oder ein Chloratom; $R^{5**}$, $R^{5***}$ bzw. $R^{5****}$ ein Wasserstoffatom oder eine Methoxygruppe und $R^{6**}$, $R^{6***}$ bzw. $R^{6****}$ ein Wasserstoffatom darstellen und $n^{**}$, $n^{***}$ bzw. $n^{****}$ die oben angegebene Bedeutung haben, und ihre pharmakologisch verträglichen Salze mit anorganischen oder organischen Basen.

9. Verbindungen nach Anspruch 4 oder 5, in denen $R^{1**}$ bzw. $R^{1***}$ eine Alkylgruppe mit 1 bis 3 Kohlenstoffatomen oder einen substituierten Phenylrest; $R^{2**}$ bzw. $R^{2***}$ ein Wasserstoffatom; $R^{3**}$ bzw. $R^{3***}$ ein Chloratom oder eine Hydroxygruppe; $R^{4**}$ bzw. $R^{4***}$ ein Wasserstoffatom; $R^{5**}$ bzw. $R^{5***}$ ein Wasserstoffatom oder eine Methoxygruppe und $R^{6**}$ bzw. $R^{6***}$ ein Wasserstoffatom darstellen und $n^{**}$ bzw. $n^{***}$ 3 oder 4 bedeuten, und ihre pharmakologisch verträglichen Salze mit anorganischen oder organischen Basen.

10. Arzneimittel, enthaltend eine oder mehrere Verbindungen nach einem oder mehreren der Ansprüche 1 bis 9 und/oder ihre pharmakologisch verträglichen Salze mit anorganischen oder organischen Basen.

11. Verfahren zur Herstellung der Acyl(bicyclisches Aryl)aminoalkansäuren der allgemeinen Formel I

$$R^1—CO—\underset{\underset{\displaystyle A}{|}}{N}—C_nH_{2n}—COOH \qquad (I)$$

sowie ihrer Salze mit anorganischen oder organischen Basen, dadurch gekennzeichnet, daß man

a) eine (bicyclische Aryl)aminoalkansäure der allgemeinen Formel II

$$A—NH—C_nH_{2n}—COOH \qquad (II)$$

gegebenenfalls unter Schutz der Carboxylgruppe, mit einem Acylderivat der allgemeinen Formel III

$$R^1—CO—R^7 \qquad (III)$$

worin $R^7$ eine Fluchtgruppe oder eine $R^1-CO-O$-Gruppe darstellt, acyliert und gewünschtenfalls anschließend in die Salze überführt oder

b) eine (bicyclisches Aryl)aminoalkensäure der allgemeinen Formel IV

$$R^1-CO-\underset{\displaystyle A}{\underset{|}{N}}-C_nH_{2n-2}-COOH \qquad (IV)$$

gegebenenfalls unter Schutz der Carboxylgruppe, hydriert und gewünschtenfalls anschließend in die Salze überführt oder

c) ein funktionelles Acyl(bicyclisches Aryl)aminoalkansäurederivat der allgemeinen Formel V

$$R^1-CO-\underset{\displaystyle A}{\underset{|}{N}}-C_nH_{2n}-G \qquad (V)$$

worin G ein funktionelles Derivat einer Carboxylgruppe bedeutet, solvolysiert und gewünschtenfalls anschließend in die Salze überführt, wobei die Substituenten $R^1$, A und n der Verbindungen I–V die in Anspruch 1 angegebenen Bedeutungen haben.

12. Verfahren nach Anspruch 11 zur Herstellung der Acyl(bicyclisches Aryl)aminoalkansäuren der allgemeinen Formel I*

$$R^{1*}-CO-\underset{\displaystyle A^*}{\underset{|}{N}}-C_{n^*}H_{2n^*}-COOH \qquad (I^*)$$

sowie ihrer Salze mit anorganischen oder organischen Basen, dadurch gekennzeichnet, daß man

a) eine (bicyclisches Aryl)aminoalkansäure der allgemeinen Formel II*

$$A^*-NH-C_{n^*}H_{2n^*}-COOH \qquad (II^*)$$

gegebenenfalls unter Schutz der Carboxylgruppe, mit einem Acylderivat der allgemeinen Formel III*

$$R^{1*}-CO-R^7 \qquad (III^*)$$

worin $R^{7*}$ eine Fluchtgruppe oder eine $R^{1*}-CO-O$-Gruppe darstellt, acyliert und gewünschtenfalls anschließend in die Salze überführt oder

b) eine (bicyclisches Aryl)aminoalkensäure der allgemeinen Formel IV*

$$R^{1*}-CO-\underset{\displaystyle A^*}{\underset{|}{N}}-C_{n^*}H_{2n^*-2}-COOH \qquad (IV^*)$$

gegebenenfalls unter Schutz der Carboxylgruppe, hydriert und gewünschtenfalls anschließend in die Salze überführt oder

c) ein funktionelles Acyl(bicyclisches Aryl)aminoalkansäurederivat der allgemeinen Formel V*

$$R^{1*}-CO-\underset{\displaystyle A^*}{\underset{|}{N}}-C_{n^*}H_{2n^*}-G^* \qquad (V^*)$$

worin G* ein funktionelles Derivat einer Carboxylgruppe bedeutet, solvolysiert und gewünschtenfalls anschließend in die Salze überführt, wobei die Substituenten $R^{1*}$, A* und n* der Verbindungen I*–V* die in Anspruch 2 angegebenen Bedeutungen haben.

13. Verfahren nach Anspruch 11 zur Herstellung der Acyl(bicyclisches Aryl)aminoalkansäuren der allgemeinen Formel I**

$$R^{1**}-CO-\underset{\displaystyle A^{**}}{\underset{|}{N}}-C_{n^{**}}H_{2n^{**}}-COOH \qquad (I^{**})$$

sowie ihrer Salze mit anorganischen oder organischen Basen, dadurch gekennzeichnet, daß man

a) eine (bicyclisches Aryl)aminoalkansäure der allgemeinen Formel II**

$$A^{**}-NH-C_{n^{**}}H_{2n^{**}}-COOH \qquad (II^{**})$$

gegebenenfalls unter Schutz der Carboxylgruppe, mit einem Acylderivat der allgemeinen Formel III**

$$R^{1**}-CO-R^{7**} \qquad (III^{**})$$

worin $R^{7**}$ eine Fluchtgruppe oder eine $R^{1**}-CO-O$-Gruppe darstellt, acyliert und gewünschtenfalls anschließend in die Salze überführt oder

b) eine (bicyclisches Aryl)aminoalkensäure der allgemeinen Formel IV**

$$R^{1**}-CO-\underset{\substack{|\\A^{**}}}{N}-C_{n^{**}}H_{2n^{**}-2}-COOH \qquad (IV^{**})$$

gegebenenfalls unter Schutz der Carboxylgruppe, hydriert und gewünschtenfalls anschließend in die Salze überführt oder

c) ein funktionelles Acyl(bicyclisches Aryl)aminoalkansäurederivat der allgemeinen Formel V**

$$R^{1**}-CO-\underset{\substack{|\\A^{**}}}{N}-C_{n^{**}}H_{2n^{**}}-G^{**} \qquad (V^{**})$$

worin G** ein funktionelles Derivat einer Carboxylgruppe bedeutet, solvolysiert und gewünschtenfalls anschließend in die Salze überführt, wobei die Substituenten $R^{1**}$, A** und n** der Verbindungen I**–V** die in Anspruch 4 angegebenen Bedeutungen haben.

14. Verfahren nach Anspruch 11 zur Herstellung der Acyl(bicyclisches Aryl)aminoalkansäuren der allgemeinen Formel I***

$$R^{1***}-CO-\underset{\substack{|\\A^{***}}}{N}-C_{n^{***}}H_{2n^{***}}-COOH \qquad (I^{***})$$

sowie ihrer Salze mit anorganischen oder organischen Basen, dadurch gekennzeichnet, daß man

a) eine (bicyclisches Aryl)aminoalkansäure der allgemeinen Formel II**

$$A^{***}-NH-C_{n^{***}}H_{2n^{***}}-COOH \qquad (II^{***})$$

gegebenenfalls unter Schutz der Carboxylgruppe, mit einem Acylderivat der allgemeinen Formel III***

$$R^{1***}-CO-R^{7***} \qquad (III^{***})$$

wobei $R^{7***}$ eine Fluchtgruppe oder eine $R^{1***}-CO-O$-Gruppe darstellt, acyliert und gewünschtenfalls anschließend in die Salze überführt oder

b) eine (bicyclisches Aryl)aminoalkansäure der allgemeinen Formel IV***

$$R^{1***}-CO-\underset{\substack{|\\A^{***}}}{N}-C_{n^{***}}H_{2n^{***}-2}-COOH \qquad (IV^{***})$$

gegebenenfalls unter Schutz der Carboxylgruppe, hydriert und gewünschtenfalls anschließend in die Salze überführt oder

c) ein funktionelles Acyl(bicyclisches Aryl)aminoalkansäurederivat der allgemeinen Formel V***

$$R^{1***}—CO—N(A^{***})—C_{n***}H_{2n***}—G^{***} \quad (V^{***})$$

worin G*** ein funktionelles Derivat einer Carboxylgruppe bedeutet, solvolysiert und gewünschtenfalls anschließend in die Salze überführt, wobei die Substituenten $R^{1***}$, A*** und n*** der Verbindungen I***–V*** die in Anspruch 5 angegebenen Bedeutungen haben.

15. Verfahren nach Anspruch 11 zur Herstellung der Acyl(bicyclisches Aryl)aminoalkansäuren der allgemeinen Formel I****

$$R^{1****}—CO—N(A^{****})—C_{n****}H_{2n****}—COOH \quad (I^{****})$$

sowie ihrer Salze mit anorganischen oder organischen Basen, dadurch gekennzeichnet, daß man

a) eine (bicyclisches Aryl)aminoalkansäure der allgemeinen Formel II****

$$A^{****}—NH—C_{n****}H_{2n****}—COOH \quad (II^{****})$$

gegebenenfalls unter Schutz der Carboxylgruppe, mit einem Acylderivat der allgemeinen Formel III***

$$R^{1****}—CO—R^{7****} \quad (III^{****})$$

worin $R^{7****}$ eine Fluchtgruppe oder eine $R^{1****}$–CO–O-Gruppe darstellt, acyliert und gewünschtenfalls anschließend in die Salze überführt oder

b) eine (bicyclisches Aryl)aminoalkensäure der allgemeinen Formel IV****

$$R^{1****}—CO—N(A^{****})—C_{n****}H_{2n****-2}—COOH \quad (IV^{****})$$

gegebenenfalls unter Schutz der Carboxylgruppe hydriert und gewünschtenfalls anschließend in die Salze überführt oder

c) ein funktionelles Acyl(bicyclisches Aryl)aminoalkansäurederivat der allgemeinen Formel V****

$$R^{1****}—CO—N(A^{****})—C_{n****}H_{2n****}—G^{****} \quad (V^{****})$$

worin G**** ein funktionelles Derivat einer Carboxylgruppe bedeutet, solvolysiert und gewünschtenfalls anschließend in die Salze überführt, wobei die Substituenten $R^{1****}$, A**** und n**** der Verbindungen I****–V**** die in Anspruch 6 angegebenen Bedeutungen haben.

**16. Verbindungen gemäß einem oder mehreren der Ansprüche 1 bis 9 zur Behandlung und Prophylaxe von Krankheiten, die auf Erkrankungen des Magens oder Darms oder auf Minderleistungen von Pankreas, Galle und/oder Leber beruhen.**

**Patentansprüche für den Vertragsstaat: AT**

1. Verfahren zur Herstellung der Acyl(bicyclisches aryl)aminoalkansäuren der allgemeinen Formel I

$$R^1—CO—N(A)—C_nH_{2n}—COOH \quad (I)$$

worin

$R^1$ einen aliphatischen oder alicyclischen Kohlenwasserstoffrest oder eine Phenylgruppe

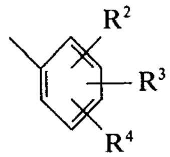

A einen gegebenenfalls teilweise hydrierten bicyclischen Arylrest mit 8 bis 12 Kohlenstoffatomen, der gegebenenfalls ein- oder mehrfach durch Halogenatome, Alkyl- oder Alkoxygruppen mit 1 bis 4 Kohlenstoffatomen, gegebenenfalls substituierte Aminogruppen, Hydroxy-, Nitro- oder Trifluormethylgruppen substituiert ist,

$R^2$, $R^3$ und $R^4$ gleich oder verschieden sind und ein Wasserstoffatom, ein Halogenatom, eine Alkylgruppe, eine Hydroxygruppe, eine Alkoxygruppe, eine Acyloxygruppe, eine gegebenenfalls substituierte Aminogruppe, eine Nitrogruppe oder eine Trifluormethylgruppe und

n 3, 4 oder 5 bedeuten,

und ihrer Salze mit anorganischen oder organischen Basen, dadurch gekennzeichnet, daß man

a) eine (bicyclische Aryl)aminoalkansäure der allgemeinen Formel II

$$A-NH-C_nH_{2n}-COOH \qquad (II)$$

worin A und n die vorstehend angegebene Bedeutung haben, gegebenenfalls unter Schutz der Carboxylgruppe, mit einem Acylderivat der allgemeinen Formel III

$$R^1-CO-R^7 \qquad (III)$$

worin $R^7$ eine Fluchtgruppe oder eine $R^1-CO-O$-Gruppe darstellt und $R^1$ die oben angegebene Bedeutung hat, acyliert und gewünschtenfalls anschließend in die Salze überführt oder

b) eine (bicyclisches Aryl)aminoalkansäure der allgemeinen Formel IV

$$R^1-CO-\underset{\displaystyle A}{\underset{|}{N}}-C_nH_{2n-2}-COOH \qquad (IV)$$

worin $R^1$, A und n die vorstehend angegebene Bedeutung haben, gegebenenfalls unter Schutz der Carboxylgruppe, hydriert und gewünschtenfalls anschließend in die Salze überführt oder

c) ein funktionelles Acyl(bicyclisches Aryl)aminoalkansäurederivat der allgemeinen Formel V

$$R^1-CO-\underset{\displaystyle A}{\underset{|}{N}}-C_nH_{2n}-G \qquad (V)$$

worin $R^1$, A und n die oben angegebene Bedeutung haben und G ein funktionelles Derivat einer Carboxylgruppe bedeutet, solvolysiert und gewünschtenfalls anschließend in die Salze überführt.

2. Verfahren nach Anspruch 1 zur Herstellung der Acyl(bicyclisches Aryl)aminoalkansäuren der allgemeinen Formel I*

$$R^{1*}-CO-\underset{\displaystyle A^*}{\underset{|}{N}}-C_{n^*}H_{2n^*}-COOH \qquad (I^*)$$

worin

$R^{1*}$ einen aliphatischen Kohlenwasserstoffrest mit 1 bis 5 Kohlenstoffatomen, einen alicyclischen Kohlenwasserstoffrest mit 5 bis 7 Kohlenstoffatomen oder einen Phenylrest

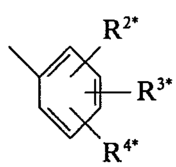

A* einen durch $R^{5*}$, $R^{6*}$ substituierten Indenyl-, Indanyl-, Naphthyl-, Dihydronaphthyl- oder Tetrahydronaphthylrest,

n* 3, 4 oder 5 bedeuten,

$R^{2*}$, $R^{3*}$ und $R^{4*}$ gleich oder verschieden sind und ein Wasserstoffatom, ein Halogenatom, eine Alkylgruppe mit 1 bis 4 Kohlenstoffatomen, eine Alkoxygruppe mit 1 bis 4 Kohlenstoffatomen, eine Alkanoyloxygruppe mit 2 bis 5 Kohlenstoffatomen, eine Nitrogruppe, eine Hydroxygruppe oder eine Trifluormethylgruppe bedeuten,

$R^{5*}$ und $R^{6*}$ gleich oder verschieden sind und ein Wasserstoffatom, ein Halogenatom, eine Methylgruppe, eine Alkoxygruppe mit 1 bis 4 Kohlenstoffatomen, eine Nitrogruppe oder eine Trifluormethylgruppe darstellen,

sowie ihrer Salze mit anorganischen oder organischen Basen, dadurch gekennzeichnet, daß man

a) eine (bicyclisches Aryl)aminoalkansäure der allgemeinen Formel II*

$$A^*-NH-C_{n^*}H_{2n^*}-COOH \qquad (II^*)$$

worin A* und n* die vorstehend angegebene Bedeutung haben, gegebenenfalls unter Schutz der Carboxylgruppe, mit einem Acylderivat der allgemeinen Formel III*

$$R^{1*}-CO-R^{7*} \qquad (III^*)$$

worin $R^{7*}$ eine Fluchtgruppe oder eine $R^{1*}-CO-O$-Gruppe darstellt und $R^{1*}$ die oben angegebene Bedeutung hat, acyliert und gewünschtenfalls anschließend in die Salze überführt oder

b) eine (bicyclisches Aryl)aminoalkansäure der allgemeinen Formel IV*

$$R^{1*}-CO-\underset{\displaystyle A^*}{\underset{|}{N}}-C_{n^*}H_{2n^*-2}-COOH \qquad (IV^*)$$

worin $R^{1*}$, A* und n* die vorstehend angegebene Bedeutung haben, gegebenenfalls unter Schutz der Carboxylgruppe, hydriert und gewünschtenfalls anschließend in die Salze überführt oder

c) ein funktionelles Acyl(bicyclisches Aryl)aminoalkansäurederivat der allgemeinen Formel V*

$$R^{1*}-CO-\underset{\displaystyle A^*}{\underset{|}{N}}-C_{n^*}H_{2n^*}-G^* \qquad (V^*)$$

worin $R^{1*}$, A* und n* die oben angegebene Bedeutung haben und G* ein funktionelles Derivat einer Carboxylgruppe bedeutet, solvolysiert und gewünschtenfalls anschließend in die Salze überführt.

3. Verfahren nach Anspruch 2, wobei A* einen durch $R^{5*}$ und $R^{6*}$ substituierten 5-Indanyl-, 1-Naphthyl- oder 1,2,3,4-Tetrahydronaphthyl-(1)-rest darstellt.

4. Verfahren nach Anspruch 1 zur Herstellung der Acyl(bicyclisches Aryl)aminoalkansäuren der allgemeinen Formel I**

$$R^{1**}-CO-\underset{\displaystyle A^{**}}{\underset{|}{N}}-C_{n^{**}}H_{2n^{**}}-COOH \qquad (I^{**})$$

worin

$R^{1**}$ einen aliphatischen Kohlenwasserstoffrest mit 1 bis 5 Kohlenstoffatomen oder einen durch $R^{2**}$, $R^{3**}$ und $R^{4**}$ substituierten Phenylrest,

A** eine Gruppe der Formel

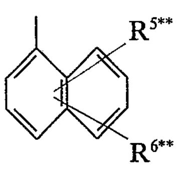

n** 3, 4 oder 5 bedeuten,

$R^{2**}$, $R^{3**}$ und $R^{4**}$ gleich oder verschieden sind und ein Wasserstoffatom, ein Halogenatom, eine

Methylgruppe, eine Methoxygruppe, eine Nitrogruppe, eine Hydroxygruppe oder eine Trifluormethylgruppe darstellen,

$R^{5**}$ und $R^{6**}$ gleich oder verschieden sind und ein Wasserstoffatom, ein Chloratom, eine Ethoxygruppe, eine Methoxygruppe oder eine Nitrogruppe darstellen,

sowie ihrer Salze mit anorganischen oder organischen Basen, dadurch gekennzeichnet, daß man

a) eine (bicyclisches Aryl)aminoalkansäure der allgemeinen Formel II**

$$A^{**}—NH—C_{n^{**}}H_{2n^{**}}—COOH \quad (II^{**})$$

worin A** und n** die vorstehend angegebene Bedeutung haben, gegebenenfalls unter Schutz der Carboxylgruppe, mit einem Acylderivat der allgemeinen Formel III**

$$R^{1**}—CO—R^{7**} \quad (III^{**})$$

worin $R^{7**}$ eine Fluchtgruppe oder eine $R^{1**}-CO-O$-Gruppe darstellt und $R^{1**}$ die oben angegebene Bedeutung hat, acyliert und gewünschtenfalls anschließend in die Salze überführt oder

b) eine (bicyclisches Aryl)aminoalkansäure der allgemeinen Formel IV**

$$R^{1**}—CO—\underset{\displaystyle A^{**}}{\underset{|}{N}}—C_{n^{**}}H_{2n^{**}-2}—COOH \quad (IV^{**})$$

worin $R^{1**}$, A** und n** die vorstehend angegebene Bedeutung haben, gegebenenfalls unter Schutz der Carboxylgruppe, hydriert und gewünschtenfalls anschließend in die Salze überführt oder

c) ein funktionelles Acyl(bicyclisches Aryl)aminoalkansäurederivat der allgemeinen Formel V**

$$R^{1**}—CO—\underset{\displaystyle A^{**}}{\underset{|}{N}}—C_{n^{**}}H_{2n^{**}}—G^{**} \quad (V^{**})$$

worin $R^{1**}$, A** und n** die oben angegebene Bedeutung haben und G** ein funktionelles Derivat einer Carboxylgruppe bedeutet, solvolysiert und gewünschtenfalls anschließend in die Salze überführt.

5. Verfahren nach Anspruch 1 zur Herstellung der Acyl(bicyclisches Aryl)aminoalkansäuren der allgemeinen Formel I***

$$R^{1***}—CO—\underset{\displaystyle A^{***}}{\underset{|}{N}}—C_{n^{***}}H_{2n^{***}}—COOH \quad (I^{***})$$

worin

$R^{1***}$ einen aliphatischen Kohlenwasserstoffrest mit 1 bis 5 Kohlenstoffatomen oder einen durch $R^{2***}$, $R^{3***}$, $R^{4***}$ substituierten Phenylrest,

A*** eine Gruppe der Formel

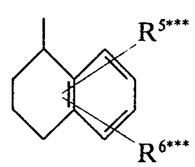

n*** 3, 4 oder 5 bedeuten,

$R^{2***}$, $R^{3***}$ und $R^{4***}$ gleich oder verschieden sind und ein Wasserstoffatom, ein Halogenatom, eine Methylgruppe, eine Methoxygruppe, eine Nitrogruppe, eine Hydroxygruppe oder eine Trifluormethylgruppe darstellen,

$R^{5***}$ und $R^{6***}$ gleich oder verschieden sind und ein Wasserstoffatom, ein Chloratom, eine Methoxygruppe oder eine Nitrogruppe darstellen,

sowie ihrer Salze mit anorganischen oder organischen Basen, dadurch gekennzeichnet, daß man

a) eine (bicyclisches Aryl)aminoalkansäure der allgemeinen Formel II***

$$A^{***}—NH—C_{n^{***}}H_{2n^{***}}—COOH \qquad (II^{***})$$

worin A*** und n*** die vorstehend angegebene Bedeutung haben, gegebenenfalls unter Schutz der Carboxylgruppe, mit einem Acylderivat der allgemeinen Formel III***

$$R^{1***}—CO—R^{7***} \qquad (III^{***})$$

worin $R^{7***}$ eine Fluchtgruppe oder eine $R^{1***}-CO-O$-Gruppe darstellt und $R^{1***}$ die oben angegebene Bedeutung hat, acyliert und gewünschtenfalls anschließend in die Salze überführt oder

b) eine (bicyclisches Aryl)aminoalkensäure der allgemeinen Formel IV***

$$R^{1***}—CO—\underset{\displaystyle A^{***}}{\underset{|}{N}}—C_{n^{***}}H_{2n^{***}-2}—COOH \qquad (IV^{***})$$

worin $R^{1***}$, A*** und n*** die vorstehend angegebene Bedeutung haben, gegebenenfalls unter Schutz der Carboxylgruppe, hydriert und gewünschtenfalls anschließend in die Salze überführt oder

c) ein funktionelles Acyl(bicyclisches Aryl)aminoalkansäurederivat der allgemeinen Formel V***

$$R^{1***}—CO—\underset{\displaystyle A^{***}}{\underset{|}{N}}—C_{n^{***}}H_{2n^{***}}—G^{***} \qquad (V^{***})$$

worin $R^{1***}$, A*** und n*** die oben angegebene Bedeutung haben und G*** ein funktionelles Derivat einer Carboxylgruppe bedeutet, solvolysiert und gewünschtenfalls anschließend in die Salze überführt.

6. Verfahren nach Anspruch 1 zur Herstellung der Acyl(bicyclisches Aryl)aminoalkansäuren der allgemeinen Formel I****

$$R^{1****}—CO—\underset{\displaystyle A^{****}}{\underset{|}{N}}—C_{n^{****}}H_{2n^{****}}—COOH \qquad (I^{****})$$

worin

$R^{1****}$ einen aliphatischen Kohlenwasserstoffrest mit 1 bis 5 Kohlenstoffatomen oder einen durch $R^{2****}$, $R^{3****}$, $R^{4****}$ substituierten Phenylrest,

A**** eine Gruppe der Formel

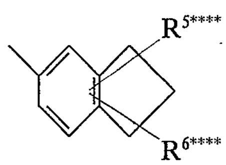

n**** 3, 4 oder 5 bedeuten,

$R^{2****}$, $R^{3****}$ und $R^{4****}$ gleich oder verschieden sind und ein Wasserstoffatom, ein Halogenatom, eine Methylgruppe, eine Methoxygruppe, eine Nitrogruppe, eine Hydroxygruppe oder eine Trifluormethylgruppe darstellen,

$R^{5****}$ und $R^{6****}$ gleich oder verschieden sind und ein Wasserstoffatom, ein Chloratom, eine Methoxygruppe oder eine Nitrogruppe darstellen,

sowie ihrer Salze mit anorganischen oder organischen Basen, dadurch gekennzeichnet, daß man

a) eine (bicyclisches Aryl)aminoalkansäure der allgemeinen Formel II****

$$A^{****}—NH—C_{n^{****}}H_{2n^{****}}—COOH \qquad (II^{****})$$

worin A**** und n**** die vorstehend angegebene Bedeutung haben, gegebenenfalls unter Schutz der Carboxylgruppe, mit einem Acylderivat der allgemeinen Formel III****

$$R^{1****}—CO—R^{7****} \qquad (III^{****})$$

worin $R^{7****}$ eine Fluchtgruppe oder eine $R^{1****}-CO-O$-Gruppe darstellt und $R^{1****}$ die oben angegebene Bedeutung hat, acyliert und gewünschtenfalls anschließend in die Salze überführt oder

b) eine (bicyclisches Aryl)aminoalkensäure der allgemeinen Formel $IV^{****}$

$$R^{1****}—CO—\underset{\displaystyle A^{****}}{\underset{|}{N}}—C_{n^{****}}H_{2n^{****}-2}—COOH \qquad (IV^{****})$$

worin $R^{1****}$, $A^{****}$ und $n^{****}$ die vorstehend angegebene Bedeutung haben, gegebenenfalls unter Schutz der Carboxylgruppe hydriert und gewünschtenfalls anschließend in die Salze überführt oder

c) ein funktionelles Acyl(bicyclisches Aryl)aminoalkansäurederivat der allgemeinen Formel $V^{****}$

$$R^{1****}—CO—\underset{\displaystyle A^{****}}{\underset{|}{N}}—C_{n^{****}}H_{2n^{****}}—G^{****} \qquad (V^{****})$$

worin $R^{1****}$, $A^{****}$ und $n^{****}$ die oben angegebene Bedeutung haben und $G^{****}$ ein funktionelles Derivat einer Carboxylgruppe bedeutet, solvolysiert und gewünschtenfalls anschließend in die Salze überführt.

7. Verfahren nach Anspruch 4 oder 5 oder 6, wobei $R^{1**}$, $R^{1***}$ bzw. $R^{1****}$ eine Alkylgruppe mit 1 bis 3 Kohlenstoffatomen oder einen substituierten Phenylrest; $R^{2**}$, $R^{2***}$ bzw. $R^{2****}$ ein Wasserstoffatom; $R^{3**}$, $R^{3***}$ bzw. $R^{3****}$ ein Wasserstoffatom, ein Chloratom, eine Methoxygruppe, eine Hydroxygruppe oder eine Trifluormethylgruppe; $R^{4**}$, $R^{4***}$ bzw. $R^{4****}$ ein Wasserstoffatom oder ein Chloratom; $R^{5**}$, $R^{5***}$ bzw. $R^{5****}$ ein Wasserstoffatom oder eine Methoxygruppe und $R^{6**}$, $R^{6***}$ bzw. $R^{6****}$ ein Wasserstoffatom darstellen und $n^{**}$, $n^{***}$ bzw. $n^{****}$ die dort angegebenen Bedeutungen haben.

8. Verfahren nach Anspruch 4 oder 5 oder 6, wobei $R^{1**}$, $R^{1***}$ bzw. $R^{1****}$ eine Alkylgruppe mit 1 bis 3 Kohlenstoffatomen oder einen substituierten Phenylrest; $R^{2**}$, $R^{2***}$ bzw. $R^{2****}$ ein Wasserstoffatom; $R^{3**}$, $R^{3***}$ bzw. $R^{3****}$ ein Chloratom oder eine Hydroxygruppe, $R^{4**}$, $R^{4***}$ bzw. $R^{4****}$ ein Wasserstoffatom oder ein Chloratom; $R^{5**}$, $R^{5***}$ bzw. $R^{5****}$ ein Wasserstoffatom oder eine Methoxygruppe und $R^{6**}$, $R^{6***}$ bzw. $R^{6****}$ ein Wasserstoffatom darstellen und $n^{**}$, $n^{***}$ bzw. $n^{****}$ die dort angegebenen Bedeutungen haben.

9. Verfahren nach Anspruch 4 oder 5, wobei $R^{1**}$ bzw. $R^{1***}$ eine Alkylgruppe mit 1 bis 3 Kohlenstoffatomen oder einen substituierten Phenylrest; $R^{2**}$ bzw. $R^{2***}$ ein Wasserstoffatom; $R^{3**}$ bzw. $R^{3***}$ ein Chloratom oder eine Hydroxygruppe; $R^{4**}$ bzw. $R^{4***}$ ein Wasserstoffatom; $R^{5**}$ bzw. $R^{5***}$ ein Wasserstoffatom oder eine Methoxygruppe und $R^{6**}$ bzw. $R^{6***}$ ein Wasserstoffatom darstellen und $n^{**}$ bzw. $n^{***}$ 3 oder 4 bedeuten.

10. Verfahren nach einem der Ansprüche 1 bis 9, wobei die Fluchtgruppe $R^7$, $R^{7*}$, $R^{7**}$, $R^{7***}$ bzw. $R^{7****}$ ein Chlor- oder Bromatom und G, $G^*$, $G^{**}$, $G^{***}$ bzw. $G^{****}$ eine Nitril-, Carbonsäureester- oder Carbonsäureamidgruppe darstellt.

**Claims for the Contracting states; BE, CH, DE, FR, GB, IT, LU, NL, SE**

1. Acyl(bicyclic aryl)-aminoalkanoic acids of the general formula I

$$R^1—CO—\underset{\displaystyle A}{\underset{|}{N}}—C_nH_{2n}—COOH \qquad (I)$$

in which

$R^1$ signifies an aliphatic or alicyclic hydrocarbon radical or a phenyl group

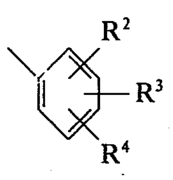

A signifies an optionally partly hydrogenated bicyclic aryl radical with 8 to 12 carbon atoms, optionally substituted by one or more halogen atoms, alkyl or alkoxy groups with 1 to 4 carbon

atoms, optionally substituted amino groups, hydroxy, nitro or trifluoromethyl groups,

$R^2$, $R^3$ and $R^4$ are the same or different and signify a hydrogen atom, a halogen atom, an alkyl group, a hydroxy group, an alkoxy group, an acyloxy group, an optionally substituted amino group, a nitro group or a trifluoromethyl group and

n signifies 3, 4 or 5

and their salts of inorganic or organic bases.

2. Acyl(bicyclic aryl)-aminoalkanoic acids in accordance with claim 1, characterized by the general formula I*

$$R^{1*}-CO-\underset{\displaystyle A^*}{\underset{|}{N}}-C_{n*}H_{2n*}-COOH \qquad (I^*)$$

in which

$R^{1*}$ signifies an aliphatic hydrocarbon radical with 1 to 5 carbon atoms, an alicyclic hydrocarbon radical with 5 to 7 carbon atoms or a phenyl radical

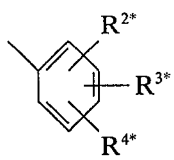

A* signifies an indenyl, indanyl, naphthyl, dihydronaphthyl or tetrahydronaphthyl radical substituted with $R^{5*}$ and $R^{6*}$,

n* signifies 3, 4 or 5,

$R^{2*}$, $R^{3*}$ and $R^{4*}$ are the same or different and signify a hydrogen atom, a halogen atom, an alkyl group with 1 to 4 carbon atoms, an alkoxy group with 1 to 4 carbon atoms, an alkanoyloxy group with 2 to 5 carbon atoms, a nitro group, a hydroxy group or a trifluoromethyl group,

$R^{5*}$ and $R^{6*}$ are the same or different and signify a hydrogen atom, a halogen atom, a methyl group, an alkoxy group with 1 to 4 carbon atoms, a nitro group or a trifluoromethyl group,

and their salts of inorganic or organic bases.

3. Compounds in accordance with claim 2, in which A* represents a 5-indanyl, 1-naphthyl or 1,2,3,4-tetrahydronaphthyl-(1) radical substituted with $R^{5*}$ and $R^{6*}$.

4. Acyl(bicyclic aryl)-aminoalkanoic acids in accordance with claim 1, characterized by the general formula I**

$$R^{1**}-CO-\underset{\displaystyle A^{**}}{\underset{|}{N}}-C_{n**}H_{2n**}-COOH \qquad (I^{**})$$

in which

$R^{1**}$ signifies an aliphatic hydrocarbon radical with 1 to 5 carbon atoms or a phenyl radical substituted with $R^{2**}$, $R^{3**}$ and $R^{4**}$,

A** signifies a group of the formula

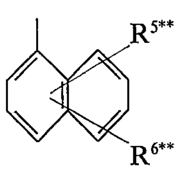

n** signifies 3, 4 or 5,

$R^{2**}$, $R^{3**}$ and $R^{4**}$ are the same or different and signify a hydrogen atom, a halogen atom, a methyl group, a methoxy group, a nitro group, a hydroxy group or a trifluoromethyl group,

$R^{5**}$ and $R^{6**}$ are the same or different and signify a hydrogen atom, a chlorine atom, an ethoxy group, a methoxy group or a nitro group,

and their salts or inorganic or organic bases.

5. Acyl(bicyclic aryl)-aminoalkanoic acids in accordance with claim 1, characterized by the general formula I***

$$R^{1***}-CO-\underset{A^{***}}{\underset{|}{N}}-C_{n^{***}}H_{2n^{***}}-COOH \qquad (I^{***})$$

in which

$R^{1***}$ signifies an aliphatic hydrocarbon radical with 1 to 5 carbon atoms or a phenyl radical substituted with $R^{2***}$, $R^{3***}$ and $R^{4***}$,

$A^{***}$ signifies a group of the formula

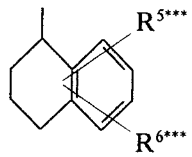

$n^{***}$ signifies 3, 4 or 5,

$R^{2***}$, $R^{3***}$ and $R^{4***}$ are the same or different and signify a hydrogen atom, a halogen atom, a methyl group, a methoxy group, a nitro group, a hydroxy group or a trifluoromethyl group,

$R^{5***}$ and $R^{6***}$ are the same or different and represent a hydrogen atom, a chlorine atom, a methoxy group or a nitro group,

and their salts of inorganic or organic bases.

6. Acyl(bicyclic aryl)-aminoalkanoic acids in accordance with claim 1, characterized by the general formula $I^{****}$

$$R^{1****}-CO-\underset{A^{****}}{\underset{|}{N}}-C_{n^{****}}H_{2n^{****}}-COOH \qquad (I^{****})$$

in which

$R^{1****}$ signifies an aliphatic hydrocarbon radical with 1 to 5 carbon atoms or a phenyl radical substituted with $R^{2****}$, $R^{3****}$ and $R^{4****}$,

$A^{****}$ signifies a group of the formula

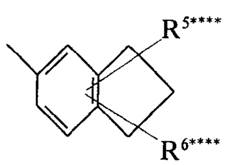

$n^{****}$ signifies 3, 4 or 5,

$R^{2****}$, $R^{3****}$ and $R^{4****}$ are the same or different and represent a hydrogen atom, a halogen atom, a methyl group, a methoxy group, a nitro group, a hydroxy group or a trifluoromethyl group,

$R^{5****}$ and $R^{6****}$ are the same or different and represent a hydrogen atom, a chlorine atom, a methoxy group or a nitro group,

and their salts of inorganic or organic bases.

7. Compounds in accordance with claims 4 or 5 or 6, in which $R^{1**}$, $R^{1***}$ or $R^{1****}$ represent an alkyl group with 1 to 3 carbon atoms or a substituted phenyl radical; $R^{2**}$, $R^{2***}$ or $R^{2****}$ signify a hydrogen atom, $R^{3**}$, $R^{3***}$ or $R^{3****}$ represent a hydrogen atom, a chlorine atom, a methoxy group, a hydroxy group or a trifluoromethyl group; $R^{4**}$, $R^{4***}$ or $R^{4****}$ represent a hydrogen atom or a chlorine atom; $R^{5**}$, $R^{5***}$ or $R^{5****}$ represent a hydrogen atom or a methoxy group and $R^{6**}$, $R^{6***}$ or $R^{6****}$ represent a hydrogen atom and $n^{**}$, $n^{***}$ or $n^{****}$ have the meaning given above, and their pharmacologically compatible salts of inorganic or organic bases.

8. Compounds in accordance with claims 4 or 5 or 6, in which $R^{1**}$, $R^{1***}$ or $R^{1****}$ signify an alkyl group with 1 to 3 carbon atoms or a substituted phenyl radical; $R^{2**}$, $R^{2***}$ or $R^{2****}$ signify a hydrogen atom; $R^{3**}$, $R^{3***}$ or $R^{3****}$ signify a chlorine atom or a hydroxy group; $R^{4**}$, $R^{4***}$ or $R^{4****}$ signify a hydrogen atom or a chlorine atome; $R^{5**}$, $R^{5***}$ or $R^{5****}$ signify a hydrogen atom or a methoxy group; $R^{6**}$, $R^{6***}$ or $R^{6****}$ signify a hydrogen atom and $n^{**}$, $n^{***}$ or $n^{****}$ have the meanings given above, and their pharmacologically compatible salts of inorganic or organic bases.

9. Compounds in accordance with claims 4 or 5, in which $R^{1**}$ or $R^{1***}$ signify an alkyl group with 1 to 3 carbon atoms or a substituted phenyl radical; $R^{2**}$ or $R^{2***}$signify a hydrogen atom; $R^{3**}$ or $R^{3***}$ signify a chlorine atom or a hydroxy group; $R^{4**}$ or $R^{4***}$ signify a hydrogen atom; $R^{5**}$ or $R^{5***}$ signify a hydrogen

atom or a methoxy group; $R^{6**}$ or $R^{6***}$ signify a hydrogen atom and n** or n*** signify 3 or 4, and their pharmacologically compatible salts of inorganic or organic bases.

10. Pharmaceutical products containing one or more compounds according to one or more of the claims 1 to 9 and/or their pharmacologically compatible salts of inorganic or organic bases.

11. Process for the production of the acyl(bicyclic aryl)-aminoalkanoic acids of the general formula I

$$R^1—CO—\underset{\displaystyle A}{\underset{|}{N}}—C_nH_{2n}—COOH \qquad (I)$$

and their salts of inorganic or organic bases, characterized by the fact that

a) a (bicyclic aryl)-aminoalkanoic acid of the general formula II

$$A—NH—C_nH_{2n}—COOH \qquad (II)$$

optionally with protection of the carboxyl group, is acylated with an acyl derivative of the general formula III

$$R^1—CO—R^7 \qquad (III)$$

in which $R^7$ signifies a leaving group or a $R^1-CO-O$-group, and if desired is then converted into a salt or

b) a (bicyclic aryl)-aminoalkenoic acid of the general formula IV

$$R^1—CO—\underset{\displaystyle A}{\underset{|}{N}}—C_nH_{2n-2}—COOH \qquad (IV)$$

optionally with protection of the carboxyl group, is hydrogenated and if desired is then converted into a salt or

c) a functional acyl(bicyclic aryl)-aminoalkanoic acid derivative of the general formula V

$$R^1—CO—\underset{\displaystyle A}{\underset{|}{N}}—C_nH_{2n}—G \qquad (V)$$

in which G signifies a functional derivative of a corboxyl group, is solvolysed and if desired is then converted into a salt, the substituents $R^1$, A and n of the compounds I to V having the meaning given in Claim 1.

12. Process in accordance with claim 11 for the production of the acyl(bicyclic aryl)-aminoalkanoic acids of the general formula I*

$$R^{1*}—CO—\underset{\displaystyle A^*}{\underset{|}{N}}—C_{n^*}H_{2n^*}—COOH \qquad (I^*)$$

and their salts of inorganic or organic bases, characterized by the fact that

a) a (bicyclic aryl)-aminoalkanoic acid of the general formula II*

$$A^*—NH—C_{n^*}H_{2n^*}—COOH \qquad (II^*)$$

optionally with protection of the carboxyl group, is acylated with an acyl derivative of the general formula III*

$$R^{1*}—CO—R^{7*} \qquad (III^*)$$

in which $R^{7*}$ signifies a leaving group or a $R^{1*}-CO-O$-group, and if desired is then converted into a salt or

b) a (bicyclic aryl)-aminoalkenoic acid of the general formula IV*

$$R^{1*}—CO—N(A^*)—C_{n^*}H_{2n^*-2}—COOH \quad (IV^*)$$

optionally with protection of the carboxyl group, is hydrogenated and if desired is then converted into a salt or

c) a functional acyl(bicyclic aryl)-aminoalkanoic acid derivative of the general formula V*

$$R^{1*}—CO—N(A^*)—C_{n^*}H_{2n^*}—G^* \quad (V^*)$$

in which G* signifies a functional derivative of a carboxyl group, is solvolysed and if desired is then converted into a salt, the substituents $R^{1*}$, A* and n* of the compounds I* to V* having the meaning given in Claim 2.

13. Process in accordance with claim 11 for the production of the acyl(bicyclic aryl)-aminoalkanoic acids of the general formula I**

$$R^{1**}—CO—N(A^{**})—C_{n^{**}}H_{2n^{**}}—COOH \quad (I^{**})$$

and their salts of inorganic or organic bases, characterized by the fact that

a) a (bicyclic aryl)-aminoalkanoic acid of the general formula II**

$$A^{**}—NH—C_{n^{**}}H_{2n^{**}}—COOH \quad (II^{**})$$

optionally with protection of the carboxyl group, is acylated with an acyl derivative of the general formula III**

$$R^{1**}—CO—R^{7**} \quad (III^{**})$$

in which $R^{7**}$ signifies a leaving group or a $R^{1**}$ – CO – O-group, and if desired is then converted into a salt or

b) a (bicyclic aryl)-aminoalkenoic acid of the general formula IV**

$$R^{1**}—CO—N(A^{**})—C_{n^{**}}H_{2n^{**}-2}—COOH \quad (IV^{**})$$

optionally with protection of the carboxyl group, is hydrogenated and if desired is then converted into a salt or

c) a functional acyl(bicyclic aryl)-aminoalkanoic acid derivative of the general formula V**

$$R^{1**}—CO—N(A^{**})—C_{n^{**}}H_{2n^{**}}—G^{**} \quad (V^{**})$$

in which G** signifies a functional derivative of a carboxyl group, is solvolysed and if desired is then converted into a salt, the substituents $R^{1**}$, A** and n** of the compounds I** to V** having the meaning given in Claim 4.

14. Process in accordance with claim 11 for the produktion of the acyl(bicyclic aryl)-aminoalkanoic acids of the general formula I***

$$R^{1***}—CO—N(A^{***})—C_{n^{***}}H_{2n^{***}}—COOH \quad (I^{***})$$

and their salts of inorganic or organic bases, characterized by the fact that

a) a (bicyclic aryl)-aminoalkanoic acid of the general formula II***

$$A^{***}—NH—C_{n^{***}}H_{2n^{***}}—COOH \qquad (II^{***})$$

optionally with protection of the carboxyl group, is acylated with an acyl derivative of the general formula III***

$$R^{1***}—CO—R^{7***} \qquad (III^{***})$$

in which $R^{7***}$ signifies a leaving group or a $R^{1***}-CO-O$-group, and if desired is then converted into a salt or

b) a (bicyclic aryl)-aminoalkenoic acid of the general formula IV***

$$R^{1***}—CO—N(A^{***})—C_{n^{***}}H_{2n^{***}-2}—COOH \qquad (IV^{***})$$

optionally with protection of the carboxyl group, is hydrogenated and if desired is then converted into a salt or

c) a functional acyl(bicyclic aryl)-aminoalkanoic acid derivative of the general formula V***

$$R^{1***}—CO—N(A^{***})—C_{n^{***}}H_{2n^{***}}—G^{***} \qquad (V^{***})$$

in which G*** signifies a functional derivative of a carboxyl group, is solvolysed and if desired is then converted into a salt, the substituents $R^{1***}$, A*** and n*** of the compounds I*** to V*** having the meaning given in Claim 5.

15. Process in accordance with claim 11 for the production of the acyl(bicyclic aryl)-aminoalkanoic acids of the general formula I****

$$R^{1****}—CO—N(A^{****})—C_{n^{****}}H_{2n^{****}}—COOH \qquad (I^{****})$$

and their salts of inorganic or organic bases, characterized by the fact that

a) a (bicyclic aryl)-aminoalkanoic acid of the general formula II****

$$A^{****}—NH—C_{n^{****}}H_{2n^{****}}—COOH \qquad (II^{****})$$

optionally with protection of the carboxyl group, is acylated with an acyl derivative of the general formula III****

$$R^{1****}—CO—R^{7****} \qquad (III^{****})$$

in which $R^{7****}$ signifies a leaving group or a $R^{1****}-CO-O$-group, and if desired is then converted into a salt or

b) a (bicyclic aryl)-aminoalkenoic acid of the general formula IV****

$$R^{1****}—CO—N(A^{****})—C_{n^{****}}H_{2n^{****}-2}—COOH \qquad (IV^{****})$$

optionally with protection of the carboxyl group, is hydrogenated and if desired is then converted into a salt or

c) a functional acyl(bicyclic aryl)-aminoalkanoic acid derivative of the general formula V****

$$R^{1****}-CO-\underset{\displaystyle A^{****}}{\underset{|}{N}}-C_{n^{****}}H_{2n^{****}}-G^{****} \qquad (V^{****})$$

in which G**** signifies a functional derivative of a carboxyl group, is solvolysed and if desired is then converted into a salt, the substituents $R^{1****}$, A**** and n**** of the compounds I**** to V**** having the meaning given in Claim 6.

16. Compounds in accordance with one or more of the claims 1 to 9 for the treatment and prophylaxis of diseases which are attributable to disorders of the stomach or intestine or to reduced performances of the pancreas, bile and/or liver.

**Claims for the Contracting state: AT**

1. Process for the production of the acyl(bicyclic aryl)-aminoalkanoic acids of the general formula I

$$R^{1}-CO-\underset{\displaystyle A}{\underset{|}{N}}-C_nH_{2n}-COOH \qquad (I)$$

in which

$R^1$ signifies an aliphatic or alicyclic hydrocarbon radical or a phenyl group

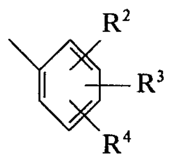

A signifies an optionally partly hydrogenated bicyclic aryl radical with 8 to 12 carbon atoms, optionally substituted by one or more halogen atoms, alkyl or alkoxy groups with 1 to 4 carbon atoms, optionally substituted amino groups, hydroxy, nitro or trifluoromethyl groups,

$R^2$, $R^3$ and $R^4$ are the same or different and signify a hydrogen atom, a halogen atom, an alkyl group, a hydroxy group, an alkoxy group, an acyloxy group, an optionally substituted amino group, a nitro group or a trifluoromethyl group and

n signifies 3, 4 or 5

and their salts of inorganic or organic bases, characterized by the fact that

a) a (bicyclic aryl)-aminoalkanoic acid of the general formula II

$$A-NH-C_nH_{2n}-COOH \qquad (II)$$

in which A and n have the meanings given above, optionally with protection of the carboxyl group, is acylated with an acyl derivative of the general formula III

$$R^1-CO-R^7 \qquad (III)$$

in which $R^7$ signifies a leaving group or a $R^1-CO-O$-group and $R^1$ has the meaning given above, and if desired is then converted into a salt or

b) a (bicyclic aryl)-aminoalkenoic acid of the general formula IV

$$R^{1}-CO-\underset{\displaystyle A}{\underset{|}{N}}-C_nH_{2n-2}-COOH \qquad (IV)$$

in which $R^1$, A and n have the meanings given above, optionally with protection of the carboxyl group, is hydrogenated and if desired is then converted into a salt or

c) a funktional acyl(bicyclic aryl)-aminoalkanoic acid derivative of the general formula V

$R^1—CO—N—C_nH_{2n}—G$ (V)
|
A

in which $R^1$, A and n have the meanings given above and G signifies a functional derivative of a carboxyl group, is solvolysed and if desired is then converted into a salt.

2. Process in accordance with claim 1 for the production of the acyl(bicyclic aryl)-aminoalkanoic acids of the general formula I*

$R^{1*}—CO—N—C_{n*}H_{2n*}—COOH$ (I*)
|
A*

in which

$R^{1*}$ signifies an aliphatic hydrocarbon radical with 1 to 5 carbon atoms, an alicyclic hydrocarbon radical with 5 to 7 carbon atoms or a phenyl radical

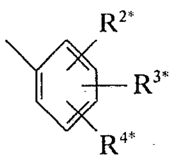

A* signifies an indenyl, indanyl, naphthyl, dihydronaphthyl or tetrahydronaphthyl radical substituted with $R^{5*}$ and $R^{6*}$,
n* signifies 3, 4 or 5,
$R^{2*}$, $R^{3*}$ and $R^{4*}$ are the same or different and signify a hydrogen atom, a halogen atom, an alkyl group with 1 to 4 carbon atoms, an alkoxy group with 1 to 4 carbon atoms, an alkanoyloxy group with 2 to 5 carbon atoms, a nitro group, a hydroxy group or a trifluoromethyl group,
$R^{5*}$ and $R^{6*}$ are the same or different and signify a hydrogen atom, a halogen atom, a methyl group, an alkoxy group with 1 to 4 carbon atoms, a nitro group or a trifluoromethyl group,

and their salts of inorganic or organic bases, characterized by the fact that

a) a (bicyclic aryl)-aminoalkanoic acid of the general formula II*

$A^*—NH—C_{n*}H_{2n*}—COOH$ (II*)

in which A* and n* have meanings given above, optionally with protection of the carboxyl group, is acylated with an acyl derivative of the general formula III*

$R^{1*}—CO—R^{7*}$ (III*)

in which $R^{7*}$ signifies a leaving group or a $R^{1*}-CO-O$-group and $R^{1*}$ has the meaning given above, and if desired is then converted into a salt or

b) a (bicyclic aryl)-aminoalkenoic acid of the general formula IV*

$R^{1*}—CO—N—C_{n*}H_{2n*-2}—COOH$ (IV*)
|
A*

in which $R^{1*}$, A* and n* have the meanings given above, optionally with protection of the carboxyl group, is hydrogenated and if desired is then converted into a salt or

c) a functional acyl(bicyclic aryl)-aminoalkanoic acid derivative of the general formula V*

$R^{1*}—CO—N—C_{n*}H_{2n*}—G^*$ (V*)
|
A*

in which $R^{1*}$, A* and n* have the meanings given above and G* signifies a functional derivative of a carboxyl group, is solvolysed and if desired is then converted into a salt.

3. Process in accordance with claim 2, in which A* represents a 5-indanyl, 1-naphthyl or 1,2,3,4-tetrahydronaphthyl-(1) radical substituted with $R^{5*}$ and $R^{6*}$.

4. Process in accordance with claim 1 for the production of the acyl(bicyclic aryl)-aminoalkanoic acids of the general formula I**

$$R^{1**}-CO-\underset{\displaystyle A^{**}}{\underset{|}{N}}-C_{n^{**}}H_{2n^{**}}-COOH \qquad (I^{**})$$

in which

$R^{1**}$ signifies an aliphatic hydrocarbon radical with 1 to 5 carbon atoms or a phenyl radical substituted with $R^{2**}$, $R^{3**}$ and $R^{4**}$,

$A^{**}$ signifies a group of the formula

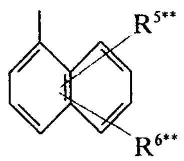

$n^{**}$ signifies 3, 4 or 5,

$R^{2**}$, $R^{3**}$ and $R^{4**}$ are the same or different and signify a hydrogen atom, a halogen atom, a methyl group, a methoxy group, a nitro group, a hydroxy group or a trifluoromethyl group,

$R^{5**}$ and $R^{6**}$ are the same or different and signify a hydrogen atom, a chlorine atom, an ethoxy group, a methoxy group or a nitro group,

and their salts of inorganic or organic bases, characterized by the fact that

a) a (bicyclic aryl)-aminoalkanoic acid of the general formula II**

$$A^{**}-NH-C_{n^{**}}H_{2n^{**}}-COOH \qquad (II^{**})$$

in which A** and n** have the meanings given above, optionally with protection of the carboxyl group, is acylated with an acyl derivative of the general formula III**

$$R^{1**}-CO-R^{7**} \qquad (III^{**})$$

in which $R^{7**}$ signifies a leaving group or a $R^{1**}-CO-O$-group and $R^{1**}$ has the meaning given above, and if desired is then converted into a salt or

b) a (bicyclic aryl)-aminoalkenoic acid of the general formula IV**

$$R^{1**}-CO-\underset{\displaystyle A^{**}}{\underset{|}{N}}-C_{n^{**}}H_{2n^{**}-2}-COOH \qquad (IV^{**})$$

in which $R^{1**}$, A** and n** have the meaning given above, optionally with protection of the carboxyl group, is hydrogenated and if desired is then converted into a salt or

c) a functional acyl(bicyclic aryl)-aminoalkanoic acid derivative of the general formula V**

$$R^{1**}-CO-\underset{\displaystyle A^{**}}{\underset{|}{N}}-C_{n^{**}}H_{2n^{**}}-G^{**} \qquad (V^{**})$$

in which $R^{1**}$, A** and n** have the meaning given above and G** signifies a functional derivative of a carboxyl group, is solvolysed and if desired is then converted into a salt.

5. Process in accordance with claim 1 for the production of the acyl(bicyclic aryl)-aminoalkanoic acids of the general formula I***

$$R^{1***}-CO-\underset{\displaystyle A^{***}}{\underset{|}{N}}-C_{n^{***}}H_{2n^{***}}-COOH \qquad (I^{***})$$

in which

$R^{1***}$ signifies an aliphatic hydrocarbon radical with 1 to 5 carbon atoms or a phenyl radical substituted with $R^{2***}$, $R^{3***}$ and $R^{4***}$,
A*** signifies a group of the formula

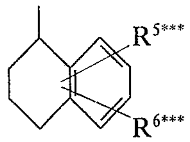

n*** signifies 3, 4 or 5,
$R^{2***}$, $R^{3***}$ and $R^{4***}$ are the same or different and signify a hydrogen atom, a halogen atom, a methyl group, a methoxy group, a nitro group, a hydroxy group or a trifluoromethyl group,
$R^{5***}$ and $R^{6***}$ are the same or different and represent a hydrogen atom, a chlorine atom, a methoxy group or a nitro group,

and their salts of inorganic or organic bases, characterized by the fact that

a) a (bicyclic aryl)-aminoalkanoic acid of the general formula II***

$$A^{***}—NH—C_{n^{***}}H_{2n^{***}}—COOH \qquad (II^{***})$$

in which A*** and n*** have the meanings given above, optionally with protection of the carboxyl group, is acylated with an acyl derivative of the general formula III***

$$R^{1***}—CO—R^{7***} \qquad (III^{***})$$

in which $R^{7***}$ signifies a leaving group or a $R^{1***}-CO-O$-group and $R^{1***}$ has the meaning given above, and if desired is then converted into a salt or

b) a (bicyclic aryl)-aminoalkenoic acid of the general formula IV***

$$R^{1***}—CO—\underset{\displaystyle A^{***}}{\underset{|}{N}}—C_{n^{***}}H_{2n^{***}-2}—COOH \qquad (IV^{***})$$

in which $R^{1***}$, A*** and n*** have the meaning given above, optionally with protection of the carboxyl group, is hydrogenated and if desired is then converted into a salt or

c) a functional acyl(bicyclic aryl)-aminoalkanoic acid derivative of the general formula V***

$$R^{1***}—CO—\underset{\displaystyle A^{***}}{\underset{|}{N}}—C_{n^{***}}H_{2n^{***}}—G^{***} \qquad (V^{***})$$

in which $R^{1***}$, A*** and n*** have the meanings given above and G*** signifies a functional derivative of a carboxyl group, is solvolysed and if desired is then converted into a salt.

6. Process in accordance with claim 1 for the production of the acyl(bicyclic aryl)-aminoalkanoic acids of the general formula I****

$$R^{1****}—CO—\underset{\displaystyle A^{****}}{\underset{|}{N}}—C_{n^{****}}H_{2n^{****}}—COOH \qquad (I^{****})$$

in which

$R^{1****}$ signifies an aliphatic hydrocarbon radical with 1 to 5 carbon atoms or a phenyl radical substituted with $R^{2****}$, $R^{3****}$ and $R^{4****}$,
A**** signifies a group of the formula

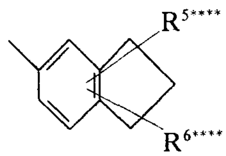

$n^{****}$ signifies 3, 4 or 5,

$R^{2****}$, $R^{3****}$ and $R^{4****}$ are the same or different and represent a hydrogen atom, a halogen atom, a methyl group, a methoxy group, a nitro group, a hydroxy group or a trifluoromethyl group,

$R^{5****}$ and $R^{6****}$ are the same or different and represent a hydrogen atom, a chlorine atom, a methoxy group or a nitro group,

and their salts of inorganic or organic bases, characterized by the fact that

a) a (bicyclic aryl)-aminoalkanoic of the general formula II****

$$A^{****}—NH—C_{n^{****}}H_{2n^{****}}—COOH \qquad (II^{****})$$

in which A**** and n**** have the meanings given above, optionally with protection of the carboxyl group, is acylated with an acyl derivative of the general formula III****

$$R^{1****}—CO—R^{7****} \qquad (III^{****})$$

in which $R^{7****}$ signifies a leaving group or a $R^{1****}-CO-O$-group and $R^{1****}$ has the meaning given above, and if desired is then converted into a salt or

b) a (bicyclic aryl)-aminoalkenoic acid of the general formula IV****

$$R^{1****}—CO—N(A^{****})—C_{n^{****}}H_{2n^{****}-2}—COOH \qquad (IV^{****})$$

in which $R^{1****}$, A**** and n**** have the meanings given above, optionally with protection of the carboxyl group, is hydrogenated and if desired is then converted into a salt or

c) a functional acyl(bicyclic aryl)-aminoalkanoic acid derivative of the general formula V****

$$R^{1****}—CO—N(A^{****})—C_{n^{****}}H_{2n^{****}}—G^{****} \qquad (V^{****})$$

in which $R^{1****}$, A**** and n**** have the meanings given above and G**** signifies a functional derivative of a carboxyl group, is solvolysed and if desired is then converted into a salt.

7. Process in accordance with claims 4 or 5 or 6, in which $R^{1**}$, $R^{1***}$ or $R^{1****}$ represent an alkyl group with 1 to 3 carbon atoms or a substituted phenyl radical; $R^{2**}$, $R^{2***}$ or $R^{2****}$ signify a hydrogen atom, $R^{3**}$, $R^{3***}$ or $R^{3****}$ represent a hydrogen atom, a chlorine atom, a methoxy group, a hydroxy group or a trifluoromethyl group; $R^{4**}$, $R^{4***}$ or $R^{4****}$ represent a hydrogen atom or a chlorine atom; $R^{5**}$, $R^{5***}$ or $R^{5****}$ represent a hydrogen atom or a methoxy group and $R^{6**}$, $R^{6***}$ or $R^{6****}$ represent a hydrogen atom and n**, n*** or n**** have the meanings there given.

8. Process in accordance with claims 4 or 5 or 6, in which $R^{1**}$, $R^{1***}$ or $R^{1****}$ signify an alkyl group with 1 to 3 carbon atoms or a substituted phenyl radical; $R^{2**}$, $R^{2***}$ or $R^{2****}$ signify a hydrogen atom; $R^{3**}$, $R^{3***}$ or $R^{3****}$ signify a chlorine atom or a hydroxy group; $R^{4**}$, $R^{4***}$ or $R^{4****}$ signify a hydrogen atom or a chlorine atome; $R^{5**}$, $R^{5***}$ or $R^{5****}$ signify a hydrogen atom or a methoxy group; $R^{6**}$, $R^{6***}$ or $R^{6****}$ signify a hydrogen atom and n**, n*** or n**** have the meanings there given.

9. Process in accordance with claims 4 or 5, in which $R^{1**}$ or $R^{1***}$ signify an alkyl group with 1 to 3 carbon atoms or a substituted phenyl radical; $R^{2**}$ or $R^{2***}$ signify a hydrogen atom;, $R^{3**}$ or $R^{3***}$ signify a chlorine atom or a hydroxy group; $R^{4**}$ or $R^{4***}$ signify a hydrogen atom; $R^{5**}$ or $R^{5***}$ signify a hydrogen atom or a methoxy group; $R^{6**}$ or $R^{6***}$ signify a hydrogen atom and n** or n*** signify 3 or 4.

10. Process in accordance with one of the claims 1 to 9, wherein the leaving group $R^7$, $R^{7*}$, $R^{7**}$, $R^{7***}$ or $R^{7****}$ denotes a chlorine or bromine atom and G, G*, G**, G*** or G**** denotes a nitrile, a carboxylic acid ester or a carboxylic acid amide group.

**Revendications pour les Etats contractants: BE, CH, DE, FR, GB, IT, LU, NL, SE**

1. Acides acyl(aryl bicyclique)aminoalcanoïques de formule générale I:

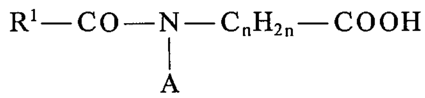

(I)

dans laquelle

$R^1$ représente un radical d'hydrocarbure aliphatique ou alicyclique ou un groupe phényle

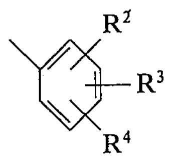

A représente un groupe aryle bicyclique contenant 8 à 12 atomes de carbone, éventuellement hydrogéné en partie et éventuellement substitué une on plusieurs fois par des atomes d'halogène, des groupes alkyle ou alcoxy contenant 1 à 4 atomes de carbone, des groupes amino éventuellement substitués, des groupes hydroxy, des groupes nitro ou des groupes trifluorométhyle,

$R^2$, $R^3$ et $R^4$ sont identiques ou différents et représentent un atome d'hydrogène, un atome d'halogène, un groupe alkyle, un gròupe hydroxy, un groupe alcoxy, un groupe acyloxy, un groupe amino éventuellement substitué, un groupe nitro ou un groupe trifluorométhyle et

n est égal à 3, 4 ou 5,

ainsi que leurs sels avec des bases inorganiques ou organiques.

2. Acides acyl(aryl bicyclique)aminoalcanoïques selon la revendication 1, charactérisés en ce qu'ils répondent a la formule générale I*:

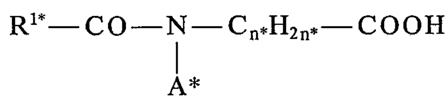

(I*)

dans laquelle

$R^{1*}$ représente un radical d'hydrocarbure aliphatique contenant 1 à 5 atomes de carbone, un radical d'hydrocarbure alicyclique contenant 5 à 7 atoms de carbone ou un groupe phényle

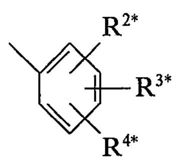

A* représente un groupe indényle, un groupe indanyle, un groupe naphtyle, un groupe dihydronaphtyle ou un groupe tétrahydronaphtyle substitué par $R^{5*}$, $R^{6*}$,

n* est égal à 3, 4 ou 5,

$R^{2*}$, $R^{3*}$ et $R^{4*}$, qui peuvent être identiques ou différents, représentent chacun un atome d'hydrogène, un atome d'halogène, un groupe alkyle contenant 1 à 4 atomes de carbone, un groupe alcoxy contenant 1 à 4 atomes de carbone, un groupe alcanoyloxy contenant 2 à 5 atomes de carbone, un groupe nitro, un groupe hydroxy ou un groupe trifluorométhyle,

$R^{5*}$ et $R^{6*}$, qui peuvent être identiques ou différents, représentent chacun un atome d'hydrogène, un atome d'halogène, un groupe méthyle, un groupe alcoxy contenant 1 à 4 atomes de carbone, un groupe nitro ou un groupe trifluorométhyle,

ainsi que leurs sels avec des bases inorganiques ou organiques.

3. Composés selon la revendication 2, dans lesquels A* représente un groupe 5-indanyle, un groupe 1-naphtyle ou un groupe 1,2,3,4-tétrahydronaphtyle-(1) substitué par $R^{5*}$ et $R^{6*}$.

4. Acides acyl(aryl bicyclique)amino-alcanoiques selon la revendication 1, charactérisés en ce qu'ils répondent a la formule générale I**:

$$R^{1**}-CO-\underset{\underset{\displaystyle A^{**}}{|}}{N}-C_{n^{**}}H_{2n^{**}}-COOH \qquad (I^{**})$$

dans laquelle

$R^{1**}$ représente un radical d'hydrocarbure aliphatique contenant 1 à 5 atomes de carbone ou un groupe phényle substitué par $R^{2**}$, $R^{3**}$ et $R^{4**}$,

$A^{**}$ représente un groupe de formule:

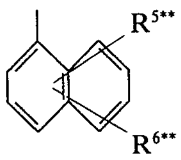

$n^{**}$ est égal à 3, 4 ou 5,

$R^{2**}$, $R^{3**}$ et $R^{4**}$, qui peuvent être identiques ou différents, représentent chacun un atome d'hydrogène, un atome d'halogène, un groupe méthyle, un groupe méthoxy, un groupe nitro, un groupe hydroxy ou un groupe trifluorométhyle,

$R^{5**}$ et $R^{6**}$, qui peuvent être identiques ou différents, représentent chacun un atome d'hydrogène, un atome de chlore, un groupe éthoxy, un groupe méthoxy ou un groupe nitro,

ainsi que leurs sels avec des bases inorganiques ou organiques.

5. Acides acyl(aryl bicyclique)amino-alcanoiques selon la revendication 1, charactérisés en ce qu'ils répondent a la formule générale $I^{***}$:

$$R^{1***}-CO-\underset{\underset{\displaystyle A^{***}}{|}}{N}-C_{n^{***}}H_{2n^{***}}-COOH \qquad (I^{***})$$

dans laquelle

$R^{1***}$ représente un radical d'hydrocarbure aliphatique contenant 1 à 5 atomes de carbone ou un groupe phényle substitué par $R^{2***}$, $R^{3***}$ et $R^{4***}$,

$A^{***}$ représente un groupe de formule:

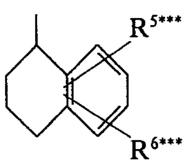

$n^{***}$ est égal à 3, 4 ou 5,

$R^{2***}$, $R^{3***}$ et $R^{4***}$, qui peuvent être identiques ou différents, représentent chacun un atome d'hydrogène, un atome d'halogène, un groupe méthyle, un groupe méthoxy, un groupe nitro, un groupe hydroxy ou un groupe trifluorométhyle,

$R^{5***}$ et $R^{6***}$, qui peuvent être identiques ou différents, représentent chacun un atome d'hydrogène, un atome de chlore, un groupe ,éthoxy ou un groupe nitro,

ainsi que leurs sels avec des bases inorganiques ou organiques.

6. Acides acyl(aryl bicyclique)amino-alcanoiques selon la revendication 1, charactérisés en ce qu'ils répondent a la formule générale $I^{****}$:

$$R^{1****}-CO-\underset{\underset{\displaystyle A^{****}}{|}}{N}-C_{n^{****}}H_{2n^{****}}-COOH \qquad (I^{****})$$

dans laquelle

$R^{1****}$ représente un radical d'hydrocarbure aliphatique contenant 1 à 5 atomes de carbone ou un groupe phényle substitué par $R^{2****}$, $R^{3****}$ et $R^{4****}$,

$A^{****}$ représente un groupe de formule:

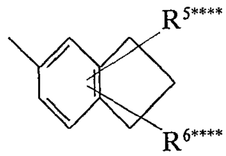

$n^{****}$ est égal à 3, 4 ou 5,

$R^{2****}$, $R^{3****}$ et $R^{4****}$, qui peuvent être identiques ou différents, représentent chacun un atome d'hydrogène, un atome d'halogène, un groupe méthyle, un groupe méthoxy, un groupe nitro, un groupe hydroxy ou un groupe trifluorométhyle,

$R^{5****}$ et $R^{6****}$, qui peuvent être identiques ou différents, représentent chacun un atome d'hydrogène, un atome de chlore un groupe méthoxy ou un groupe nitro,

ainsi que leurs sels avec des bases inorganiques ou organiques.

7. Composés selon les revendications 4 ou 5 ou 6 dans lesquels $R^{1**}$, $R^{1***}$, ou $R^{1****}$ représente un groupe alkyle contenant 1 à 3 atomes de carbone ou un groupe phényle substitué; $R^{2**}$, $R^{2***}$ ou $R^{2****}$ représente un atome d'hydrogène; $R^{3**}$, $R^{3***}$ ou $R^{3****}$ représente un atome d'hydrogène, un atome de chlore, un groupe méthoxy, un groupe hydroxy ou un groupe trifluorométhyle; $R^{4**}$, $R^{4***}$ ou $R^{4****}$ représente un atome d'hydrogène ou un atome de chlore; $R^{5**}$, $R^{5***}$ ou $R^{5****}$ représente un atome d'hydrogène ou un groupe méthoxy et $R^{6**}$, $R^{6***}$ ou $R^{6****}$ représente un atome d'hydrogène, tandis que $n^{**}$, $n^{***}$ ou $n^{****}$ a la signification indiquée ci-dessus, de même que leurs sels pharmacologiquement acceptables avec des bases inorganiques ou organiques.

8. Composés selon les revendications 4 ou 5 ou 6, dans lesqueles $R^{1**}$, $R^{1***}$ ou $R^{1****}$ représente un groupe alkyle contenant 1 à 3 atomes de carbone ou un groupe phényle substitué; $R^{2**}$, $R^{2***}$ ou $R^{2****}$ représente un atome d'hydrogène; $R^{3**}$, $R^{3***}$ ou $R^{3****}$ représente un atome de chlore ou un groupe hydroxy; $R^{4**}$, $R^{4***}$ ou $R^{4****}$ représente un atome d'hydrogène ou un atome de chlore; $R^{5**}$, $R^{5***}$ ou $R^{5****}$ représente un atome d'hydrogène ou un groupe méthoxy et $R^{6**}$, $R^{6***}$ ou $R^{6****}$ représente un atome d'hydrogène, tandis que $n^{**}$, $n^{***}$ ou $n^{****}$ a la signification indiquée ci-dessus, de même que leurs sels pharmacologiquement acceptables avec des Bases inorganiques ou organiques.

9. Composés selon les revendications 4 0u 5, dans lesquels $R^{1**}$ ou $R^{1***}$ est un groupe alkyle contenant 1 à 3 atomes de carbone ou un groupe phényle substitué, $R^{2**}$ ou $R^{2***}$ est un atome d'hydrogène; $R^{3**}$ ou $R^{3***}$ est un atome de chlore ou un groupe hydroxy; $R^{4**}$ ou $R^{4***}$ est un atome d'hydrogène; $R^{5**}$ ou $R^{5***}$ est un atome d'hydrogène ou un groupe méthoxy et $R^{6**}$ ou $R^{6***}$ est un atome d'hydrogène, tandis que $n^{**}$ ou $n^{***}$est égal à 3 ou 4, de même que leurs sels pharmacologiquement acceptables avec des bases inorganiques ou organiques.

10. Médicaments contenant un ou plusieurs composés selon une ou plusieurs des revendications 1 à 9 et/ou leurs sels pharmacologiquement acceptables avec des bases inorganiqus ou organiques.

11. Procédé de préparation d'acides acyl(aryl bicyclique)amino-alcanoïques de formule générale I:

$$R^1-CO-\underset{\underset{A}{|}}{N}-C_nH_{2n}-COOH \qquad (I)$$

ainsi que de leurs sels avec des bases inorganiques ou organiques, caractérisé en ce que:

a) on acyle un acide (aryl bicyclique)amino-alcanoïque de formule générale II:

$$A-NH-C_nH_{2n}-COOH \qquad (II)$$

en protégeant éventuellement le groupe carboxy, avec un dérivé acyle de formule générale III:

$$R^1-CO-R^7 \qquad (III)$$

dans laquelle $R^7$ représente un groupe qui s'éloigne ou un groupe $R^1-CO-O$-, puis on le transforme éventuellement en un sel, ou

b) on hydrogène un acide (aryl bicyclique)amino-alcénoïque de formule générale IV:

$$R^1-CO-\underset{\underset{A}{|}}{N}-C_nH_{2n-2}-COOH \qquad (IV)$$

en protégeant éventuellement le groupe carboxy, puis on le transforme éventuellement en sel, ou

c) on solvolyse un dérivé fonctionnel d'un acide acyl(aryl bicyclique)amino-alcanoïque de formule générale V:

$$R^1—CO—N(A)—C_nH_{2n}—G \qquad (V)$$

dans laquelle G représente un dérivé fonctionnel d'un groupe carboxy, puis on le transforme éventuellement en un sel, les substituants $R^1$, A et n des composés I à V ayant les significations indiquées dans la revendication 1.

12. Procédé selon la revendication 11 pour la préparation d'acides acyl(aryl bicyclique)amino-alcanoïques de formule générale I*:

$$R^{1*}—CO—N(A^*)—C_{n^*}H_{2n^*}—COOH \qquad (I^*)$$

ainsi que de leurs sels avec des bases inorganiques ou organiques, caractérisé en ce que:

a) on acyle un acide (aryl bicyclique)amino-alcanoïque de formule générale II*:

$$A^*—NH—C_{n^*}H_{2n^*}—COOH \qquad (II^*)$$

en protégeant eventuellement le groupe carboxy, avec un dérivé acyle de formule générale III*:

$$R^{1*}—CO—R^{7*} \qquad (III^*)$$

dans laquelle $R^{7*}$ représente un groupe qui s'éloigne ou un groupe $R^{1*}$ $-CO-O-$, puis on le transforme éventuellement en un sel, ou

b) on hydrogène un acide (aryl bicyclique)amino-alcénoïque de formule générale IV*:

$$R^{1*}—CO—N(A^*)—C_{n^*}H_{2n^*-2}—COOH \qquad (IV^*)$$

en protégeant éventuellement le groupe carboxy, puis on le transforme éventuellement en un sel, ou

c) on solvolyse un dérivé fonctionnel d'un acide acyl(aryl bicyclique)amino-alcanoïque de formule générale V*:

$$R^{1*}—CO—N(A^*)—C_{n^*}H_{2n^*}—G^* \qquad (V^*)$$

dans laquelle G* représente un dérivé fonctionnel d'un groupe carboxy, puis on le transforme éventuellement en un sel, les substituants $R^{1*}$, A* et n* des composés I* à V* ayant les significations indiquées dans la revendication 2.

13. Procédé selon la revendication 11 pour la préparation d'acides acyl(aryl bicyclique)amino-alcanoïques de formule générale I**:

$$R^{1**}—CO—N(A^{**})—C_{n^{**}}H_{2n^{**}}—COOH \qquad (I^{**})$$

ainsi que de leurs sels avec des bases inorganiques ou oraniques, caractérisé en ce que:

a) on acyle un acide (aryl bicyclique)amino-alcanoïque de formule générale II**:

$$A^{**}—NH—C_{n^{**}}H_{2n^{**}}—COOH \qquad (II^{**})$$

en protégeant éventuellement le groupe carboxy, avec un dérivé acyle de formule générale III**:

$$R^{1**}—CO—R^{7**} \qquad (III^{**})$$

dans laquelle $R^{7**}$ représente un groupe qui s'éloigne ou un groupe $R^{1**}$ –CO–O-, puis on le transforme éventuellement en un sel, ou

b) on hydrogène un acide (aryl bicyclique)amino-alcénoïque de formule générale IV**:

$$R^{1**}—CO—N(A^{**})—C_{n**}H_{2n**-2}—COOH \quad (IV^{**})$$

en protégeant éventuellement le groupe carboxy, puis on le transforme éventuellement en un sel, ou

c) on solvolyse un dérivé fonctionnel d'un acide acyl(aryl bicyclique)amino-alcanoïque de formule générale V**:

$$R^{1**}—CO—N(A^{**})—C_{n**}H_{2n**}—G^{**} \quad (V^{**})$$

dans laquelle G** représente un dérivé fonctionnel d'un groupe carboxy, puis on le transforme éventuellement en un sel, les substituants $R^{1**}$, A** et n** des composés I** à V** ayant les significations indiquées dans la revendication 4.

14. Procédé selon la revendication 11 pour la préparation d'acides acyl(aryl bicyclique)amino-alcanoïques de formule générale I***:

$$R^{1***}—CO—N(A^{***})—C_{n***}H_{2n***}—COOH \quad (I^{***})$$

ainsi que de leurs sels avec des bases inorganiques ou organiques, caractérisé en ce que:

a) on acyle un acide (aryl bicyclique)amino-alcanoïque de formule générale II***:

$$A^{***}—NH—C_{n***}H_{2n***}—COOH \quad (II^{***})$$

en protégeant éventuellement le groupe carboxy, avec un dérivé acyle de formule générale III***:

$$R^{1***}—CO—R^{7***} \quad (III^{***})$$

dans laquelle $R^{7***}$ représente un groupe qui s'éloigne ou un groupe $R^{1***}$ –CO–O-, puis on le transforme éventuellement en un sel, ou

b) on hydrogène un acide (aryl bicyclique)amino-alcénoïque de formule générale IV***:

$$R^{1***}—CO—N(A^{***})—C_{n***}H_{2n***-2}—COOH \quad (IV^{***})$$

en protégeant éventuellement le groupe carboxy, puis on le transforme éventuellement en un sel, ou

c) on solvolyse un dérivé fonctionnel d'un acide acyl (aryl bicyclique)amino-alcanoïque de formule générale V***:

$$R^{1***}—CO—N(A^{***})—C_{n***}H_{2n***}—G^{***} \quad (V^{***})$$

dans laquelle G*** représente un dérivé fonctionnel d'un groupe carboxy, puis on le transforme éventuellement en un sel, les substituants $R^{1***}$, A*** et n*** des composés I*** à I*** ayant les significants indiquées dans la revendication 5.

15. Procédé selon la revendication 11, pour la préparation d'acides acyl(aryl bicyclique)amino-alcanoïques de formule générale I****:

$$R^{1****}-CO-\underset{\displaystyle A^{****}}{\underset{|}{N}}-C_{n^{****}}H_{2n^{****}}-COOH \qquad (I^{****})$$

ainsi que de leurs sels avec des bases inorganiques ou organiques, caractérisé en ce que:

a) on acyle un acide (aryl bicyclique)amino-alcanoïque de formule générale II****:

$$A^{****}-NH-C_{n^{****}}H_{2n^{****}}-COOH \qquad (II^{****})$$

en protégeant éventuellement le groupe carboxy, avec un dérivé acyle de formule générale III****:

$$R^{1****}-CO-R^{7****} \qquad (III^{****})$$

dans laquelle $R^{7****}$ représente un groupe qui s'éloigne ou un groupe $R^{1****}-CO-O-$, puis on le transforme éventuellement en un sel, ou

b) on hydrogène un acide (aryl bicyclique)amino-alcénoïque de formule générale IV****:

$$R^{1****}-CO-\underset{\displaystyle A^{****}}{\underset{|}{N}}-C_{n^{****}}H_{2n^{****}-2}-COOH \qquad (IV^{****})$$

en protégeant éventuellement le groupe carboxy, puis on le transforme éventuellement en un sel, ou

c) on solvolyse un dérivé fonctionnel d'un acide acyl(aryl bicyclique)amino-alcanoïque de formule générale V****:

$$R^{1****}-CO-\underset{\displaystyle A^{****}}{\underset{|}{N}}-C_{n^{****}}H_{2n^{****}}-G^{****} \qquad (V^{****})$$

dans laquelle G**** représente un dérivé fonctionnel d'un groupe carboxy, puis on le transforme éventuellement en un sel, les substituants $R^{1****}$, A**** et n**** des composés I**** à V**** ayant les significations indiquées dans la revendication 6.

16. Composés selon une ou plusieurs des revendications 1 à 9 pour le traitement et la prophylaxie de maladies dues à des affections de l'estomac ou de l'intestine ou encore à un rendement moins bon du pancréas, de la bile et/ou du foie.

**Revendications pour l'Etat contractant: AT**

1. Procédé de préparation d'acides acyl(aryl bicyclique)amino-alcanoiques de formule générale I:

$$R^{1}-CO-\underset{\displaystyle A}{\underset{|}{N}}-C_{n}H_{2n}-COOH \qquad (I)$$

dans laquelle

$R^1$ représente un radical d'hydrocarbure aliphatique ou alicyclique ou un groupe phényle

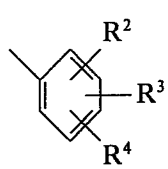

A représente un groupe aryle bicyclique contenant 8 à 12 atomes de carbone, éventuellement hydrogéné en partie et éventuellement substitué une on plusieurs fois par des atomes d'halogène, des groupes alkyle ou alcoxy contenant 1 à 4 atomes de carbone, des groupes amino éventuellement substitués, des groupes hydroxy, des groupes nitro ou des groupes trifluorométhyle,

$R^2$, $R^3$ et $R^4$ sont identiques ou différents et représentent un atome d'hydrogène, un atome d'halogène, un groupe alkyle, un groupe hydroxy, un groupe alcoxy, un groupe acyloxy, un groupe amino éventuellement substitué, un groupe nitro ou un groupe trifluorométhyle et

n est égal à 3, 4 ou 5,

ainsi que de leurs sels avec des bases inorganiques ou orgniques, caractérisé en ce que:

a) on acyle un acide (aryl bicyclique)amino-alcanoïque de formule générale II:

$$A-NH-C_nH_{2n}-COOH \qquad (II)$$

dans laquelle A et n ont les significations indiquées ci-dessus, en protégeant éventuellement le groupe carboxy, avec un dérivé acyle de formule générale III:

$$R^1-CO-R^7 \qquad (III)$$

dans laquelle $R^7$ représente un groupe qui s'éloigne ou un groupe $R^1-CO-O-$, tandis que $R^1$ a la signification indiquée ci-dessus, puis on le transforme éventuellement en un sel, ou

b) on hydrogène un acide (aryl bicyclique)amino-alcénoïque de formule générale IV:

$$R^1-CO-\underset{\displaystyle A}{\underset{|}{N}}-C_nH_{2n-2}-COOH \qquad (IV)$$

dans laquelle $R^1$, A et n ont les significations indiquées ci-dessus, en protégeant éventuellement le groupe carboxy, puis on le transforme éventuellement en un sel, ou

c) on solvolyse un dérivé fonctionnel d'un acide acyl(aryl bicyclique)amino-alcanoïque de formule générale V:

$$R^1-CO-\underset{\displaystyle A}{\underset{|}{N}}-C_nH_{2n}-G \qquad (V)$$

dans laquelle $R^1$, A et n ont les significations indiquées ci-dessus, tandis que G représente un dérivé fonctionnel d'un groupe carboxy, puis on le transforme éventuellement en un sel.

2. Procédé selon la revendication 1 pour la préparation d'acides acyl(aryl bicyclique)amino-alcanoïques de formule générale I*·

$$R^{1*}-CO-\underset{\displaystyle A^*}{\underset{|}{N}}-C_{n^*}H_{2n^*}-COOH \qquad (I^*)$$

dans laquelle

$R^{1*}$ représente un radical d'hydrocarbure aliphatique contenant 1 à 5 atomes de carbone, un radical d'hydrocarbure alicyclique contenant 5 à 7 atomes de carbone ou un groupe phényle:

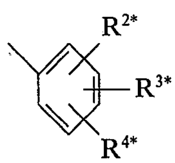

A* représente un groupe indényle, un groupe indanyle, un groupe naphtyle, un groupe dihydronaphtyle ou un groupe tétrahydronaphtyle substitué par $R^{5*}$, $R^{6*}$,

n* est égal à 3, 4 ou 5,

$R^{2*}$, $R^{3*}$ et $R^{4*}$, qui peuvent être identiques ou différents, représentent chacun un atome d'hydrogène, un atome d'halogène, un groupe alkyle contenant 1 à 4 atomes de carbone, un groupe alcoxy contenant 1 à 4 atomes de carbone, un groupe alcanoyloxy contenant 2 à 5 aomtes de carbone, un groupe nitro, un groupe hydroxy ou un groupe trifluorométhyle,

$R^{5*}$ et $R^{6*}$, qui peuvent être identiques ou différents, représentent chacun un atome d'hydrogène, un atome d'halogène, un groupe méthyle, un groupe alcoxy contenant 1 à 4 atomes de carbone, un groupe nitro ou un groupe trifluorométhyle,

ainsi que de leurs sels avec des bases inorganiques ou organiques, caractérisé en ce que:

a) on acyle un acide (aryl bicyclique)amino-alcanoıque de formule générale II*:

$$A^*-NH-C_{n^*}H_{2n^*}-COOH \qquad (II^*)$$

dans laquelle A* et n* ont les significations indiquées ci-dessus, en protégeant éventuellement le groupe carboxy, avec un dérivé acyle de formule générale III*:

$$R^{1^*}-CO-R^{7^*} \qquad (III^*)$$

dans laquelle $R^{7^*}$ représente un groupe qui s'éloigne ou un groupe $R^{1^*}-CO-O-$, tandis que $R^{1^*}$ a la signification indiquée ci-dessus, puis on le transforme éventuellement en un sel, ou

b) on hydrogène un acide (aryl bicyclique)amino-alcénoïque de formule générale IV*:

$$R^{1^*}-CO-\underset{\displaystyle A^*}{\underset{|}{N}}-C_{n^*}H_{2n^*-2}-COOH \qquad (IV^*)$$

dans laquelle $R^{1^*}$, A* et n* ont les significations indiquées ci-dessus, en protégeant éventuellement le groupe carboxy, puis on le transforme éventuellement en un sel, ou

c) on solvolyse un dérivé fonctionnel d'un acide acyl(aryl bicyclique)amino-alcanoïque de formule générale V*:

$$R^{1^*}-CO-\underset{\displaystyle A^*}{\underset{|}{N}}-C_{n^*}H_{2n^*}-G^* \qquad (V^*)$$

dans laquelle $R^{1^*}$, A* et n* ont les significations indiquées ci-dessus, tandis que G* représente un dérivé fonctionnel d'un groupe carboxy, puis on le transforme éventuellement en un sel.

3. Procédé selon la revendication 2, dans laquelle A* représente un groupe 5-indanyle, un groupe 1-naphtyle ou un groupe 1,2,3,4-tétrahydronaphtyle-(1) substitué par $R^{5^*}$ et $R^{6^*}$.

4. Procédé selon la revendication 1 pour la préparation d'acides acyl(aryl bicyclique)amino-alcanoiques de formule générale I**:

$$R^{1^{**}}-CO-\underset{\displaystyle A^{**}}{\underset{|}{N}}-C_{n^{**}}H_{2n^{**}}-COOH \qquad (I^{**})$$

dans laquelle

$R^{1^{**}}$ représente un radical d'hydrocarbure aliphatique contenant 1 à 5 atomes de carbone ou un groupe phényle substitué par $R^{2^{**}}$, $R^{3^{**}}$ et $R^{4^{**}}$,

$A^{**}$ représente un groupe de formule:

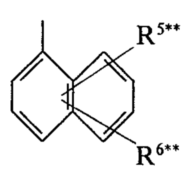

$n^{**}$ est égal à 3, 4 ou 5,

$R^{2^{**}}$, $R^{3^{**}}$ et $R^{4^{**}}$, qui peuvent être identiques ou différents, représentent chacun un atome d'hydrogène, un atome d'halogène, un groupe méthyle, un groupe méthoxy, un groupe nitro, un groupe hydroxy ou un groupe trifluorométhyle,

$R^{5^{**}}$ et $R^{6^{**}}$, qui peuvent être identiques ou différents, représentent chacun un atome d'hacrogène, un atome de chlore, un groupe éthoxy, un groupe méthoxy ou un groupe nitro,

ainsi que de leurs sels avec des bases inorganiques ou organiques, caractérisé en ce que:

a) on acyle un acide (aryl bicyclique)amino-alcanoïque de formule générale II**:

$$A^{**}—NH—C_{n^{**}}H_{2n^{**}}—COOH \qquad (II^{**})$$

dans laquelle A** et n** ont les significations indiquées ci-dessus, en protégeant éventuellement le groupe carboxy, avec un dérivé acyle de formule générale III**:

$$R^{1**}—CO—R^{7**} \qquad (III^{**})$$

dans laquelle $R^{7**}$ représente un groupe qui s'éloigne ou un groupe $R^{1**}-CO-O-$, tandis que $R^{1**}$ a la signification indiquée ci-dessus, puis on le transforme éventuellement en un sel, ou

b) on hydrogène un acide (aryl bicyclique)amino-alcénoïque de formule générale IV**:

$$R^{1**}—CO—\underset{\displaystyle A^{**}}{\underset{|}{N}}—C_{n^{**}}H_{2n^{**}-2}—COOH \qquad (IV^{**})$$

dans laquelle $R^{1**}$, A** et n** ont les significations indiquées ci-dessus, en protégeant éventuellement le groupe carboxy, puis on le transforme éventuellement en un sel, ou

c) on solvolyse un dérivé fonctionnel d'un acide acyl(aryl bicyclique)amino-alcanoïque de formule générale V**:

$$R^{1**}—CO—\underset{\displaystyle A^{**}}{\underset{|}{N}}—C_{n^{**}}H_{2n^{**}}—G^{**} \qquad (V^{**})$$

dans laquelle $R^{1**}$, A** et n** ont les significations indiquées ci-dessus, tandis que G** représente un dérivé fonctionnel d'un groupe carboxy, puis on le transforme éventuellement en un sel.

5. Procédé selon la revendication 1 pour la préparation d'acides acyl(aryl bicyclique)amino-alcanoïques de formule générale I***:

$$R^{1***}—CO—\underset{\displaystyle A^{***}}{\underset{|}{N}}—C_{n^{***}}H_{2n^{***}}—COOH \qquad (I^{***})$$

dans laquelle

$R^{1***}$ représente un radical d'hydrocarbure aliphatique contenant 1 à 5 atomes de carbone ou un groupe phényle substitué par $R^{2***}$, $R^{3***}$ et $R^{4***}$,

A*** représente un groupe de formule

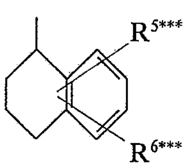

n*** est égal à 3, 4 ou 5,

$R^{2***}$, $R^{3***}$ et $R^{4***}$, qui peuvent être identiques ou différents, représentent chacun un atome d'hydrogène, un atome d'halogène, un groupe méthyle, un groupe méthoxy, un groupe nitro, un groupe hydroxy ou un groupe trifluorométhyle,

$R^{5***}$ et $R^{6***}$, qui peuvent être identiques ou différents, représentent chacun un atome d'hydrogène, un atome de chlore, un groupe méthoxy ou un groupe nitro,

ainsi que de leurs sels avec des bases inorganiques ou organiques, caractérisé en ce que:

a) on acyle un acide (aryl bicyclique)amino-alcanoïque de formule générale II***:

$$A^{***}—NH—C_{n^{***}}H_{2n^{***}}—COOH \qquad (II^{***})$$

dans laquelle A*** et n*** ont les significations indiquées ci-dessus, en protégeant éventuellement le groupe carboxy, avec un dérivé acyle de formule générale III***:

$$R^{1***}-CO-R^{7***} \qquad (III^{***})$$

dans laquelle $R^{7***}$ représente un groupe qui s'éloigne ou un groupe $R^{1***}-CO-O-$, tandis que $R^{1***}$ a la signification indiquée ci-dessus, puis on le transforme éventuellement en un sel, ou

b) on hydrogène un acide (aryl bicyclique)amino-alcénoïque de formule générale $IV^{***}$:

$$R^{1***}-CO-\underset{\substack{|\\ A^{***}}}{N}-C_{n^{***}}H_{2n^{***}-2}-COOH \qquad (IV^{***})$$

dans laquelle $R^{1***}$, $A^{***}$ et $n^{***}$ ont les significations indiquées ci-dessus, en protégeant éventuellement le groupe carboxy, puis on le transforme éventuellement en un sel, ou

c) on solvolyse un dérivé fonctionnel d'un acide acyl (aryl bicyclique)amino-alcanoïque de formule générale $V^{***}$:

$$R^{1***}-CO-\underset{\substack{|\\ A^{***}}}{N}-C_{n^{***}}H_{2n^{***}}-G^{***} \qquad (V^{***})$$

dans laquelle $R^{1***}$, $A^{***}$ et $n^{***}$ ont les significations indiquées ci-dessus, tandis que $G^{***}$ représente un dérivé fonctionnel d'un groupe carboxy, puis on le transforme éventuellement en un sel.

6. Procédé selon la revendication 1, pour la préparation d'acides acyl(aryl bicyclique)amino-alcanoïques de formule générale $I^{****}$:

$$R^{1****}-CO-\underset{\substack{|\\ A^{****}}}{N}-C-C_{n^{****}}H_{2n^{****}}-COOH \qquad (I^{****})$$

dans laquelle

$R^{1****}$ représente un radical d'hydrocarbure aliphatique contenant 1 à 5 atomes de carbone ou un groupe phényle substitué par $R^{2****}$, $R^{3****}$ et $R^{4****}$,

$A^{****}$ représente un groupe de formule:

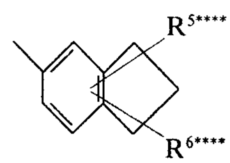

$n^{****}$ est égal à 3, 4 ou 5,

$R^{2****}$, $R^{3****}$ et $R^{4****}$, qui peuvent être identiques ou différents, représentent chacun un atome d'hydrogène, un atome d'halogène, un groupe méthyle, un groupe méthoxy, un groupe nitro, un groupe hydroxy ou un groupe trifluorométhyle,

$R^{5***}$ et $R^{6****}$, qui peuvent être identiques ou différents, représentent chacun un atome d'hydrogène, un atome de chlore, un groupe méthoxy ou un groupe nitro,

ainsi que de leurs sels avec des bases inorganiques ou organiques, caractérisé en ce que:

a) on acyle un acide (aryl bicyclique)amino-alcanoïque de formule générale $II^{****}$:

$$A^{****}-NH-C_{n^{****}}H_{2n^{****}}-COOH \qquad (II^{****})$$

dans laquelle $A^{****}$ et $n^{****}$ ont les significations indiquées ci-dessus, en protégeant éventuellement le froupe carboxy, avec un dérivé acyle de formule générale $III^{****}$:

$$R^{1****}-CO-R^{7****} \qquad (III^{****})$$

dans laquelle $R^{7****}$ représente un groupe qui s'éloigne ou un groupe $R^{1****}-CO-O-$, tandis que $R^{1****}$ a la signification indiquée ci-dessus, puis on le transforme éventuellement en un sel, ou

b) on hydrogène un acide (aryl bicyclique)amino-alcénoïque de formule générale IV****:

$$R^{1****}—CO—\underset{\displaystyle A^{****}}{\underset{|}{N}}—C_{n^{****}}H_{2n^{****}-2}—COOH \qquad (IV^{****})$$

dans laquelle $R^{1****}$, A**** et n**** ont les significations indiquées ci-dessus, en protégeant éventuellement le groupe carboxy, puis on le transforme éventuellement en un sel, ou

c) on solvolyse un dérivé fonctionnel d'un acide acyl(aryl bicyclique)amino-alcanoïque de formule générale V****:

$$R^{1****}—CO—\underset{\displaystyle A^{****}}{\underset{|}{N}}—C_{n^{****}}H_{2n^{****}}—G^{****} \qquad (V^{****})$$

dans laquelle $R^{1****}$, A**** et n**** ont les significations indiquées ci-dessus, tandis que G**** représente un dérivé fonctionnel d'un groupe carboxy, puis on le transforme éventuellement en un sel.

7. Procédé selon les revendications 4 ou 5 ou 6 dans lesquels $R^{1**}$, $R^{1***}$, ou $R^{1****}$ représente un groupe alkyle contenant 1 à 3 atomes de carbone ou un groupe phényle substitué; $R^{2**}$, $R^{2***}$ ou $R^{2****}$ représente un atome d'hydrogène; $R^{3**}$, $R^{3***}$ ou $R^{3****}$ représente un atome d'hydrogène, un atome de chlore, un groupe méthoxy, un groupe hydroxy ou un groupe trifluorométhyle; $R^{4**}$, $R^{4***}$ ou $R^{4****}$ représente un atome d'hydrogène ou un atome de chlore; $R^{5**}$, $R^{5***}$ ou $R^{5****}$ représente un atome d'hydrogène ou un groupe méthoxy et $R^{6**}$, $R^{6***}$ ou $R^{6****}$ représente un atome d'hydrogène, tandis que n**, n*** ou n**** a la signification y indiquée.

8. Procédé selon les revendiactions 4 ou 5 ou 6, dans lesqueles $R^{1**}$, $R^{1***}$ ou $R^{1****}$ représente un groupe alkyle contenant 1 à 3 atomes de carbone ou un groupe phényle substitué; $R^{2**}$, $R^{2***}$ ou $R^{2****}$ représente un atome d'hydrogène, $R^{3**}$, $R^{3***}$ ou $R^{3****}$ représente un atome de chlore ou un groupe hydroxy; $R^{4**}$, $R^{4***}$ ou $R^{4****}$ représente un atome d'hydrogène ou un atome de chlore; $R^{5**}$, $R^{5***}$ ou $R^{5****}$ représente un atome d'hydrogène ou un groupe méthoxy et $R^{6**}$, $R^{6***}$ ou $R^{6****}$ représente un atome d'hydrogène, tandis que n**, n*** ou n**** a la signification y indiquée.

9. Procédé selon les revendications 4 ou 5, dans lesquells $R^{1**}$ ou $R^{1***}$ est un groupe alkyle contenant 1 à 3 atomes de carbone ou un groupe phényle substitué, $R^{2**}$ ou $R^{2***}$ est un atome d'hydrogène; $R^{3**}$ ou $R^{3***}$ est un atome de chlore ou un groupe hydroxy; $R^{4**}$ ou $R^{4***}$ est un atome d'hydrogène $R^{5**}$ ou $R^{5***}$ est un atome d'hydrogène ou un groupe méthoxy et $R^{6**}$ ou $R^{6***}$ est un atome d'hydrogène, tandis que n** ou n*** est égal à 3 ou 4.

10. Procédé selon l'une des revendications 1 à 9, où le groupe $R^7$, $R^{7*}$, $R^{7**}$, $R^{7***}$ ou $R^{7****}$ qui s'éloigne est un atome de chlore ou un atome de brome, et où G, G*, G**, G*** ou G**** est un groupe nitrile, un ester d'acide carboxylique ou un amide d'acide carboxylique.